# EUROPEAN PATENT SPECIFICATION

(11) **EP 1 558 571 B1**
(45) Date of publication and mention of the grant of the patent: **02.06.2010**
(21) Application number: 03757742.6
(22) Date of filing: 27.10.2003
(51) Int. Cl.: C07C 321/28, C07C 409/10, A61K 31/095, A61P 3/10

(54) **NOVEL COMPOUNDS USEFUL IN TREATING PPAR MEDIATED DISEASES**
NEUE VERBINDUNGEN, DIE SICH F R DIE BEHANDLUNG VON DURCH PPAR VERMITTELTEN KRANKHEITEN EIGNEN
NOUVEAUX COMPOSES TRAITANT LES MALADIES MEDIEES PAR LE PPAR

(30) Priority: 28.10.2002 DK 200201629
(43) Date of publication of application: 03.08.2005
(73) Proprietor: High Point Pharmaceuticals, LLC, High Point, NC 27265 (US)
(72) Inventor: JEPPESEN, Lone, DK-2830 Virum (DK); PETTERSSON, Ingrid, DK-1864 Frederiksberg (DK); SAUERBERG, Per, DK-3520 Farum (DK); PIHERA, Pavel, 190 00 Praha 18 (CZ); HAVRANEK, Miroslav, 198 00 Praha 9 (CZ)
(74) Representative: Russell, Lindsey
(86) International application number: PCT/DK2003/000723
(87) International publication number: WO 2004/037775

(56) References cited:
- WO-A-01/55085
- WO-A-03/033453
- US-A- 4 148 915

## Description

### FIELD OF THE INVENTION

The present invention relates to novel compounds, to the use of these compounds as pharmaceutical compositions and to pharmaceutical compositions comprising the compounds. More specifically, the compounds of the invention can be utilised in the treatment and/or prevention of conditions mediated by the Peroxisome Proliferator-Activated Receptors (PPAR), in particular the PPARδ subtype.

### BACKGROUND OF THE INVENTION

Coronary artery disease (CAD) is the major cause of death in Type 2 diabetic and metabolic syndrome patients (i.e. patients that fall within the 'deadly quartet' category of impaired glucose tolerance, insulin resistance, hypertriglyceridaemia and/or obesity).

The hypolipidaemic fibrates and antidiabetic thiazolidinediones separately display moderately effective triglyceride-lowering activities although they are neither patent nor efficacious enough to be a single therapy of choice for the dyslipidaemia often observed in Type 2 diabetic or metabolic syndrome patients. The thiazolidinediones also patently lower circulating glucose levels of Type 2 diabetic animal models and humans. However, the fibrate class of compounds are without beneficial effects on glycaemia. Studies on the molecular actions of these compounds indicate that thiazolidinediones and fibrates exert their action by activating distinct transcription factors of the peroxisome proliferator activated receptor (PPAR) family, resulting in increased and decreased expression of specific enzymes and apolipoproteins respectively, both key-players in regulation of plasma triglyceride content. Fibrates, on the one hand, are PPARα activators, acting primarily in the liver. Thiazolidinediones, on the other hand, are high affinity ligands for PPARγ acting primarily on adipose tissue.

Adipose tissue plays a central role in lipid homeostasis and the maintenance of energy balance in vertebrates. Adipocytes store energy in the form of triglycerides during periods of nutritional affluence and release it in the form of free fatty acids at times of nutritional deprivation. The development of white adipose tissue is the result of a continuous differentiation process throughout life. Much evidence points to the central role of PPARγ activation in initiating and regulating this cell differentiation. Several highly specialised proteins are induced during adipocyte differentiation, most of them being involved in lipid storage and metabolism. The exact link from activation of PPARγ to changes in glucose metabolism, most notably a decrease in insulin resistance in muscle, has not yet been clarified. A possible link is via free fatty acids such that activation of PPARγ induces Lipoprotein Lipase (LPL), Fatty Acid Transport Protein (FATP) and Acyl-CoA Synthetase (ACS) in adipose tissue but not in muscle tissue. This, in turn, reduces the concentration of free fatty acids in plasma dramatically, and due to substrate competition at the cellular level, skeletal muscle and other tissues with high metabolic rates eventually switch from fatty acid oxidation to glucose oxidation with decreased insulin resistance as a consequence.

PPARα is involved in stimulating β-oxidation of fatty acids. In rodents, a PPARα-mediated change in the expression of genes involved in fatty acid metabolism lies at the basis of the phenomenon of peroxisome proliferation, a pleiotropic cellular response, mainly limited to liver and kidney and which can lead to hepatocarcinogenesis in rodents. The phenomenon of peroxisome proliferation is not seen in man. In addition to its role in peroxisome proliferation in rodents, PPARα is also involved in the control of HDL cholesterol levels in rodents and humans. This effect is, at least partially, based on a PPARα-mediated transcriptional regulation of the major HDL apolipoproteins, apo A-I and apo A-II. The hypotriglyceridemic action of fibrates and fatty acids also involves PPARα and can be summarised as follows: (I) an increased lipolysis and clearance of remnant particles, due to changes in lipoprotein lipase and apo C-III levels, (II) a stimulation of cellular fatty acid uptake and their subsequent conversion to acyl-CoA derivatives by the induction of fatty acid binding protein and acyl-CoA synthase, (III) an induction of fatty acid β-oxidation pathways, (IV) a reduction in fatty acid and triglyceride synthesis, and finally (V) a decrease in VLDL production. Hence, both enhanced catabolism of triglyceride-rich particles as well as reduced secretion of VLDL particles constitutes mechanisms that contribute to the hypolipidemic effect of fibrates.

PPARδ activation was initially reported not to be involved in modulation of glucose or triglyceride levels. (Berger et al., j. Biol. Chem. , 1999, Vol 274, pp. 6718-6725). Later it has been shown that PPARδ activation leads to increased levels of HDL cholesterol in dbldb mice (Leibowitz et al. FEBS letters 2000, 473, 333-336). Further, a PPARδ agonist when dosed to insulin-resistant middle-aged obese rhesus monkeys caused a dramitic dose-dependent rise in serum HDL cholesterol while lowering the levels of small dense LDL, fasting triglycerides and fasting insulin (Oliver et al. PNAS 2001, 98, 5306-5311).The same paper also showed that PPARδ activation increased the reverse cholesterol transporter ATP-binding cassette A1 and induced apolipoprotein A1-specific cholesterol efflux. The involvement of PPARδ in fatty acid oxidation in muscles was further substantiated in PPARα knoCk-out mice. Muoio et al. (J. Biol. Chem. 2002, 277, 26089-26097) showed that the high levels of PPARδ in skeletal muscle can compensate for deficiency in PPARα. Taken together these observations suggest that PPARδ activation is useful in the treatment and prevention of cardiovascular diseases and conditions including atherosclerosis, hypertriglyceridemia, and mixed dyslipidaemia (WO 01/00603).

A number of PPARδ compounds have been reported to be useful in the treatment of hyperglycemia, hyperlipidemia and hypercholesterolemia (WO 02/59098, WO 01/603, WO 01/25181, WO 02/14299, WO 01/799197, WO 99/4815, WO 97/28149, WO 98/27974, WO 97/28115, WO 97/27857, WO 97/28137, WO 97/27847).

Glucose lowering as a single approach does not overcome the macrovascular complications associated with Type 2 diabetes and metabolic syndrome. Novel treatments of Type 2 diabetes and metabolic syndrome must therefore aim at lowering both the overt hypertriglyceridaemia associated with these syndromes as well as alleviation of hyperglycaemia.

This indicate that research for compounds displaying various degree of PPARα, PPARγ and PPARδ activation should lead to the discovery of efficacious triglyceride and/or cholesterol and/or glucose lowering drugs that have great potential in the treatment of diseases such as type 2 diabetes, dyslipidemia, syndrome X (including the metabolic syndrome, i.e. impaired glucose tolerance, insulin resistance, hypertrigyceridaemia and/or obesity), cardiovascular diseases (including atherosclerosis) and hypercholesteremia.

In WO 97/48674, various antimicrobial diaryls has been described as anti-infective agents. The invention comprises compounds of the formula: 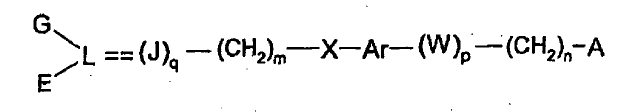 wherein L may be selected from the group consisting of N, CH and C; G, E may independently be selected from i.a. phenyl, substituted phenyl (the substituents being halogen, alkyl or alkoxy), phenylC₁₋₄-alkyl, substituted phenylC₁₋₄-alkyl. 2-pyridyl, 3-pyridyl, 4-pyridyl, 2-thienyl and 3-thienyl; J may be CH or O; X may be selected from the group consisting of is O, S, NR, and C(O)NR; Ar may be aryl or substituted aryl (the substituents being halogen, alkyl or alkoxy); W may be O or S; A may be selected from the group consisting of i.a. NRR, amidino, COOH; CHRCOOH, CH=CHR, CH=C(COOH)₂; m, n may independently be 0-6; and q, p may independently be 0 or 1.

WO 01/55085 A1 discloses compounds of general formula (I) for use in the treatment and/or prevention of conditions mediated by nuclear receptors, in particular the Peroxisome Proliferator-Activated Receptors (PPAR):

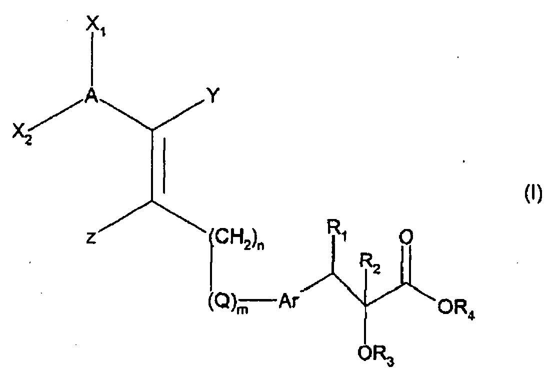

### DEFINITIONS

In the structural formulas given herein and throughout the present specification the following terms have the indicated meaning:

The term "C₁₋₆-alkyl" as used herein, alone or in combination, represent a linear or branched, saturated hydrocarbon chain having the indicated number of carbon atoms. Representative examples include, but are not limited to methyl, ethyl, n-propyl, isopropyl, butyl, isobutyl, *sec*-butyl, *tert*-butyl, pentyl, isopentyl, hexyl, isohexyl and the like.

The term "C₁₋₆-alkylcarbonyl as used herein, represents a "C₁₋₆-alkyl" group as defined above having the indicated number of carbon atoms linked through a carbonyl group. Representative examples include, but are not limited to, methylcarbonyl, ethylcarbonyl, n-propylcarbonyl, isopropylcarbonyl, butylcarbonyl, isobutylcarbonyl, *sec*-butylcarbonyl, *tert-*butylcarbonyl, n-pentylcarbonyl, isopentylcarbonyl, neopentylcarbonyl, *tert*-pentylcarbonyl, n-hexylcarbonyl, isohexylcarbonyl and the like.

The term "C₁₋₆-alkylsulfonyl" as used herein refers to a monovalent substituent comprising a "C₁₋₆-alkyl" group as defined above linked through a sulfonyl group. Representative examples include, but are not limited to, methylsulfonyl, ethylsulfonyl, n-propylsulfonyl, isopropylsulfonyl, n-butylsulfonyl, isobutylsulfonyl, *sec*-butylsulfonyl, *tert*-butylsulfonyl, n-pentylsulfonyl, isopentylsulfonyl, neopentylsulfonyl, *tert*-pentylsulfonyl, n-hexylsulfonyl, isohexylsulfonyl and the like.

The term "C₁₋₆-alkylsulfonyloxy" as used herein refers to a monovalent substituent comprising a "C₁₋₆-alkyl" group as defined above linked through a sulfonyloxy group. Representative examples include, but are not limited to, methylsulfonyloxy, ethylsulfonyloxy, n-propylsulfonyloxy, isopropylsulfonyloxy, n-butylsulfonyloxy, isobutylsulfonyloxy, *sec*-butylsulfonyloxy, *tert*-butylsulfonyloxy, n-pentylsulfonyloxy, isopentylsulfonyloxy, neopentylsulfonyloxy, *tert*-pentylsulfonyloxy, n-hexylsulfonyloxy, isohexylsulfonyloxy and the like.

The term"C₁₋₆-alkylamido" as used herein, refers to an acyl group linked through an amino group; Representative examples include, but are not limited to acetylamino, propionylamino, butyrylamino, isobutyrylamino, pivaloylamino, valerylamino and the like.

The term "C₃₋₆-cycloalkyl" as used herein, alone or in combination, represent a saturated monocyclic hydrocarbon group having the indicated number of carbon atoms. Representative examples include, but are not limited to cyclopropyl, cyclobutyl, cyclopentyl, cyclohexyl and the like.

The term "C₂₋₆-alkenyl" as used herein, represent an olefinically unsaturated branched or straight hydrocarbon group having from 2 to the specified number of carbon atoms and at least one double bond. Representative examples include, but are not limited to, vinyl, 1-propenyl, 2-propenyl, allyl, iso-propenyl, 1,3-butadienyl, 1-butenyl, hexenyl, pentenyl and the like.

The term "C₂₋₆-alkynyl" as used herein, represent an unsaturated branched or straight hydrocarbon group having from 2 to the specified number of carbon atoms and at least one triple bond. Representative examples include, but are not limited to, 1-propynyl, 2-propynyl, 1-butynyl, 2-butynyl, 1-pentynyl, 2-pentynyl and the like.

The term "C₄₋₆-alkenynyl" as used herein, represent an unsaturated branched or straight hydrocarbon group having from 4 to the specified number of carbon atoms and both at least one double bond and at least one triple bond. Representative examples include, but are not limited to, 1-penten-4-ynyl, 3-penten-1-ynyl, 1,3-hexadiene-5-ynyl and the like.

The term "C₁₋₆-alkoxy" as used herein, alone or in combination, refers to a straight or branched configuration linked through an ether oxygen having its free valence bond from the ether oxygen. Examples of linear alkoxy groups are methoxy, ethoxy, propoxy, butoxy, pentoxy, hexoxy and the like. Examples of branched alkoxy are isopropoxy, *sec*-butoxy, tert-butoxy, isopentyloxy, isohexyloxy and the like.

The term "C₃₋₆-cycloalkoxy" as used herein, alone or in combination, represent a saturated monocyclic hydrocarbon group having the indicated number of carbon atoms linked through an ether oxygen having its free valence bond from the ether oxygen. Examples of cycloalkoxy groups are cyclopropyloxy, cyclobutyloxy, cyclopentyloxy, cyclohexyloxy and the like.

The term "C₁₋₆-alkylthio" as used herein, alone or in combination, refers to a straight or branched monovalent substituent comprising a "C₁₋₆-alkyl" group as defined above linked through a divalent sulfur atom having its free valence bond from the sulfur atom and having 1 to 6 carbon atoms. Representative examples include, but are not limited to, methylthio, ethylthio, propylthio, butylthio, pentylthio and the like.

The term "C₃-6-cycloalkylthio" as used herein, alone or in combination, represent a saturated monocyclic hydrocarbon group having the indicated number of carbon atoms linked through a divalent sulfur atom having its free valence bond from the sulfur atom. Examples of cycloalkoxy groups are cyclopropylthio, cyclobutylthio, cyclopentylthio, cyclohexylthio and the like.

The term "C₁₋₆-alkylamino" as used herein, alone or in combination, refers to a straight or branched monovalent substituent comprising a "C₁₋₆-alkyl" group as defined above linked through amino having a free valence bond from the nitrogen atom. Representative examples include, but are not limited to, methylamino, ethylamino, propylamino, butylamino, pentylamino and the like.

The term "C₁₋₆-alkylaminocarbonyl" as used herein refers to a monovalent substituent comprising a C₁₋₆-monoalkylamino group linked through a carbonyl group such as e.g. methylaminocarbonyl, ethylaminocarbonyl, n-propylaminocarbonyl, isopropylaminocarbonyl, n-butylaminocarbonyl, sec-butylaminocarbonyl, isobutylaminocarbonyl, tert-butylaminocarbonyl, n-pentylaminocarbonyl, 2-methylbutylaminocarbonyl, 3-methylbutylaminocarbonyl, n-hexylaminocarbonyl, 4-methylpentylaminocarbonyl, neopentylaminocarbonyl, n-hexylaminocarbonyl and 2-2-dimethylpropylaminocarbonyl and the like.

The term "C₃₋₆-cycloalkylamino" as used herein, alone or in combination, represent a saturated monocyclic hydrocarbon group having the indicated number of carbon atoms linked through amino having a free valence bond from the nitrogen atom. Representative examples include, but are not limited to, cyclopropylamino, cyclobutylamino, cyclopentylamino, cyclohexylamino and the like.

The term "C₁₋₆-alkoxyC₁₋₆-alkyl" as used herein, alone or in combination, refers to a "C₁₋₆-alkyl" group as defined above whereto is attached a "C₁₋₆-alkoxy" group as defined above. Representative examples include, but are not limited to, methoxymethyl, ethoxymethyl, methoxyethyl, ethoxyethyl and the like.

The term "aryl" as used herein refers to an aromatic monocyclic or an aromatic fused bi- or tricyclic hydrocarbon group. Representative examples include, but are not limited to, phenyl, naphthyl, anthracenyl, phenanthrenyl, azulenyl and the like.

The term "arylene" as used herein refers to divalent aromatic monocyclic or a divalent aromatic fused bi- or tricyclic hydrocarbon group. Representative examples include, but are not limited to, phenylene, naphthylene and the like.

The term "arylcarbonyl" as used herein represents an "aryl" group as defined above linked through a carbonyl group. Representative examples include, but are not limited to, phenylcarbonyl, naphthylcarbonyl, anthracenylcarbonyl, phenanthrenylcarbonyl, azulenylcarbonyl and the like

The term "arylsulfonyl" as used herein refers to an "aryl" group as defined above linked through a sulfonyl group. Representative examples include, but are not limited to, phenylsulfonyl, naphthylsulfonyl, anthracenylsulfonyl, phenanthrenylsulfonyl, azulenylsulfonyl, and the like.

The term "arylsulfonyloxy" as used herein refers to an "aryl" group as defined above linked through a sulfonyloxy group. Representative examples include, but are not limited to, phenylsulfonyloxy, naphthylsulfonyloxy, anthracenylsulfonyloxy, phenanthrenylsulfonyloxy, azulenylsulfonyloxy, and the like.

The term "arylamido" as used herein refers to an arylcarbonyl group linked through an amino group. Representative examples include, but are not limited to phenylcarbonylamino, naphthylcarbonylamino, anthracenylcarbonylamino, phenanthrenylcarbonylamino, azulenylcarbonylamino and the like.

The term "halogen" means fluorine, chlorine, bromine or iodine.

The term "perhalomethyl" means trifluoromethyl, trichloromethyl, tribromomethyl or triiodomethyl.

The term "perhalomethoxy" means trifluoromethoxy, trichloromethoxy, tribromomethoxy or triiodomethoxy.

The term "C₁₋₆-dialkylamino" as used herein refers to an amino group wherein the two hydrogen atoms independently are substituted with a straight or branched, saturated hydrocarbon chain having the indicated number of carbon atoms. Representative examples include, but are not limited to, dimethylamino, N-ethyl-N-methylamino, diethylamino, dipropylamino, N-(n-butyl)-N-methylamino, di(n-pentyl)amino and the like.

The term "acyl" as used herein refers to a monovalent substituent comprising a "C₁₋₆-alkyl" group as defined above linked through a carbonyl group. Representative examples include, but are not limited to, acetyl, propionyl, butyryl, isobutyryl, pivaloyl, valeryl and the like.

The term "heteroaryl" as used herein, alone or in combination, refers to a monovalent substituent comprising a 5-7 membered monocyclic aromatic system or a 8-10 membered bicyclic aromatic system containing one or more heteroatoms selected from nitrogen, oxygen and sulfur, e.g. furyl, thienyl, pyrrolyl, imidazolyl, pyrazolyl, triazolyl, pyridyl, pyrazinyl, pyrimidinyl, pyridazinyl, isothiazolyl, isoxazolyl, oxazolyl, oxadiazolyl, thiadiazolyl, quinolyl, isoquinolyl, quinazolinyl, quinoxalinnyl, indolyl, benzimidazolyl, benzofuranyl, benzothienyl, pteridinyl and purinyl and the like.

The term "heteroarylene" as used herein, alone or in combination, refers to divalent 5-7 membered monocyclic aromatic system or a 8-10 membered bicyclic aromatic system containing one or more heteroatoms selected from nitrogen, oxygen and sulfur, e.g. furylene, thienylene, pyrrolylene, imidazolylene, pyrazolylene, triazolylene, pyrazinylene, pyrimidinylene, pyridazinylene, isothiazolylene, isoxazolylene, oxazolylene, oxadiazolylene, thiadiazolylene, quinolylene, isoquinolylene, quinazolinylene, quinoxalinnylene, indolylene, benzimidazolylene, benzofuranylene, pteridinylene and purinylene and the like.

The term "heteroaryloxy" as used herein, alone or in combination, refers to a heteroaryl as defined herein linked to an oxygen atom having its free valence bond from the oxygen atom e.g. pyrrolyloxy, imidazolyloxy, pyrazolyloxy, triazolyloxy, pyrazinyloxy, pyrimidinyloxy, pyridazinyloxy, isothiazolyloxy, isoxazolyloxy, oxazolyloxy, oxadiazolyloxy, thiadiazolyloxy, quinolinyloxy, isoquinolinyloxy, quinazolinyloxy, quinoxalinyloxy, indolyloxy, benzimidazolyloxy, benzofuranyloxy, pteridinyloxy and purinyloxy and the like.

The term "aralkyl" as used herein refers to a straight or branched saturated carbon chain containing from 1 to 6 carbons substituted with an aromatic carbohydride. Representative examples include, but are not limited to, benzyl, phenethyl, 3-phenylpropyl, 1-naphthylmethyl, 2-(1-naphthyl)ethyl and the like.

The term "aryloxy" as used herein refers to phenoxy, 1-naphthyloxy, 2-naphthyloxy and the like.

The term "aralkoxy" as used herein refers to a C₁₋₆-alkoxy group substituted with an aromatic carbohydride, such as benzyloxy, phenethoxy, 3-phenylpropoxy, 1-naphthylmethoxy, 2-(1-naphtyl)ethoxy and the like.

The term "heteroaralkyl" as used herein refers to a straight or branched saturated carbon chain containing from 1 to 6 carbons substituted with a heteroaryl group; such as (2-furyl)methyl, (3-furyl)methyl, (2-thienyl)methyl, (3-thienyl)methyl, (2-pyridyl)methyl, 1-methyl-1-(2-pyrimidyl)ethyl and the like.

The term "heteroaralkoxy" as used herein refers to a heteroarylalkyl as defined herein linked to an oxygen atom having its free valence bond from the oxygen atom. Representative examples include, but are not limited to, (2-furyl)methyloxy, (3-furyl)-methyloxy, (2-thienyl)methyloxy, (3-thienyl)methyloxy, (2-pyridyl)methyloxy, 1-methyl-1-(2-pyrimidyl)ethyloxy and the like.

The term "arylthio" as used herein, alone or in combination, refers to an aryl group linked through a divalent sulfur atom having its free valence bond from the sulfur atom, the aryl group optionally being mono- or polysubstituted with C₁₋₆-alkyl, halogen, hydroxy or C₁₋₆-alkoxy. Representative examples include, but are not limited to, phenylthio, (4-methylphenyl)-thio, (2-chlorophenyl)thio and the like.

Certain of the above defined terms may occur more than once in the structural formulae, and upon such occurrence each term shall be defined independently of the other.

The term "optionally substituted" as used herein means that the groups in question are either unsubstituted or substituted with one or more of the substituents specified. When the groups in question are substituted with more than one substituent the substituents may be the same or different.

### DESCRIPTION OF THE INVENTION

The present invention relates to compounds of the general formula (I): 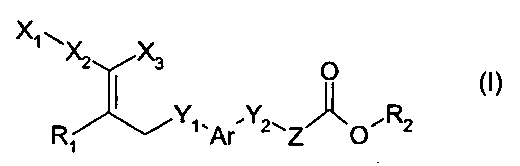
wherein Xᵢ is aryl or heteroaryl each of which is optionally substituted with one or more substituents selected from
   - halogen, hydroxy, cyano, amino or carboxy; or
   - C₁₋₆-alkyl, C₃₋₆-cycloalkyl, C₂₋₆-alkenyl, C₂₋₆-alkynyl, aryl, aralkyl, heteroaryl, heteroaralkyl, C₁₋₆-alkoxy, C₃₋₆-cycloalkoxy, aryloxy, aralkoxy, heteroaralkoxy, C₁₋₆-alkylthio, arylthio, C₃₋₆-cycloalkylthio, C₁₋₆-alkylcarbonyl, arylcarbonyl, C₁₋₆-alkylsulfonyl, arylsulfonyl, C₁₋₆-alkylsulfonyloxy, arylsulfonyloxy, C₁₋₆-alkylamido, arylamido, C₁₋₆-alkylaminocarbonyl, C₁₋₆-alkylamino, C₁₋₆-dialkylamino or C₃₋₆-cycloalkylamino each of which is optionally substituted with one or more halogens; and
X₂ is arylene or heteroarylene each of which is optionally substituted with one or more substituents selected from
   - halogen, hydroxy, cyano, amino or carboxy; or
   - C₁₋₆-alkyl, C₃₋₆-cycloalkyl, C₂₋₆-alkenyl, C₂₋₈-alkynyl, C₁₋₈-alkoxy, C₃₋₆-cycloalkoxy, C₁₋₆-alkylthio, C₃₋₆-cycloalkylthio, C₁₋₆-alkylamino, C₁₋₈-dialkylamino or C₃₋₆-cycloalkylamino each of which is optionally substituted with one or more halogens; and
X₃ is aryl or heteroaryl each of which is optionally substituted with one or more substituents selected from
   - halogen, hydroxy, cyano, amino or carboxy; or
   - C₁₋₆-alkyl, C₃₋₆-cycloalkyl, C₂₋₆-alkenyl, C₂₋₆-alkynyl, aralkyl, heteroaralkyl, C₁₋₆-alkoxy, C₃₋₆-cycloalkoxy, aryloxy, aralkoxy, heteroaralkoxy, C₁₋₆-alkylthio, arylthio, C₃₋₆-cycloalkylthio, C₁₋₈-alkylcarbonyl, arylcarbonyl, C₁₋₈-alkylsulfonyl, arylsulfonyl, C₁₋₆-alkylsulfonyloxy, arylsulfonyloxy, C₁₋₆-alkylamido, arylamido, C₁₋₈-alkylaminocarbonyl, C₁₋₆-alkylamino, C₁₋₆-dialkylamino or C₃₋₆-cycloalkylamino each of which is optionally substituted with one or more halogens; and
Ar is arylene which is optionally substituted with one or more substituents selected from
   - halogen, hydroxy or cyano; or
   - C₁₋₆-alkyl, C₃₋₆-cycloalkyl, C₂₋₆-alkenyl, C₂₋₆-alkynyl, aryl, heteroaryl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio each of which is optionally substituted with one or more halogens; and
Y₁ is O or S; and
Y₂ is O or S; and
Z is -(CH₂)ₙ- wherein n is 1, 2 or 3; and
R₁ is hydrogen, halogen or a substituent selected from
   - C₁₋₆-alkyl, C₃₋₆-cycloalkyl, C₂₋₆-alkenyl, C₂₋₆-alkynyl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio each of which is optionally substituted with one or more halogens; and
R₂ is hydrogen, C₁₋₆-alkyl, C₃₋₆-cycloalkyl, C₂₋₆-alkenyl, C₂₋₆-alkynyl, C₄₋₆-alkenynyl or aryl; or
a pharmaceutically acceptable salt thereof, or a pharmaceutically acceptable solvate thereof, or any tautomeric forms, stereoisomers, mixture of stereoisomers including a racemic mixture, or polymorphs.

In one embodiment, the present invention is concerned with compounds of formula (I) wherein X₁ is aryl optionally substituted with one or more substituents selected from
- halogen; or
- C₁₋₆-alkyl, aryl, C₁₋₆-alkoxy, C₁₋₆-alkylsulfonyl or C₁₋₆-alkylsulfonyloxy each of which is optionally substituted with one or more halogens.

In another embodiment, the present invention is concerned with compounds of formula (I) wherein X₁ is aryl optionally substituted with one or more substituents selected from
- halogen; or
- C₁₋₆-alkyl or aryl optionally substituted with one or more halogens.

In another embodiment, the present invention is concerned with compounds of fomula (I) wherein X₁ is aryl optionally substituted with one or more halogens, phenyl or perhalomethyl.

In another embodiment, the present invention is concerned with compounds of fomula (I) wherein X₁ is aryl.

In another embodiment, the present invention is concerned with compounds of formula (I) wherein X₁ is phenyl optionally substituted with one or more substituents selected from
- halogen; or
- C₁₋₆-alkyl, aryl, C₁₋₆-alkoxy, C₁-₆-alkylsulfonyl or C₁₋₆-alkylsulfonyloxy each of which is optionally substituted with one or more halogens.

In another embodiment, the present invention is concerned with compounds of formula (I) wherein X₁ is phenyl optionally substituted with one or more substituents selected from
- halogen; or
- C₁₋₆-alkyl or aryl optionally substituted with one or more halogens.

In another embodiment, the present invention is concerned with compounds of formula (I) wherein X₁ is phenyl optionally substituted with one or more halogens.

In another embodiment, the present invention is concerned with compounds of formula (I) wherein X₁ is phenyl optionally substituted with one or more of phenyl.

In another embodiment, the present invention is concerned with compounds of formula (I) wherein X₁ is phenyl optionally substituted with one or more of perhalomethyl.

In another embodiment, the present invention is concerned with compounds of formula (I) wherein X₁ is phenyl.

In another embodiment, the present invention is concerned with compounds of formula (I) wherein X₁ is heteroaryl optionally substituted with one or more substituents selected from
- halogen; or
- C₁-alkyl, aryl, C₁₋₆-alkoxy, C₁₋₆-alkylsulfonyl or C₁₋₆-alkylsulfonyloxy each of which is optionally substituted with one or more halogens.

In another embodiment, the present invention is concerned with compounds of formula (I) wherein X₁ is heteroaryl optionally substituted with one or more substituents selected from
- halogen; or
- C₁₋₆-alkyl or aryl optionally substituted with one or more halogens.

In another embodiment, the present invention is concerned with compounds of formula (I) wherein X₁ is heteroaryl optionally substituted with one or more halogens.

In another embodiment, the present invention is concerned with compounds of formula (I) wherein X₁ is heteroaryl optionally substituted with one or more of C₁₋₆-alkyl or perhalomethyl.

In another embodiment, the present invention is concerned with compounds of formula (I) wherein X₁ is heteroaryl.

In another embodiment, the present invention is concerned with compounds of formula (I) wherein X₁ is furyl, thienyl, benzothienyl or benzofuranyl optionally substituted with one or more halogens.

In another embodiment, the present invention is concerned with compounds of formula (I) wherein X₁ is furyl, thienyl, benzothienyl or benzofuranyl optionally substituted with one or more of C₁₋₆-alkyl.

In another embodiment, the present invention is concerned with compounds of formula (I) wherein X₂ is arylene optionally substituted with one or more substituents selected from
- halogen; or
- C₁₋₆-alkyl or C₁₋₆-alkoxy each of which is optionally substituted with one or more halogens.

In another embodiment, the present invention is concerned with compounds of formula (I) wherein X₂ is arylene optionally substituted with one or more halogens.

In another embodiment, the present invention is concerned with compounds of formula (I) wherein X₂ is arylene optionally substituted with one or more of C₁₋₆-alkyl.

In another embodiment, the present invention is concerned with compounds of formula (I) wherein X₂ is arylene.

In another embodiment, the present invention is concerned with compounds of formula (I) wherein X₂ is phenylene optionally substituted with one or more substituents selected from
- halogen; or
- C₁₋₆-alkyl or C₁₋₆-alkoxy each of which is optionally substituted with one or more halogens.

In another embodiment, the present invention is concerned with compounds of formula (I) wherein X₂ is phenylene optionally substituted with one or more halogens.

In another embodiment, the present invention is concerned with compounds of formula (I) wherein X₂ is phenylene optionally substituted with one or more of C₁₋₆-alkyl.

In another embodiment, the present invention is concerned with compounds of formula (I) wherein X₂ is phenylene.

In another embodiment, the present invention is concerned with compounds of formula (I) wherein X₂ is heteroarylene optionally substituted with one or more substituents selected from
- halogen; or
- C₁₋₆-alkyl or C₁₋₆-alkoxy each of which is optionally substituted with one or more halogens.

In another embodiment, the present invention is concerned with compounds of for mula (I) wherein X₂ is heteroarylene optionally substituted with one or more halogens.

In another embodiment, the present invention is concerned with compounds of formula (I) wherein X₂ is heteroarylene.

In another embodiment, the present invention is concerned with compounds of formula (I) wherein X₂ is benzofuranylene.

In another embodiment, the present invention is concerned with compounds of formula (I) wherein X₃ is aryl optionally substituted with one or more substituents selected from
- halogen; or
- C₁₋₆-alkyl, C₁₋₆-alkoxy, C₁₋₆-alkylsulfonyl or C₁₋₆-alkylsulfonyloxy each of which is optionally substituted with one or more halogens.

In another embodiment, the present invention is concerned with compounds of formula (I) wherein X₃ is aryl optionally substituted with one or more substituents selected from
- halogen; or
- C₁₋₆-alkyl optionally substituted with one or more halogens.

In another embodiment, the present invention is concerned with compounds of formula (I) wherein X₃ is aryl.

In another embodiment, the present invention is concerned with compounds of formula (I) wherein X₃ is phenyl optionally substituted with one or more substituents selected from
- halogen; or
- C₁₋₆-alkyl, C₁₋₆-alkoxy, C₁₋₆-alkylsulfonyl or C₁₋₆-alkylsulfonyloxy each of which is optionally substituted with one or more halogens.

In another embodiment, the present invention is concerned with compounds of formula (I) wherein X₃ is phenyl optionally substituted with one or more substituents selected from
- halogen; or
- C₁₋₆-alkyl optionally substituted with one or more halogens.

In another embodiment, the present invention is concerned with compounds of formula (I) wherein X₃ is phenyl optionally substituted with one or more halogens.

In another embodiment, the present invention is concerned with compounds of formula (I) wherein X₃ is phenyl.

In another embodiment, the present invention is concerned with compounds of formula (I) wherein X₃ is heteroaryl optionally substituted with one or more substituents selected from
- halogen; or
- C₁₋₆-alkyl, C₁₋₆-alkoxy, C₁₋₆-alkylsulfonyl or C₁₋₆-alkylsulfonyloxy each of which is optionally substituted with one or more halogens.

In another embodiment, the present invention is concerned with compounds of formula (I) wherein X₃ is heteroaryl optionally substituted with one or more substituents selected from
- halogen; or
- C₁₋₆-alkyl optionally substituted with one or more halogens.

In another embodiment, the present invention is concerned with compounds of formula (I) wherein X₃ is heteroaryl optionally substituted with one or more halogens.

In another embodiment, the present invention is concerned with compounds of formula (I) wherein X₃ is heteroaryl optionally substituted with one or more of C₁₋₆-alkyl.

In another embodiment, the present invention is concerned with compounds of formula (I) wherein X₃ is heteroaryl.

In another embodiment, the present invention is concerned with compounds of formula (I) wherein X₃ is furyl, thienyl, benzothienyl or benzofuranyl optionally substituted with one or more halogens.

In another embodiment, the present invention is concerned with compounds of formula (I) wherein X₃ is furyl, thienyl, benzothienyl or benzofuranyl optionally substituted with one or more of C₁₋₆-alkyl.

In another embodiment, the present invention is concerned with compounds of formula (I) wherein X₃ is furyl, thienyl, benzothienyl or benzofuranyl.

In another embodiment, the present invention is concerned with compounds of formula (I) wherein Ar is phenylene which is optionally substituted with one or more substituents selected from
- halogen, hydroxy or cyano; or
- C₁₋₆-alkyl, C₃₋₆-cycloalkyl, C₂₋₆-alkenyl, C₂₋₆-alkynyl, aryl, heteroaryl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio each of which is optionally substituted with one or more halogens.

In another embodiment, the present invention is concerned with compounds of formula (I) wherein Ar is phenylene which is optionally substituted with one or more substituents selected from
- halogen; or
- C₁₋₆-alkyl, aryl, C₁₋₆-alkoxy, aryloxy or aralkoxy each of which is optionally substituted with one or more halogens.

In another embodiment, the present invention is concerned with compounds of formula (I) wherein Ar is phenylene which is optionally substituted with one or more halogens.

In another embodiment, the present invention is concerned with compounds of formula (I) wherein Ar is phenylene which is optionally substituted with one or more C₁₋₆-alkyl optionally substituted with one or more halogens.

In another embodiment, the present invention is concerned with compounds of formula (I) wherein Ar is phenylene which is optionally substituted with one or more C₁₋₆-alkoxy optionally substituted with one or more halogens.

In another embodiment, the present invention is concerned with compounds of formula (I) wherein Ar is phenylene which is optionally substituted with one or more aryl optionally substituted with one or more halogens.

In another embodiment, the present invention is concerned with compounds of formula (I) wherein Ar is phenylene which is optionally substituted with methyl.

In another embodiment, the present invention is concerned with compounds of formula (I) wherein Ar is phenylene which is optionally substituted with metoxy.

In another embodiment, the present invention is concerned with compounds of formula (I) wherein Ar is phenylene which is optionally substituted with one or more of phenyl.

In another embodiment, the present invention is concerned with compounds of formula (I) wherein Ar is phenylene.

In another embodiment, the present invention is concerned with compounds of formula (I) wherein Y₁ is S.

In another embodiment, the present invention is concerned with compounds of formula (I) wherein Y₁ is O.

In another embodiment, the present invention is concerned with compounds of formula (I) wherein Y₂ is O.

In another embodiment, the present invention is concerned with compounds of formula (I) wherein Y₂ is S.

In another embodiment, the present invention is concerned with compounds of formula (I) wherein n is 1.

In another embodiment, the present invention is concerned with compounds of formula (I) wherein R₁ is hydrogen or a substituent selected from
- C₁₋₆-alkyl, aralkyl, C₁₋₆-alkoxy, aryloxy, aralkoxy each of which is optionally substituted with one or more halogens.

In another embodiment, the present invention is concerned with compounds of formula (I) wherein R₁ is hydrogen or a substituent selected from
- C₁₋₆-alkyl, C₁₋₆-alkoxy each of which is optionally substituted with one or more halogens.

In another embodiment, the present invention is concerned with compounds of formula (I) wherein R₁ is hydrogen.

In another embodiment, the present invention is concerned with compounds of formula (I) wherein R₁ is methoxy or ethoxy.

In another embodiment, the present invention is concerned with compounds of formula (I) wherein R₂ is hydrogen.

In another embodiment, the present invention is concerned with compounds of formula (I) wherein R₂ is methyl or ethyl.

In another embodiment, the present invention is concerned with compounds of formula I wherein alkyl is methyl or ethyl.

In another embodiment, the present invention is concerned with compounds of formula I wherein alkenyl is vinyl or 1-propenyl.

In another embodiment, the present invention is concerned with compounds of formula I wherein alkynyl is 1-propynyl.

In another embodiment, the present invention is concerned with compounds of formula I wherein alkenynyl is 1-pentene-4-yne.

In another embodiment, the present invention is concerned with compounds of formula I wherein alkoxy is methoxy, ethoxy, isopropoxy or cyclopropoxy.

In another embodiment, the present invention is concerned with compounds of formula I wherein aryl is phenyl.

In another embodiment, the present invention is concerned with compounds of formula I wherein arylene is phenylene.

In another embodiment, the present invention is concerned with compounds of formula I wherein halogen is bromine, fluorine or chlorine.

In another embodiment, the present invention is concerned with compounds of formula I wherein perhalomethyl is trifluoromethyl.

In another embodiment, the present invention is concerned with compounds of formula I wherein perhalomethoxy is trifluoromethoxy,

In another embodiment, the present invention is concerned with compounds of formula I wherein heteroaryl is furyl or thienyl.

In another embodiment, the present invention is concerned with compounds of formula I wherein aralkyl is benzyl.

In another embodiment, the present invention is concerned with compounds of formula I wherein aryloxy is phenoxy.

In another embodiment, the present invention is concerned with compounds of formula I wherein aralkoxy is benzyloxy.

In another embodiment, the present invention is concerned with compounds of formula I wherein the substituents R₁ and X₃ are arranged in a trans-configuration.

In another embodiment, the present invention is concerned with compounds of formula I wherein the substituents R₁ and X₃ are arranged in a cis-configuration.

In another embodiment, the present invention is concerned with compounds of formula I which are PPARδ agonists.

In another embodiment, the present invention is concerned with compounds of formula I which are selective PPARδ agonists.

Examples of specific compounds of the invention are:
(E/Z) {4-[3-Biphenyl-4-yl-3-(4-bromo-phenyl)-allylsulfanyl]-phenoxy}-acetic acid,
(E/Z) {4-[3-(4-Bromo-phenyl)-3-(3'-trifluoromethyl-biphenyl-4-yl)-allylsulfanyl]-2-methylphenoxy}-acetic acid; or
a pharmaceutically acceptable salt thereof, or a pharmaceutically acceptable solvate thereof, or any tautomeric forms, stereoisomers, mixture of stereoisomers including a racemic mixture, or polymorphs.

Other examples of specific compounds of the invention are:
{4-[3-(4-Bromo-phenyl)-3-[1,1';4' 1"]terphenyl-4"-yl-allylsulfanyl]-2-methyl-phenoxy}-acetic acid,
(E/Z)-[2-Methyl-4-[3-[5-(5-methylthiophen-2-yl)benzo[b]furan-2-yl]-3-(thiophen-2-yl)allylsulfanyl]phenoxy]acetic acid,
(E/Z)-[4-[3-(Biphenyl-4-yl)-3-(furan-2-yl)allylsulfanyl]-2-methylphenoxy]acetic acid,
(E/Z)-[4-[3-(Benzo[b]thiophen-3-yl)-3-(biphenyl-4-yl)allylsulfanyl]-2-methylphenoxy]acetic acid,
[4-[(3-Benzo[b]thiophen-2-yl)-3-(biphenyl-4-yl)-allylsulfanyl]-2-methyl-phenoxy]-acetic acid,
(E/Z)-[4-[3-(4-Biphenylyl)-3-(5-methylthiophen-2-yl)allylsulfanyl]-2-methylphenoxy]acetic acid,
or a pharmaceutically acceptable salt thereof, or a pharmaceutically acceptable solvate thereof, or any tautomeric forms, stereoisomers, mixture of stereoisomers including a racemic mixture, or polymorphs.

Other examples of specific compounds of the invention are:
(E) {4-[3-Biphenyl-4-yl-3-(2-fluoro-phenyl)-allylsulfanyl]-phenoxy}-acetic acid;
(Z) {4-[3-Biphenyl-4-yl-3-(2-fluoro-phenyl)-allylsulfanyl]-phenoxyl}-acetic acid;
(E) {4-[3-Biphenyl-4-yl-3-(2-chloro-phenyl)-allylsulfanyl]-phenoxy}-acetic acid;
(Z) {4-[3-Biphenyl-4-yl-3-(2-chloro-phenyl)-allylsulfanyl]-phenoxy}-acetic acid;
(E) {4-[3-Biphenyl-4-yl-3-(2-bromo-phenyl)-allylsulfanyl]-phenoxy}-acetic acid;
(Z) {4-[3-Biphenyl-4-yl-3-(2-bromo-phenyl)-allylsulfanyl]-phenoxy}-acetic acid;
(E) {4-[3-Biphenyl-4-yl-3-(2-iodo-phenyl)-allylsulfanyl]-phenoxy}-acetic acid;
(Z) {4-[3-Biphenyl-4-yl-3-(2-iodo-phenyl)-allylsulfanyl]-phenoxy}-acetic acid;
(E) {4-[3-Biphenyl-4-yl-3-(2-methoxy-phenyl)-allylsulfanyl]-phenoxy}-acetic acid;
(Z) {4-[3-Biphenyl-4-yl-3-(2-methoxy-phenyl)-allylsulfanyl]-phenoxy}-acetic acid;
(E) {4-[3-Biphenyl-4-yl-3-(2-trifluoromethoxy-phenyl)-allylsulfanyl]-phenoxy}-acetic acid;
(Z) {4-[3-Biphenyl-4-yl-3-(2-trifluoromethoxy-phenyl)-allylsulfanyl]-phenoxy}-acetic acid;
(E) {4-[3-Biphenyl-4-yl-3-(2-methyl-phenyl)-allylsulfanyl]-phenoxy}-acetic acid;
(Z) {4-[3-Biphenyl-4-yl-3-(2-methyl-phenyl)-allylsulfanyl]-phenoxy}-acetic acid;
(E) {4-[3-Biphenyl-4-yl-3-(2-trifluoromethyl-phenyl)-allylsulfanyl]-phenoxy}-acetic acid;
(Z) {4-[3-Biphenyl-4-yl-3-(2-trifluoromethyl-phenyl)-allylsulfanyl]-phenoxy}-acetic acid;
(E) {4-[3-Biphenyl-4-yl-3-(3-fluoro-phenyl)-allylsulfanyl]-phenoxy}-acetic acid;
(Z) {4-[3-Biphenyl-4-yl-3-(3-fluoro-phenyl)-allylsulfanyl]-phenoxy}-acetic acid;
(E) {4-[3-Biphenyl-4-yl-3-(3-chloro-phenyl)-allylsulfanyl]-phenoxy}-acetic acid;
(Z)-{4-[3-Biphenyl-4-yl-3-(3-chloro-phenyl)-allylsulfanyl]-phenoxy}-acetic acid;
(E) {4-[3-Biphenyl-4-yl-3-(3-bromo-phenyl)-allylsulfanyl]-phenoxy}-acetic acid;
(Z) {4-[3-Biphenyl-4-yl-3-(3-bromo-phenyl)-allylsulfanyl]-phenoxy}-acetic acid;
(E) {4-[3-Biphenyl-4-yl-3-(3-iodo-phenyl)-allylsulfanyl]-phenoxy}-acetic acid;
(Z) {4-[3-Biphenyl-4-yl-3-(3-iodo-phenyl)-allylsulfanyl]-phenoxy}-acetic acid;
(E) {4-[3-Biphenyl-4-yl-3-(3-methoxy-phenyl)-allylsulfanyl]-phenoxy}-acetic acid;
(Z) {4-[3-Biphenyl-4-yl-3-(3-methoxy-phenyl)-allylsulfanyl]-phenoxy}-acetic acid;
(E) {4-[3-Biphenyl-4-yl-3-(3-trifluoromethoxy-phenyl)-allylsuIfanyl]-phenoxy}-acetic acid;
(Z) (4-[3-Biphenyl-4-yl-3-(3-trifluoromethoxy-phenyl)-allylsulfanyl]-phenoxy)-acetic acid;
(E) {4-[3-Biphenyl-4-yl-3-(3-methyl-phenyl)-allylsulfanyl]-phenoxy}-acetic acid;
(Z) {4-[3-Biphenyl-4-yl-3-(3-methyl-phenyl)-allylsulfanyl]-phenoxy}-acetic acid;
(E) {4-[3-Biphenyl-4-yl-3-(3-trifluoromethyl-phenyl)-allylsulfanyl]-phenoxy}-acetic acid;
(Z) {4-[3-Biphenyl-4-yl-3-(3-trifluoromethyl-phenyl)-allylsulfanyl]-phenoxy}-acetic acid;
(E) {4-[3-Biphenyl-4-yl-3-(4-fluoro-phenyl)-allylsulfanyl]-phenoxy}-acetic acid;
(Z) {4-[3-Biphenyl-4-yl-3-(4-fluoro-phenyl)-allylsulfanyl]-phenoxy}-acetic acid;
(E) {4-[3-Biphenyl-4-yl-3-(4-chloro-phenyl)-allylsulfanyl]-phenoxy}-acetic acid;
(Z) {4-[3-Biphenyl-4-yl-3-(4-chloro-phenyl)-allylsulfanyl]-phenoxy}-acetic acid;
(E/Z) {4-[3-Biphenyl-4-yl-3-(4-bromo-phenyl)-allylsulfanyl]-phenoxy}-acetic acid;
(E) {4-[3-Biphenyl-4-yl-3-(4-bromo-phenyl)-allylsulfanyl]-phenoxy}-acetic acid;
(Z) {4-[3-Biphenyl-4-yl-3-(4-bromo-phenyl)-allylsulfanyl]-phenoxy}-acetic acid;
(E) {4-[3-Biphenyl-4-yl-3-(4-iodo-phenyl)-allylsulfanyl]-phenoxy}-acetic acid;
(Z) {4-[3-Biphenyl-4-yl-3-(4-iodo-phenyl)-allylsulfanyl]-phenoxy}-acetic acid;
(E) {4-[3-Biphenyl-4-yl-3-(4-methoxy-phenyl)-allylsulfanyl]-phenoxy}-acetic acid;
(Z) {4-[3-Biphenyl-4-yl-3-(4-methoxy-phenyl)-allylsulfanyl]-phenoxy}-acetic acid;
(E) {4-[3-Biphenyl-4-yl-3-(4-trifluoromethoxy-phenyl)-allylsulfanyl]-phenoxy}-acetic acid;
(Z) {4-[3-Biphenyl-4-yl-3-(4-trifluoromethoxy-phenyl)-allylsulfanyl]-phenoxy}-acetic acid;
(E) {4-[3-Biphenyl-4-yl-3-(4-methyl-phenyl)-allylsulfanyl]-phenoxy}-acetic acid;
(Z) {4-[3-Biphenyl-4-yl-3-(4-methyl-phenyl)-allylsulfanyl]-phenoxy}-acetic acid;
(E) {4-[3-Biphenyl-4-yl-3-(4-trifluoromethyl-phenyl)-allylsulfanyl]-phenoxy}-acetic acid;
(Z) {4-[3-Biphenyl-4-yl-3-(4-trifluoromethyl-phenyl)-allylsulfanyl]-phenoxy}-acetic acid;
(E) {4-[3-(4-Fluoro-phenyl)-3-(2-trifluoromethyl-biphenyl-4-yl)-allylsulfanyl]-2-methylphenoxy}-acetic acid;
(Z) {4-[3-(4-Fluoro-phenyl)-3-(2-trifluoromethyl-biphenyl-4-yl)-allylsulfanyl]-2-methylphenoxy}-acetic acid;
(E/Z) {4-[3-(4-Fluoro-phenyl)-3-(3'-trifluoromethyl-biphenyl-4-yl)-allylsulfanyl]-2-methylphenoxy}-acetic acid;
(E) {4-[3-(4-Fluoro-phenyl)-3-(3'-trifluoromethyl-biphenyl-4-yl)-allylsulfanyl]-2-methylphenoxy}-acetic acid;
(Z) {4-[3-(4-Fluoro-phenyl)-3-(3'-trifluoromethyl-biphenyl-4-yl)-allylsulfanyl]-2-methylphenoxy}-acetic acid;
(E) {4-[3-(4-Fluoro-phenyl)-3-(4'-trifluoromethyl-biphenyl-4-yl)-allylsulfanyl]-2-methylphenoxy}-acetic acid;
(Z) {4-[3-(4-Fluoro-phenyl)-3-(4'-trifluoromethyl-biphenyl-4-yl)-allylsulfanyl]-2-methylphenoxy}-acetic acid;
(E) (4-[3-(4-Fluoro-phenyl)-3-(3'-chloro-biphenyl-4-yl)-allylsulfanyl]-2-methyl-phenoxy)-acetic acid;
(Z) {4-[3-(4-Fluoro-phenyl)-3-(3'-chloro-biphenyl-4-yl)-allylsulfanyl]-2-methyl-phenoxy}-acetic acid;
(E) {4-[3-(4-Fluoro-phenyl)-3-(4'-chloro-biphenyl-4-yl)-allylsulfanyl]-2-methyl-phenoxy}-acetic acid;
(Z) {4-[3-(4-Fluoro-phenyl)-3-(4'-chloro-biphenyl-4-yl)-allylsulfanyl]-2-methyl-phenoxy}-acetic acid;
(E) {4-[3-(4-Fluoro-phenyl)-3-(3'-methoxy-biphenyl-4-yl)-allylsulfanyl]-2-methyl-phenoxy}-acetic acid;
(Z) (4-[3-(4-Fluoro-phenyl)-3-(3'-methoxy-biphenyl-4-yl)-allylsulfanyl]-2-methyl-phenoxy)-acetic acid;
(E) {4-[3-(4-Fluoro-phenyl)-3-(4'-methoxy-biphenyl-4-yl)-allylsulfanyl]-2-methyl-phenoxy}-acetic acid;
(Z) {4-[3-(4-Fluoro-phenyl)-3-(4'-methoxy-biphenyl-4-yl)-allylsulfanyl]-2-methyl-phenoxy}-acetic acid;
(E) {4-[3-(4-Chloro-phenyl)-3-(2'-trifluoromethyl-biphenyl-4-yl)-allylsulfanyl]-2-methylphenoxy}-acetic acid;
(Z) {4-[3-(4-Chloro-phenyl)-3-(2'-trifluoromethyl-biphenyl-4-yl)-allylsulfanyl]-2-methylphenoxy}-acetic acid;
(E/Z) {4-[3-(4-Chloro-phenyl)-3-(3'-trifluoromethyl-biphenyl-4-yl)-allylsulfanyl]-2-methylphenoxy}-acetic acid;
(E) {4-[3-(4-Chloro-phenyl)-3-(3'-trifluoromethyl-biphenyl-4-yl)-allylsulfanyl]-2-methylphenoxy}-acetic acid;
(Z) {4-[3-(4-Chloro-phenyl)-3-(3'-trifluoromethyl-biphenyl-4-yl)-allylsulfanyl]-2-methylphenoxy}-acetic acid;
(E) {4-[3-(4-Chloro-phenyl)-3-(4'-trifluoromethyl-biphenyl-4-yl)-allylsulfanyl]-2-methylphenoxy}-acetic acid;
(Z) {4-[3-(4-Chloro-phenyl)-3-(4'-trifluoromethyl-biphenyl-4-yl)-allylsulfanyl]-2-methylphenoxy}-acetic acid;
(E) {4-[3-(4-Chloro-phenyl)-3-(3'-chloro-biphenyl-4-yl)-allylsulfanyl]-2-methyl-phenoxy}-acetic acid;
(Z) {4-[3-(4-Chloro-phenyl)-3-(3'-chloro-biphenyl-4-yl)-allylsulfanyl]-2-methyl-phenoxy}-acetic acid;
(E) {4-[3-(4-Chloro-phenyl)-3-(4'-chloro-biphenyl-4-yl)-allylsulfanyl]-2-methyl-phenoxy}-acetic acid;
(Z) {4-[3-(4-Chloro-phenyl)-3-(4'-chloro-biphenyl-4-yl)-allylsulfanyl]-2-methyl-phenoxy}-acetic acid;
(E) {4-[3-(4-Chloro-phenyl)-3-(3'-methoxy-biphenyl-4-yl)-allyisulfanyl]-2-methyl-phenoxy}-acetic acid;
(Z) {4-[3-(4-Chloro-phenyl)-3-(3'-methoxy-biphenyl-4-yl)-allylsulfanyl]-2-methyl-phenoxy}-acetic acid;
(E) {4-[3-(4-Chloro-phenyl)-3-(4'-methoxy-biphenyl-4-yl)-allylsulfanyl]-2-methyl-phenoxy}-acetic acid;
(Z) {4-[3-(4-Chloro-phenyl)-3-(4'-methoxy-biphenyl-4-yl)-allylsulfanyl]-2-methyl-phenoxy}-acetic acid;
(E) {4-[3-(4-Bromo-phenyl)-3-(2'-trifluoromethyl-biphenyl-4-yl)-allylsulfanyl]-2-methylphenoxy}-acetic acid;
(Z) {4-[3-(4-Bromo-phenyl)-3-(2'-trifluoromethyl-biphenyl-4-yl)-allylsulfanyl]-2-methylphenoxy}-acetic acid;
(E/Z) {4-[3-(4-Bromo-phenyl)-3-(3'-trifluoromethyl-biphenyl-4-yl)-allylsulfanyl]-2-methylphenoxy}-acetic acid;
(E) {4-[3-(4-Bromo-phenyl)3-(3'-trifluoromethyl-biphenyl-4-yl)-allylsulfanyl]-2-methylphenoxy}-acetic acid;
(Z) {4-[3-(4-Bromo-phenyl)-3-(3'-trifluoromethyl-biphenyl-4-yl)-allylsulfanyl]-2-methylphenoxy}-acetic acid;
(E) {4-[3-(4-Bromo-phenyl)-3-(4'-trifluoromethyl-biphenyl-4-yl)-allylsulfanyl]-2-methylphenoxy}-acetic acid;
(Z) {4-[3-(4-Bromo-phenyl)-3-(4'-trifluoromethyl-biphenyl-4-yl)-allylsulfanyl]-2-methylphenoxy}-acetic acid;
(E) {4-[3-(4-Bromo-phenyl)-3-(3'-chloro-biphenyl-4-yl)-allylsulfanyl]-2-methyl-phenoxy}-acetic acid;
(Z) {4-[3-(4-Bromo-phenyl)-3-(3'-chloro-biphenyl-4-yl)-allylsulfanyl]-2-methyl-phenoxy}-acetic acid;
(E) {4-[3-(4-Bromo-phenyl)-3-(4'-chloro-biphenyl-4-yl)-allylsulfanyl]-2-methyl-phenoxy}-acetic acid;
(Z) {4-[3-(4-Bromo-phenyl)-3-(4'-chloro-biphenyl-4-yl)-allylsulfanyl]-2-methyl-phenoxy}-acetic acid;
(E) {4-[3-(4-Bromo-phenyl)-3-(3'-methoxy-biphenyl-4-yl)-allylsulfanyl]-2-methyl-phenoxy}-acetic acid;
(Z) {4-[3-(4-Bromo-phenyl)-3-(3'-methoxy-biphenyl-4-yl)-allylsulfanyl]-2-methyl-phenoxy}-acetic acid;
(E) (4-[3-(4-Bromo-phenyl)-3-(4'-methoxy-biphenyl-4-yl)-allylsulfanyl]-2-methyl-phenoxy)-acetic acid;
(Z) {4-[3-(4-Bromo-phenyl)-3-(4'-methoxy-biphenyt-4-yl)-allylsulfanyl]-2-methyl-phenoxy}-acetic acid; or
a pharmaceutically acceptable salt thereof, or a pharmaceutically acceptable solvate thereof, or any tautomeric forms, stereoisomers, mixture of stereoisomers including a racemic mixture, or polymorphs.

The present invention also encompasses pharmaceutically acceptable salts of the present compounds. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Further examples of pharmaceutically acceptable inorganic or organic acid addition salts include the pharmaceutically acceptable salts listed in J. Pharm. Sci. 1977, 66, 2, which is incorporated herein by reference. Examples of metal salts include lithium, sodium, potassium, magnesium, zinc, calcium salts and the like. Examples of amines and organic amines include ammonium, methylamine, dimethylamine, trimethylamine, ethylamine, diethylamine, propylamine, butylamine, tetramethylamine, ethanolamine, diethanolamine, triethanolamine, meglumine, ethylenediamine, choline, N,N'-dibenzylethylenediamine, N-benzylphenylethylamine, N-methyl-D-glucamine, guanidine and the like. Examples of cationic amino acids include lysine, arginine, histidine and the like.

The pharmaceutically acceptable salts are prepared by reacting the compound of formula I with 1 to 4 equivalents of a base such as sodium hydroxide, sodium methoxide, sodium hydride, potassium t-butoxide, calcium hydroxide, magnesium hydroxide and the like, in solvents like ether, THF, methanol, t-butanol, dioxane, isopropanol, ethanol etc. Mixture of solvents may be used. Organic bases like lysine, arginine, diethanolamine, choline, guandine and their derivatives etc. may also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, acetic acid, citric acid, maleic acid salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, THF, dioxane etc. Mixture of solvents may also be used.

The stereoisomers of the compounds forming part of this invention may be prepared by using reactants in their single enantiomeric form in the process wherever possible or by conducting the reaction in the presence of reagents or catalysts in their single enantiomer form or by resolving the mixture of stereoisomers by conventional methods. Some of the preferred methods include use of microbial resolution, enzymatic resolution, resolving the diastereomeric salts formed with chiral acids such as mandelic acid, camphorsulfonic acid, tartaric acid, lactic acid, and the like wherever applicable or chiral bases such as brucine, (*R*)- or (*S*)-phenylethylamine, cinchona alkaloids and their derivatives and the like. Commonly used methods are compiled by Jaques et al in "Enantiomers, Racemates and Resolution" (Wiley Interscience, 1981). More specifically the compound of formula I may be converted to a 1:1 mixture of diastereomeric amides by treating with chiral amines, aminoacids, aminoalcohols derived from aminoacids; conventional reaction conditions may be employed to convert acid into an amide; the dia-stereomers may be separated either by fractional crystallization or chromatography and the stereoisomers of compound of formula I may be prepared by hydrolysing the pure diastereomeric amide.

Various polymorphs of compound of general formula I forming part of this invention may be prepared by crystallization of compound of formula I under different conditions. For example, using different solvents commonly used or their mixtures for recrystallization; crystallizations at different temperatures; various modes of cooling, ranging from very fast to very slow cooling during crystallizations. Polymorphs may also be obtained by heating or melting the compound followed by gradual or fast cooling. The presence of polymorphs may be determined by solid probe nmr spectroscopy, ir spectroscopy, differential scanning calorimetry, powder X-ray diffraction or such other techniques.

Prodrugs of the present compounds undergo chemical conversion by metabolic processes on administration before becoming active pharmacological substances. In general, such prodrugs will be functional derivatives of the present compounds, which are readily convertible in vivo into the required compound of the formula (I). Conventional procedures for the selection and preparation of suitable prodrug derivatives are described, for example, in "Design of Prodrugs", ed. H. Bundgaard, Elsevier, 1985.

The invention also relates to pharmaceutical compositions comprising, as an active ingredient, at least one compound of the formula I or any optical or geometric isomer or tautomeric form thereof including mixtures of these or a pharmaceutically acceptable salt thereof together with one or more pharmaceutically acceptable carriers or diluents.

Furthermore, the invention relates to the use of compounds of the general formula I or their tautomeric forms, their stereoisomers, their polymorphs, their pharmaceutically acceptable salts or pharmaceutically acceptable solvates thereof for the preparation of a pharmaceutical composition for the treatment and/or prevention of conditions mediated by nuclear receptors, in particular the Peroxisome Proliferator-Activated Receptors (PPAR) such as the conditions mentioned above.

In another aspect, the present invention relates to compounds for use in treating and/or preventing Type I or Type II diabetes.

In a still further aspect, the present invention relates to the use of one or more compounds of the general formula I or pharmaceutically acceptable salts thereof for the preparation of a pharmaceutical composition for the treatment and/or prevention of Type I or Type II diabetes.

In a still further aspect, the present compounds are useful for the treatment and/or prevention of IGT.

In a still further aspect, the present compounds are useful for the treatment and/or prevention of Type 2 diabetes.

In a still further aspect, the present compounds are useful for the delaying or prevention of the progression from IGT to Type 2 diabetes.

In a still further aspect, the present compounds are useful for the delaying or prevention of the progression from non-insulin requiring Type 2 diabetes to insulin requiring Type 2 diabetes.

In another aspect, the present compounds reduce blood glucose and triglyceride levels and are accordingly useful for the treatment and/or prevention of ailments and disorders such as diabetes and/or obesity.

In still another aspect, the present compounds are useful for the treatment and/or prophylaxis of insulin resistance (Type 2 diabetes), impaired glucose tolerance, dyslipidemia, disorders related to Syndrome X such as hypertension, obesity, insulin resistance, hyperglycaemia, atherosclerosis, hyperlipidemia, coronary artery disease, myocardial ischemia and other cardiovascular disorders.

In still another aspect, the present compounds are effective in decreasing apoptosis in mammalian cells such as beta cells of Islets of Langerhans.

In still another aspect, the present compounds are useful for the treatment of certain renal diseases including glomerulonephritis, glomerulosclerosis, nephrotic syndrome, hypertensive nephrosclerosis.

In still another aspect, the present compounds may also be useful for improving cognitive functions in dementia, treating diabetic complications, psoriasis, polycystic ovarian syndrome (PCOS) and prevention and treatment of bone loss, e.g. osteoporosis.

In yet another aspect, the invention also relates to the use of the present compounds, which after administration lower the bio-markers of atherosclerosis like, but not limited to, c-reactive protein (CRP), TNFα and IL-6.

The present compounds may also be administered in combination with one or more further pharmacologically active substances eg., selected from antiobesity agents, antidiabetics, antihypertensive agents, agents for the treatment and/or prevention of complications resulting from or associated with diabetes and agents for the treatment and/or prevention of complications and disorders resulting from or associated with obesity.

Thus, in a further aspect of the invention the present compounds may be administered in combination with one or more antiobesity agents or appetite regulating agents.

Such agents may be selected from the group consisting of CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators or TR β agonists.

In one embodiment of the invention the antiobesity agent is leptin.

In another embodiment the antiobesity agent is dexamphetamine or amphetamine.

In another embodiment the antiobesity agent is fenfluramine or dexfenfluramine.

In still another embodiment the antiobesity agent is sibutramine.

In a further embodiment the antiobesity agent is orlistat.

In another embodiment the antiobesity agent is mazindol or phentermine.

Suitable antidiabetics comprise insulin, GLP-1 (glucagon like peptide-1) derivatives such as those disclosed in WO 98/08871 to Novo Nordisk A/S, which is incorporated herein by reference as well as orally active hypoglycaemic agents.

The orally active hypoglycaemic agents preferably comprise sulphonylureas, biguanides, meglitinides, glucosidase inhibitors, glucagon antagonists such as those disclosed in WO 99/01423 to Novo Nordisk A/S and Agouron Pharmaceuticals, Inc., GLP-1 agonists, potassium channel openers such as those disclosed in WO 97/26265 and WO 99/03861 to Novo Nordisk A/S which are incorporated herein by reference, DPP-IV (dipeptidyl peptidase-IV) inhibitors, inhibitors of hepatic enzymes involved in stimulation of gluconeogenesis and/or glycogenolysis, glucose uptake modulators, compounds modifying the lipid metabolism such as antihyperlipidemic agents and antilipidemic agents as HMG CoA inhibitors (statins), compounds lowering food intake, RXR agonists and agents acting on the ATP-dependent potassium channel of the β-cells.

In one embodiment of the invention the present compounds are administered in combination with insulin.

In a further embodiment the present compounds are administered in combination with a sulphonylurea eg. tolbutamide, glibenclamide, glipizide or glicazide.

In another embodiment the present compounds are administered in combination with a biguanide eg. metformin.

In yet another embodiment the present compounds are administered in combination with a meglitinide eg. repaglinide or senaglinide.

In a further embodiment the present compounds are administered in combination with an α-glucosidase inhibitor eg. miglitol or acarbose.

In another embodiment the present compounds are administered in combination with an agent acting on the ATP-dependent potassium channel of the β-cells eg. tolbutamide, glibenclamide, glipizide, glicazide or repaglinide.

Furthermore, the present compounds may be administered in combination with nateglinide.

In still another embodiment the present compounds are administered in combination with an antihyperlipidemic agent or antilipidemic agent eg. cholestyramine, colestipol, clofibrate, gemfibrozil, fenofibrate, bezafibrate, tesaglitazar, EML-4156, LY-518674, LY-519818, MK-767, atorvastatin, fluvastatin, lovastatin, pravastatin, simvastatin, cerivastin, acipimox, ezetimibe, probucol, dextrothyroxine or nicotinic acid.

In yet another embodiment the present compounds are administered in combination with a thiazolidinedione e.g. troglitazone, ciglitazone, pioglitazone or rosiglitazone.

In a further embodiment the present compounds are administered in combination with more than one of the above-mentioned compounds eg. in combination with a sulphonylurea and metformin, a sulphonylurea and acarbose, repaglinide and metformin, insulin and a sulphonylurea, insulin and metformin, insulin, insulin and lovastatin, etc.

Furthermore, the present compounds may be administered in combination with one or more antihypertensive agents. Examples of antihypertensive agents are β-blockers such as alprenolol, atenolol, timolol, pindolol, propranolol and metoprolol, ACE (angiotensin converting enzyme) inhibitors such as benazepril, captopril, enalapril, fosinopril, lisinopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin. Further reference can be made to Remington: The Science and Practice of Pharmacy, 19th Edition, Gennaro, Ed., Mack Publishing Co., Easton, PA, 1995.

It should be understood that any suitable combination of the compounds according to the invention with one or more of the above-mentioned compounds and optionally one or more further pharmacologically active substances are considered to be within the scope of the present invention.

The present invention also relates to a process for the preparation of the above said novel compounds, their derivatives, their analogs, their tautomeric forms, their stereoisomers, their polymorphs, their pharmaceutically acceptable salts or pharmaceutically acceptable solvates.

### PHARMACEUTICAL COMPOSITIONS

The compounds of the invention may be administered alone or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19th Edition, Gennaro, Ed., Mack Publishing Co., Easton, PA, 1995. The compositions may appear in conventional forms, for example capsules, tablets, aerosols, solutions, suspensions or topical applications.

Typical compositions include a compound of formula I or a pharmaceutically acceptable acid addition salt thereof, associated with a pharmaceutically acceptable excipient which may be a carrier or a diluent or be diluted by a carrier, or enclosed within a carrier which can be in the form of a capsule, sachet, paper or other container. In making the compositions, conventional techniques for the preparation of pharmaceutical compositions may be used. For example, the active compound will usually be mixed with a carrier, or diluted by a carrier, or enclosed within a carrier which may be in the form of a ampoule, capsule, sachet, paper, or other container. When the carrier serves as a diluent, it may be solid, semi-solid, or liquid material which acts as a vehicle, excipient, or medium for the active compound. The active compound can be adsorbed on a granular solid container for example in a sachet. Some examples of suitable carriers are water, salt solutions, alcohols, polyethylene glycols, polyhydroxyethoxylated castor oil, peanut oil, olive oil, gelatine, lactose, terra alba, sucrose, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone. Similarly, the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents. The formulations of the invention may be formulated so as to provide quick, sustained, or delayed release of the active ingredient after administration to the patient by employing procedures well known in the art.

The pharmaceutical compositions can be sterilized and mixed, if desired, with auxiliary agents, emulsifiers, salt for influencing osmotic pressure, buffers and/or colouring substances and the like, which do not deleteriously react with the active compounds.

The route of administration may be any route, which effectively transports the active compound to the appropriate or desired site of action, such as oral, nasal, pulmonary, transdermal or parenteral e.g. rectal, depot, subcutaneous, intravenous, intraurethral, intramuscular, intranasal, ophthalmic solution or an ointment, the oral route being preferred.

If a solid carrier is used for oral administration, the preparation may be tabletted, placed in a hard gelatin capsule in powder or pellet form or it can be in the form of a troche or lozenge. If a liquid carrier is used, the preparation may be in the form of a syrup, emulsion, soft gelatin capsule or sterile injectable liquid such as an aqueous or non-aqueous liquid suspension or solution.

For nasal administration, the preparation may contain a compound of formula I dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

For parenteral application, particularly suitable are injectable solutions or suspensions, preferably aqueous solutions with the active compound dissolved in polyhydroxylated castor oil.

Tablets, dragees, or capsules having talc and/or a carbohydrate carrier or binder or the like are particularly suitable for oral application. Preferable carriers for tablets, dragees, or capsules include lactose, corn starch, and/or potato starch. A syrup or elixir can be used in cases where a sweetened vehicle can be employed.

A typical tablet which may be prepared by conventional tabletting techniques may contain:

| Core: | |
|---|---|
| Active compound (as free compound or salt thereof) | 5 mg |
| Colloidal silicon dioxide (Aerosil) | 1.5 mg |
| Cellulose, microcryst. (Avicel) | 70 mg |
| Modified cellulose gum (Ac-Di-Sol) | 7.5 mg |
| Magnesium stearate | Ad. |
| | |

| Coating: | |
|---|---|
| HPMC approx. | 9 mg |
| *Mywacett 9-40 T approx. | 0.9 mg |

| | |
|---|---|
| *Acylated monoglyceride used as plasticizer for film coating. | |

If desired, the pharmaceutical composition of the invention may comprise the compound of formula (I) in combination with further pharmacologically active substances such as those described in the foregoing.

The compounds of the invention may be administered to a mammal, especially a human in need of such treatment, prevention, elimination, alleviation or amelioration of diseases related to the regulation of blood sugar.

Such mammals include also animals, both domestic animals, e.g. household pets, and non-domestic animals such as wildlife.

The compounds of the invention are effective over a wide dosage range. A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain of from 0.05 to about 1000 mg, preferably from about 0.1 to about 500 mg, and more preferred from about 0.5 mg to about 200 mg.

Any novel feature or combination of features described herein is considered essential to this invention.

### EXAMPLES

The following examples and general procedures refer to intermediate compounds and final products identified in the specification and in the synthesis schemes. The preparation of the compounds of the present invention is described in detail using the following examples. Occasionally, the reaction may not be applicable as described to each compound included within the disclosed scope of the invention. The compounds for which this occurs will be readily recognised by those skilled in the art. In these cases the reactions can be successfully performed by conventional modifications known to those skilled in the art, that is, by appropriate protection of interfering groups, by changing to other conventional reagents, or by routine modification of reaction conditions. Alternatively, other reactions disclosed herein or otherwise conventional will be applicable to the preparation of the corresponding compounds of the invention. In all preparative methods, all starting materials are known or may easily be prepared from known starting materials. The structures of the compounds are confirmed nuclear magnetic resonance (NMR). NMR shifts (δ) are given in parts per million (ppm. Mp is melting point and is given in °C.

The abbreviations as used in the examples have the following meaning:
- THF:: tetrahydrofuran
- DMSO:: dimethylsulfoxide
- CDCl₃:: deutorated chloroform
- DMF:: N,N-dimethylformamide
- min:: minutes
- h:: hours

### General procedure (A)

### Step A:

Reacting a compound of formula (II) 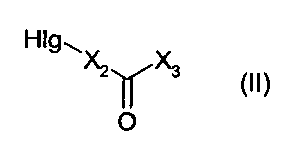 wherein X₂ and X₃ are defined as above and wherein Hlg is bromine or iodine, through a Wittig-like process with for example (EtO)₂PO(CHR₁)COOR₆, wherein R₆ is an alkyl group and R₁ is defined as above, in the presence of a base such as sodium hydride, EtONa and the like to give a compound of formula (III) 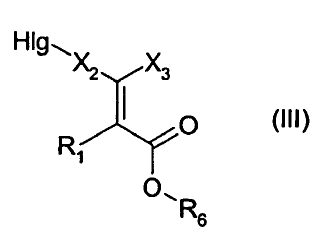 wherein X₂, X₃, R₁ and R₆ are defined as above and wherein Hlg is bromine or iodine, and

### Step B:

Reducing the compound of formula (III) wherein X₂, X₃, R₁ and R₆ are defined as above and wherein Hlg is bromine or iodine with a suitable reagent such as diisobutylaluminium hydride, to give a compound of formula (IV) 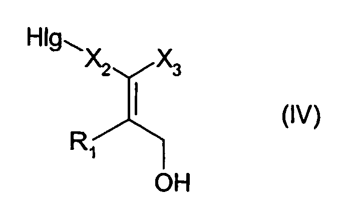 wherein X₂, X₃ and R₁ are defined as above and wherein Hlg is bromine or iodine, and

### Step C:

Reacting the compound of formula (IV), wherein X₂, X₃ and R₁ are defined as above and wherein Hlg is bromine or iodine, (except that when X₂ or X₃ are substituted with hydroxy, this functionality has to be protected) with a compound of formula (V) 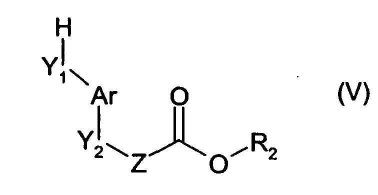 wherein Y₁, Ar, Y₂, Z and R₂ are defined as above, except that R₂ is not hydrogen under Mitsunobu conditions, using a reagent such as triphenylphosphine/diethylazodicarboxylate and the like to obtain a compound of formula (VI) 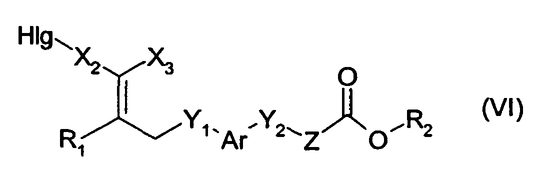 wherein X₂, X₃, Y₁, Y₂, Ar, Z, R₁ and R₂ are defined as above, except that R₂ is not hydrogen and wherein Hlg is bromine or iodine, and

### Step D:

Reacting a compound of formula (VI) wherein X₂, X₃, Y₁, Y₂, Ar, Z, R₁ and R₂ are defined as above, and wherein HIg is bromine or iodine, with an boronic acid or with a tributyltin derivative of X₁ under appropriate coupling conditions as Pd2(dba)₃/Pd(P(t-Bu)₃)₂/KF/THF, to give a compound of formula (I), wherein X₁, X₂, X₃, Y₁, Y₂, Ar, Z, R₁ and R₂ are defined as above, except that R₂ is not hydrogen.

### General procedure (B)

### Step A:

By chemical or enzymatic saponification of a compound of formula (I) wherein X₁, X₂, X₃, Y₁, Y₂, Ar, Z, R₁ and R₂ are defined as above, except that R₂ is not hydrogen to give a compound of formula (I) wherein X₁, X₂, X₃, Y₁, Y₂, Ar, Z, R₁ and R₂ are defined as above, except that R₂ is hydrogen.

### General procedure (C)

### Step A:

By chemical or enzymatic saponification of a compound of formula (VI) wherein X₂, X₃, Y₁, Y₂, Ar, Z, R₁ and R₂ are defined as above, except that R₂ is not hydrogen and wherein HIg is bromine or iodine to give a compound of formula (VI) wherein X₁, X₂, X₃, Y₁, Y₂, Ar, Z, R₁ and R₂ are defined as above, except that R₂ is hydrogen and wherein HIg is bromine or iodine, and

### Step B:

Reacting an compound of formula (VI) wherein X₂, X₃, Y₁, Y₂, Ar, Z, R₁ and R₂ are defined as above, except that R₂ is hydrogen and wherein Hlg is bromine or iodine, with an boronic acid derivative of X₁ under appropriate coupling conditions as Pd2(dba)₃/Pd(P(t-Bu)₃)₂/KF/THF, to give a compound of formula (I), wherein X₁, X₂, X₃, Y₁, Y₂, Ar, Z, R₁ and R₂ are defined as above, except that R₂ is hydrogen.

### General procedure (D)

### Step A:

Reacting a compound of formula (VII) 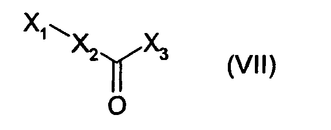 wherein X₁, X₂ and X₃ are defined as above, through a Wittig-like process with for example (EtO)₂PO(CHR₁)COOR₆, wherein R₆ is an alkyl group and R₁ is defined as above, in the presence of a base such as sodium hydride, EtONa and the like to give a compound of formula (VIII) 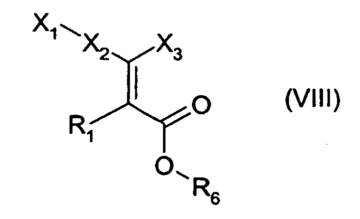 wherein X₁, X₂, X₃, R₁ and R₆ are defined as above, and

### Step B:

Reducing the compound of formula (VIII) wherein X₁, X₂, X₃, R₁ and R₆ are defined as above, with a suitable reagent such as diisobutylaluminium hydride, to give a compound of formula (IX) 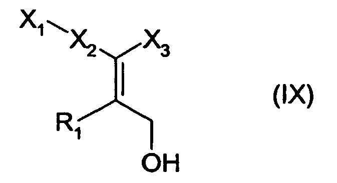 wherein X₁, X₂, X₃ and R₁ are defined as, and

### Step C:

Reacting the compound of formula (IX), wherein X₁, X_{2,} X₃ and R₁ are defined as above, (except that when X₁, X₂ or X₃ are substituted with hydroxy, this functionality has to be protected) with a compound of formula (V) wherein Y₁, Ar, Y₂, Z and R₂ are defined as above, except that R₂ is not hydrogen under Mitsunobu conditions, using a reagent such as triphenylphosphine/diethylazodicarboxylate and the like to obtain a compound of formula (I), wherein X₁, X₂, X₃, Y₁, Y₂, Ar, Z, R₁ and R₂ are defined as above, except that R₂ is not hydrogen.

### General procedure (E)

### Step A:

Converting the -OH functionality in the compound of formula (IX), wherein X₁, X₂, X₃ and R₁ are defined as above, to an appropriate leaving group (L) such as p-toluenesulfonate, methanesulfonate, halogen (for example by methods according to: Houben-Weyl, Methoden der organischen Chemie, Alkohole III, 6/1b, Thieme-Verlag 1984, 4th Ed., pp. 927-939; Comprehensive Organic Transformations. A guide to functional group preparations, VCH Publishers 1989, 1s1 Ed., pp. 353-363 and J. Org. Chem., Vol. 36 (20), 3044-3045, 1971), triflate and the like, to give a compound of formula (X) 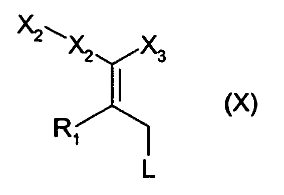 wherein, X₁, X₂, X₃, R₁ and L are defined as above.

### Step B:

Reacting the compound of formula (X) wherein L is a leaving group such as p-toluenesulfonate, methanesulfonate, halogen, triflate and the like and wherein X₁, X₂, X₃ and R₁ are defined as above, with a compound of formula (V) wherein Y₁, Ar, Y₂, Z and R₂ are defined as above, except that R₂ is not hydrogen, to give a compound of formula (I) wherein X₁, X₂, X₃, Y₁, Y₂, Ar, Z, R₁ and R₂ are defined as above, except that R₂ is not hydrogen.

### General procedure (F)

### Step A:

Reacting a compound of formula (XI) 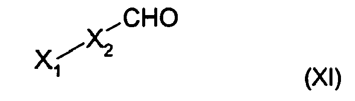 wherein X₁ and X₂ are as defined above, with carbon tetrabromide and triphenylphosphine to give a compound of formula (XII) 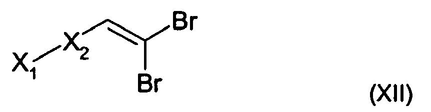 wherein X₁ and X₂ are as defined above.

### Step B:

Reacting the compound of formula (XII), wherein X₁ and X₂ are as defined above, with paraformaldehyde in the presence of a strong base like BuLi, to give a compound of formula (XIII) 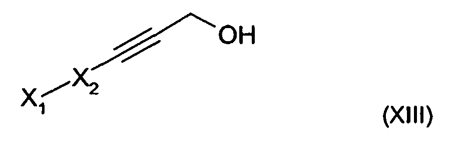 wherein X₁ and X₂ are as defined above.

### Step C:

Reducing the compound of formula (XIII), wherein X₁ and X₂ are as defined above, with LiAIH in the presence of a base, like sodium methoxide, followed by treatment with dimethylcarbonate and iodine to give a compound of formula (XIV) 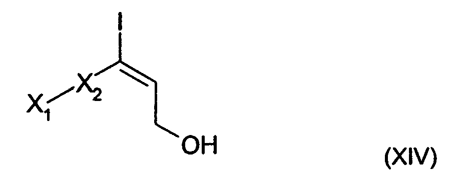 wherein X₁ and X₂ are as defined above.

### Step D:

Converting the hydroxyl function in the compound of formula (XIV) to a leaving group (L), as described under the General procedure B, to give a compound of formula (XV) 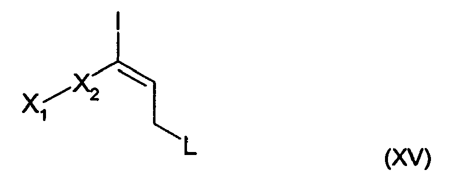 wherein X₁, X₂ and L are as defined above.

### Step E:

Reacting the compound of formula (XV), wherein L is a leaving group, such as p-toluenesulfonate, methanesulfonate, halogen, triflate and the like, and wherein X₁ and X₂ are as defined above, with the compound of formula (V), wherein Y₁, Ar, Y₂, Z and R₂ are as defined above, except that R₂ is not hydrogen, to give a compound of formula (XVI) 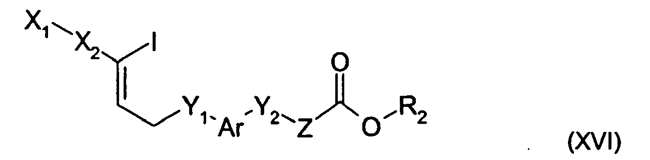 wherein X₁, X₂, Y₁, Y₂, Ar, Z and R₂ are as defined above, except that R₂ is not hydrogen.

### Step F:

Reacting the compound of formula (XVI), wherein X₁, X₂, Y₁, Y₂, Ar, Z and R₂ are defined as above, except that R₂ is not hydrogen, with X₂-tributyltin in the presence of a palladium catalyst, like Pd₂(dba)₃, and tri(t.-butyl)phosphine to give the compound of formula (I), wherein X₁, X₂, X₃, Y₁, Y₂, Ar, Z, R₁ and R₂ are defined as above, except that R₁ is hydrogen and R₂ is not hydrogen.

### General procedure (G)

### Step A:

Reacting a compound of formula X₁-X₂-halogen, wherein X₁ and X₂ are as defined above, under Heck like conditions with propargylalcohol in the presence of a palladium catalyst, like Pd₂(dba)₃, and cubber(I) to give the compound of formula (XIII), wherein X₁ and X₂ are as defined above

### General procedure (H)

### Step A:

Reacting the compound of formula (XIV), wherein X₁ and X₂ are defined as above, with X₃-tributyltin in the presence of a palladium catalyst, like Pd₂(dba)₃, and tri(t.-butyl)phosphine to give a compound of formula (XVII) 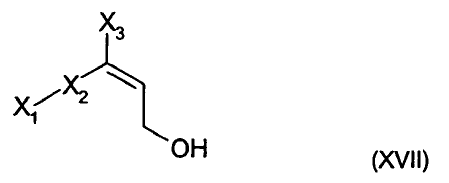 wherein X, X₂ and X₃, are defined as above.

Using a combination of the above methods, or methods analogous hereof, various compounds may be made within the scope of the present invention.

The present invention is further exemplified by the following examples, which illustrate the preparation of the compounds according to the invention. The examples are, however, not intended to limit the scope of the invention in any way.

### Example 1

### (E/Z) {4-[3-Biphenyl-4-yl-3-(4-bromo-phenyl)-allylsulfanyl]-phenoxy}-acetic acid

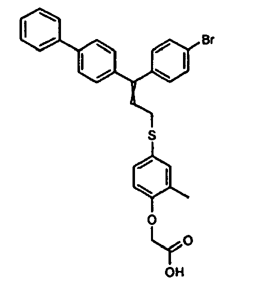

### General procedure A:

### Step A:

To a solution of NaH (3.53 g, 88.2 mmol) in dry toluene (300 ml) was added dropwise at 0 °C a solution of trietylphosphonoacetate (13.2 g, 58.8 mmol) in toluene (100 ml). The reaction mixture was stirred for 30 min. after which a solution of 4,4-dibromobenzophenone (10.0 g, 29.4 mmol) in THF (100 ml) was added. The reaction mixture was stirred for 48 h. Ethanol (10 ml) and water (300 ml) were added and the mixture was extracted with ethyl acetate-methanol (2%, 2x150 ml). The combined organic phases were washed with brine, dried with MgSO₄, filtered and evaporated. The residue was purified by column chromatography (eluent: ether) to give ethyl 3,3-bis-(4-bromophenyl)-acrylate as a gum. Crystallization from hexanes gave white crystals in 8.77 g (73%) yield.
¹H NMR (CDCl₃, 300 MHz); δ 1.20 (3H, t), 4.05 (2H, q), 6.35 (1H, s), 7.0-7.1 (4H, m), 7.40-7.52 (4H, m).

### Step B:

Ethyl 3,3-bis-(4-bromophenyl)-acrylate (8.75 g, 21.3 mmol) was dissolved in dry THF (35 ml). DIBAL-H (1.5 M in toluene, 43 ml, 64.0 mmol) was added at -15°C and the reaction mixture was stirred for 30 min. A solution of ammonium chloride in water was added and the mixture was extracted with ethyl acetate (3x50 ml). The combined organic phases were washed with brine, dried with MgSO₄, filtered and evaporated to give 3,3-bis-(4-bromophenyl)-pro-2-en-1-ol in 6.0 g (76%) yield.
¹H NMR (CDCl₃, 300 MHz); δ 1.15 (1H, br s), 4.16-4.20 (2H, dd), 6.25 (1H, t), 7.0-7.1 (4H, m), 7.40-7.52 (4H, m).

### Step C:

3,3-Bis-(4-bromophenyl)-pro-2-en-1-ol (2.98 g, 8.1 mmol) and tributylphosphine (2.4 g, 12.1 mmol) were dissolved in dry THF (150 ml) and cooled to 0 °C under an atmosphere of nitrogen. 1,1'-(Azodicarbonyl)dipiperidine (ADDP) (3.1 g, 12.1 mmol) was added and the reaction mixture was stirred for 5 min. (4-Mercapto-2-methyl-phenoxy)-acetic acid methyl ester (2.06 g, 9.7 mmol) was slowly added (5 min) and the stirring continued for 2 h at 0 °C. Water (100 ml) was added and the mixture was extracted with dichloromethane (2x150 ml). The combined organic phases were dried with MgSO₄, filtered and evaporated. The residue was purified by column chromatography (eluent: dichloromethane) to give 4.0 g (88%) of {4-[3,3-bis-(4-bromo-phenyl)-allylsulfanyl]-2-methyl-phenoxy}-acetic acid methyl ester.
¹H NMR (CDCl₃, 300 MHz); δ 2.20 (3H, s), 3.44 (2H, d), 3.78 (3H, s), 4.64 (2H, s), 6.11 (1H, t), 6.55 (1H, d), 6.73 (2H, d), 6.98 (2H, d), 7.10 (2H, bs), 7.38 (2H, d), 7.43 (2H, d).

### General procedure C:

### Step A:

A solution of {4-[3,3-bis-(4-bromo-phenyl)-allylsulfanyl]-2-methyl-phenoxy}-acetic acid methyl ester (530 mg, 0.94 mmol) in ethanol (20 ml) and 1 M NaOH (2.0 ml, 2.0 mmol) was stirred at room temp. for 2 h. The reaction mixture added water (20 ml) and 1 N HCl (3.0 ml). The water phase was extracted dichloromethane (2x50 ml) and the combined organic phases dried with MgSO₄, filtered and evaporated to give 482 mg (93%) of {4-[3,3-bis-(4-bromophenyl)-allylsulfanyl]-2-methyl-phenoxy}-acetic acid.
¹H NMR (CDCl₃, 300 MHz); δ 2.20 (3H, s), 3.45 (2H, d), 4.68 (2H, s), 6.10 (1H, t), 6.58 (1H, d), 6.75 (2H, d), 6.98 (2H, d), 7.10-7.13 (2H, m), 7.38 (2H, d), 7.43 (2H, d).

### Step B:

A mixture of {4-[3,3-bis-(4-bromophenyl)-allylsulfanyl]-2-methyl-phenoxy}-acetic acid (97 mg, 0.177 mmol), phenylboronic acid (47 mg, 389 mmol), KF (34 mg, 0.584 mmol), Pd₂(dba)₃ (10 mg, 0.011 mmol) and Pd(P(t-Bu)₃)₂ (11 mg, 0.021 mmol) was evacuated for air and kept under nitrogen. THF (2 ml) was added and the reaction mixture was stirred at room temperature for 4 hours. A saturated aqueous NH₄Cl (5 ml) solution was added, and the mixture was extracted with methylene chloride (2 x 20 ml). The combined organic phases were dried and purified on column chromatography using methylene chloride: THF (8:3) as eluent. The isolated products were further purified on HPLC using acetonitril:water (4:6) increasing to pure acetonitril as eluent. The title product was isolated as an E/Z mixture in 4 mg yield. ¹H NMR (CDCl₃, 400 MHz); δ 7.60-6.75 (m, 15H); 6.47 (d, J= 7 Hz, 1H); 6.16 (t, J= 7Hz, 0.7H); 6.07 (t, J= 7 Hz, 0.3H); 4.37 (s, 2H); 3.53 (d, J= 7 Hz, 0.6H); 3.45 (d, J= 7 Hz, 1.4H); 2.07 (s, 3H).

### Example 2

### (E/Z) {4-[3-(4-Bromo-phenyl)-3-(3'-trifluoromethyl-biphenyl-4-yl)-allylsulfanyl]-2-methylphenoxy}-acetic acid.

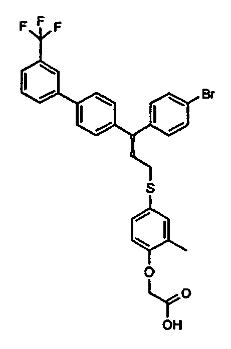

### General procedure C:

### Step B:

A mixture of {4-[3,3-bis-(4-bromo-phenyl)-allylsulfanyl]-2-methyl-phenoxy}-acetic acid (described above) (231 mg, 0.421 mmol), 3-(trifluoromethyl)phenylboronic acid (203 mg, 1.07 mmol), KF (81 mg, 1.39 mmol), Pd₂(dba)₃ (23 mg, 0.025 mmol) and Pd(P(t-Bu)₃)₂ (26 mg, 0.051 mmol) was evacuated for air and kept under nitrogen. THF (5 ml) was added and the reaction mixture was stirred at 50°C overnight. A saturated aqueous NH₄Cl (5 ml) solution was added, and the mixture was extracted with methylene chloride (2 x 20 ml). The combined organic phases were dried an evaporated. The isolated products were further purified on HPLC using acetonitril:water (4:6) increasing to pure acetonitril as eluent. The title product was isolated as an E/Z mixture in 95 mg (37%) yield.
¹H NMR (CDCl₃, 400 MHz); δ 7.87-7.00 (m, 13H), 6.84 (d, J= 8 Hz, 1H), 6.60 (d, J= 8 Hz, 1H), 6.21 (t, J= 6 Hz, 0.3H), 6.14 (t, J= 6 Hz, 0.6H), 4.66 (s, 2H), 3.55 (d, J= 7 Hz, 1.4H), 3.51 (d, J= 7 Hz, 0.6H), 2.21 (s, 1H), 2.19 (s, 2H).

### Example 3

### {4-[3-(4-Bromo-phenyl)-3-[1,1';4',1 "]terphenyl-4"-yl-allylsulfanyl]-2-methyl-phenoxy}-acetic acid

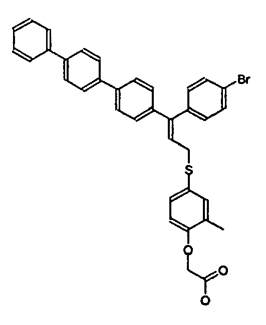

### General procedure C:

### Step B:

A mixture of {4-[3,3-bis-(4-bromo-phenyl)-allylsulfanyl]-2-methyl-phenoxy}-acetic acid (example 1) (225 mg, 0.410 mmol), 4-biphenylboronic acid (163 mg, 0.82 mmol), KF (79 mg, 1.35 mmol), Pd₂(dba)₃ (23 mg, 0.025 mmol) and Pd(P(t-Bu)₃)₂ (25 mg, 0.049 mmol) was evacuated for air and kept under nitrogen. THF (6 ml) was added and the reaction mixture was stirred at 70°C overnight. A saturated aqueous NH₄Cl (5 ml) solution was added and the mixture was extracted with methylene chloride (2 x 20 ml). The combined organic phases were dried an evaporated. The isolated products were further purified on column chromatography using methylene chloride:THF (40:1) as eluent. The isolated products were further purified on HPLC using acetonitril:water (7:3) increasing to pure acetonotril as eluent. The title product was isolated as an E/Z mixture in 40 mg (16%) yield.
¹H NMR (CDCl₃, 400 MHz); δ 7.70-6.97 (m, xxH), 6.83 (d, 1H), 6.57 (d, 1H), 6.20 (t, 1/3 H), 6.12 (t, 2/3 H), 4.64 (s, 2/3 H), 4.63 (s, 4/3 H), 3.57 (d, 4/3), 3.47 (d, 2/3 H), 2.18 (s, 2/3 H), 2.17 (s, 4/3 H).

### Example 4 (General procedure (A))

### (E/Z)-[2-Methyl-4-[3-[5-(5-methylthiophen-2-yl)benzo[b]furan-2-yl]-3-(thiophen-2-yl)ally-Isulfanyl]phenoxy]acetic acid

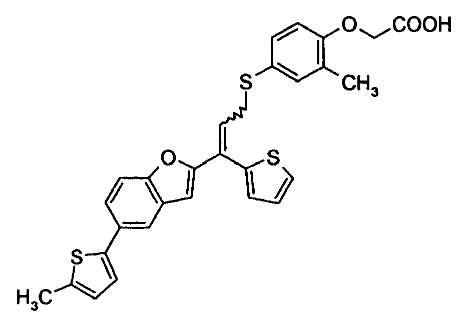

Potassium carbonate (12.0 g, 0.087 mol) and subsequently 2-bromo-1-(thiophen-2-yl)ethanone (7.0 g, 0.034 mol; prepared as described in J.Med.Chem. 30, 1497 (1987)) were added to a stirred solution of 5-bromosalicylaldehyde (6.9 g, 0.034 mol) in acetone (150 mL). The mixture was stirred at ambient temperature for 30 min at first and then refluxed for 1 h. Solid mass was filtered off, washed with hot acetone (2x50 mL) and the filtrate was evaporated *in vacuo.* The residue (11.3 g) was crystallized from ethanol (15 mL) giving (5-bromobenzo[b]furan-2-yl)-(thiophen-2-yl)methanone. Yield: 8.0 g (77%). M.p. 84-86 °C.
R_{F} (SiO₂, hexane/ethyl acetate 3:1) 0.70.

### Step A:

In atmosphere of nitrogen, 2M solution of lithium diisopropylamide in tetrahydrofuran/ heptane/ethylbenzene (33 mL, 66.0 mmol) was added dropwise to an ice-water cooled solution of triethyl phosphonoacetate (12 mL, 60.0 mmol) in tetrahydrofuran (180 mL). The mixture was stirred at ambient temperature for 30 min, a solution of the above methanone (9.2 g, 30.0 mmol) in tetrahydrofuran (92 mL) was added dropwise and the whole mixture was stirred at ambient temperature for 39 h. The mixture was diluted with dichloromethane (150 mL), washed with water (150 mL) and the aqueous layer was extracted with dichloromethane (100 mL). The combined organic layers were washed with water (200 mL), brine (200 mL), dried over anhydrous magnesium sulfate and evaporated *in vacuo.* Purification by column chromatography (silica gel Fluka 60, hexane/ethyl acetate 9:1) of the obtained residue gave (E/Z)-3-(5-bromobenzo[b]furan-2-yl)-3-(thiophen-2-yl)acrylic acid ethyl ester as an yellow oil.
Yield: 8.0 g (71%). R_{F} (SiO₂, hexane/ethyl acetate 9:1) 0.30.

### Step B:

In atmosphere of nitrogen, a solution of anhydrous aluminum chloride (1.03 g, 7.71 mmol) in dry ether (39 mL) was added dropwise to a suspension of lithium aluminum hydride (0.88 g, 23.1 mmol) in dry ether (74 mL) at -15°C. The mixture was stirred for 30 min allowing the reaction temperature to reach 0 °C. The suspension was cooled at -15°C again, a solution of the above ester (2.90 g, 7.71 mmol) in dry ether (39 mL) was added dropwise and the resulting mixture was stirred for 1 h under cooling. Water (0.6 mL), 10% aqueous solution of sodium hydroxide (0.6 mL) and water (1.8 mL) were added dropwise to the cold mixture; the segregated precipitate was filtered off and washed with ether (70 mL). The combined ethereal layers were washed with water (2x50 mL), brine (2x50 mL), dried over anhydrous magnesium sulfate and evaporated *in vacuo.* The obtained crude product was purified by column chromatography (silica gel Fluka 60, hexane/ethyl acetate 4:1) yielding (E/Z)-3-(5-bromobenzo[b]furan-2-yl)-3-(thiophen-2-yl)prop-2-en-1-ol as a white crystalline solid.
Yield: 1.61 g (62%). R_{F} (SiO₂, hexane/ethyl acetate 4:1) 0.30.
¹H NMR spectrum of the main isomer (250 MHz, CDCl₃): 7.71 (dd, J=1.8 and 0.7 Hz, 1H); 7.43 (dd, J=8.8 and 1.9 Hz, 1H); 7.37 (dm, J=8.8 Hz, 1H); 7.29 (dd, J=5.1 and 1.2 Hz, 1H); 7.10 (m, 1H); 7.03 (m, 1H); 6.76 (s, 1H); 6.35 (t, J=6.6 Hz, 1H); 4.60 (d, J=6.6 Hz, 2 H).

### Procedure analogues to "General procedure E, Step A-B")

In atmosphere of nitrogen, tetrabromomethane (1.48 g, 4.46 mmol) was added to an ice-cooled solution of the above hydroxy derivative (1.00 g, 2.98 mmol) and triphenylphosphine (1.25 g, 4.77 mmol) in dry methylene chloride (40 mL). The reaction mixture was stirred for 2 h at ambient temperature, quickly filtered through a short path of silica gel and the filtrate was evaporated *in vacuo.* In atmosphere of nitrogen, tetrahydrofuran (38 mL), N,N-diisopropylethylamine (0.94 mL, 5.40 mmol) and a solution of ethyl (4-mercapto-2-methylphenoxy)acetate (1.27 g, 5.61 mmol) in tetrahydrofuran (2 mL) were added to the residue. The reaction mixture was stirred overnight, filtered, the precipitated solid was washed with tetrahydrofuran (10 mL) and the collected organic solutions were evaporated *in vacuo.* The residue was purified by column chromatography (silica gel Merck 60, hexane/ethyl acetate 15:1) yielding (E/Z)-[4-[3-(5-bromobenzo[b]furan-2-yl)-3-(thiophen-2-yl)allylsulfanyl]-2-methylphenoxy]acetic acid ethyl ester.
Yield: 1.4 g (87%). R_{F} (SiO₂, hexane/ ethyl acetate 4:1) 0.50.
¹H NMR spectrum (250 MHz, CDCl₃): 7.60-6.27 (m, ~9 H); 6.73 and 6.22 (t, J=8.3 Hz, 1H); 6.59 and 6.46 (d, J=8.3 Hz, 1H); 4.60 and 4.53 (s, 2 H); 4.24 and 4.12 (q, J=7.2 Hz, 2 H); 3.86 and 3.62 (d, J=8.3 Hz, 2 H); 2.21 and 2.08 (s, 3 H); 1.28 and 1.27 (t, J=7.2 Hz, 3 H).

### General procedure A:

### Step D:

To a solution of (E/Z)-[4-[3-(5-bromobenzo[b]furan-2-yl)-3-(thiophen-2-yl)allylsulfanyl]-2-methylphenoxy]acetic acid ethyl ester (330 mg, 0.607 mmol) and tributyl-(5-methylthiophen-2-yl)tin (250 mg, 0.644 mmol, prepared in 69% yield according to J. Med. Chem. 44, 3355 (2001)) in dry N,N-dimethylformamide (15 mL) tris(dibenzylideneacetone) - dipalladium chloroform complex (19.5 mg, 0.019 mmol) was added. Traces of moisture and oxygen were removed and 0.20M solution of tri(tert.butyl)phosphine in cyclohexane (0.4 mL, 0.080 mmol) was added under atmosphere of nitrogen and the whole mixture was stirred at 50 °C for 3 h. The mixture was diluted with ethyl acetate (50 mL), washed with water (40 mL), brine (40 mL), 10% aqueous solution of potassium fluoride (40 mL) and brine (50 mL). The organic solution was dried with anhydrous sodium sulfate and its evaporation gave an oil that was purified by column chromatography (silica gel Fluka 60, hexane/ethyl acetate 16:1) yielding (E/Z)-[2-methyl-4-[3-[5-(5-methylthiophen-2-yl)benzo[b]furan-2-yl]-3-(thiophen-2-yl)allylsulfanyl]phenoxy]acetic acid ethyl ester as an yellow oil.
Yield: 256 mg (75%). R_{F} (SiO₂, hexane/ethyl acetate 16:1). 0.30.
¹H NMR spectrum (250 MHz, CDCl₃): 7.68-6.30 (m, ~11 H); 6.21 (t, J=8.3 Hz, 1 H); 6.59 and 6.47 (d, J=8.3 Hz, 1 H); 4.60 and 4.52 (s, 2 H); 4.23 and 4.12 (q, J=7.2 Hz, 2 H); 3.91 and 3.63 (d, J=8.2 Hz, 2 H); 2.51 and 2.49 (s, 3 H); 2.22 and 2.09 (s, 3 H); 1.26 and 1.25 (t, J=7.2 Hz, 3 H).

### General procedure B:

### Step A:

To an ice-water cooled solution of the above ester (144 mg, 0.257 mmol) in a mixture tetrahydrofuran/methanol/water (5:1:1, 7 mL) lithium hydroxide monohydrate (16 mg, 0.381 mmol) was added. The resulting solution was stirred for 70 min under cooling and subsequently a diluted solution of tartaric acid (5 mL) was added followed by addition of ether (20 mL). The layers were separated, the aqueous layer was extracted with ether (10 mL) and the combined ethereal layers were washed with water (3x10 mL) and brine (2x10 mL) and dried with anhydrous sodium sulfate. The oil obtained by evaporation of the organic solution was purified by column chromatography (silica gel Fluka 60, chloroform/methanol 99:1-98:2) yielding the title compound. Yield: 25 mg (18%). M.p. ― (foam). R_{F} (SiO₂, methylene chloride/methanol 85:15) 0.25.

L-Lysine (6 mg, 0.041 mmol) was added to a solution of the above acid (25 mg, 0.047 mmol) in dry methanol (3 mL). The mixture was stirred for 2 h, evaporated *in vacuo* and the residue twice triturated with anhydrous ether yielding the L-lysinate of the title acid. Yield: 20 mg (63%). M.p. 142-161 °C (amorphous).
¹H NMR spectrum (250 MHz, DMSO-d₆): 7.81-6.21 (m, ~13 H); 4.38 and 4.34 (s, 2 H); 3.88 and 3.68 (d, ~2 H); 3.16 (m, ~1 H); 2.75 (m, 2 H); 2.47 and 2.45 (d, ~3 H); 2.10 and 1.96 (s, 3 H); 1.70-1.25 (m, ~6 H).

### Example 5 (General procedure (F))

### (E/Z)-[4-[3-(Biphenyl-4-yl)-3-(furan-2-yl)allylsulfanyl]-2-methylphenoxy]acetic acid

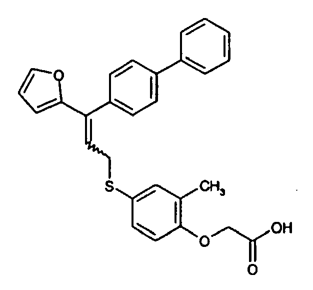

### Step A:

Tetrabromomethane (21.8 g, 166 mmol) was added to a cooled solution of biphenyl-4-carbaldehyde (10.0 g, 54.9 mmol) and triphenylphosphine (35.5 g, 131.8 mmol) in dry methylene chloride (100 mL). Reaction mixture was stirred for 3 h at ambient temperature and saturated solution of sodium hydrogen carbonate (50 mL) was added. The organic layer was washed with water (50 mL), dried with anhydrous magnesium sulfate and subsequently evaporated *in vacuo.* The crude product was twice re-crystallized from methanol giving 1,1-dibromo-2-(4-biphenylyl)ethene as a white solid.
Yield: 14.9 g (80%). R_{F} (SiO₂, hexane) 0.80.

### Step B:

The above bromo derivative (3.0 g, 8.9 mmol) was dissolved in dry tetrahydrofuran (100 mL) and under inert atmosphere cooled to -78°C. 2M Solution of n-butyllithium (12 mL, 22 mmol) was added dropwise to the solution and the reaction mixture was stirred for 2 h under cooling. Finely powdered paraformaldehyde (0.7 g, 22 mmol) was added to the mixture and it was stirred for 3h at -60°C slowly increasing the reaction temperature to ambient temperature. Brine (50 mL) was added and reaction mixture was extracted with ether (4x50 mL). The collected organic layers were dried with anhydrous magnesium sulfate and subsequently evaporated in vacuo. The residue was purified by column chromatography (silica gel Merck 60, hexane /ethyl acetate 1:0 - 3:1) yielding 3-(4-biphenylyl)-prop-2-yn-1-ol.
Yield: 4.1 g (74%). R_{F} (SiO₂, hexane/ethyl acetate 4:1) 0.45.
¹H NMR spectrum (250 MHz, CDCl₃): 7.63-7.30 (m, 9 H); 4.52 (s, 2 H).

### Step C:

A solution of the above alkyne (1.0 g, 4.8 mmol) in dry tetrahydrofuran (20 mL) was added dropwise to an ice-cooled solution of lithium aluminum hydride (380 mg, 10 mmol) and sodium methoxide (10 mg, 250 µmol, 5%) in dry tetrahydrofuran (10 mL) under inert atmosphere. The reaction mixture was stirred for 3 h, a solution of dimethyl carbonate (900 mg, 10 mmol) in dry tetrahydrofuran (10 mL) was added dropwise at 0°C and the reaction mixture was stirred for further 2 h. A solution of iodine (2.5 g, 10 mmol) in tetrahydrofuran (10 mL) was added and the resulting mixture was allowed to stand overnight in fridge. Methanol (5 mL) was added and reaction mixture was stirred for 0.5 h. Saturated aqueous solution of sodium thiosulfate (25 mL) and brine (100 mL) were added and the heterogenous mixture was extracted with ether (4x100 mL). The collected organic solutions were dried with anhydrous magnesium sulfate and subsequently evaporated *in vacuo.* The residue was purified by column chromatography (silica gel Merck 60, hexane/methylene chloride 9:1 - methylene chloride/methanol 3:1) yielding (Z)-3-(4-biphenylyl)-3-iodoprop-2-en-1-ol.
Yield: 1.3g (80%). R_{F} (SiO₂, hexane/ethyl acetate 4:1) 0.50.

### Step D-E:

A solution of tetrabromethane (1.0 g, 3.0 mmol) in dry methylene chloride (20 mL) was added dropwise to an ice-cooled solution of the above hydroxy derivative (0.6 g, 2.0 mmol) and triphenylphosphine (0.8 g, 3.0 mmol) in dry methylene chloride (50 mL). The reaction mixture was stirred for 2 h at ambient temperature, N,N-diisopropylethylamine (250 mg, 2 mmol) and a drop of water were added, the solution was stirred for further 0.5 h and evaporated *in vacuo.* In atmosphere of nitrogen, tetrahydrofuran (25 mL), N,N-diisopropylethylamine (390 µL, 3.0 mmol) and ethyl (4-mercapto-2-methylphenoxy)acetate (560 mg, 2.5 mmol) were added to the residue. The reaction mixture was stirred overnight, filtered through a short path of silica gel and the filtrate was evaporated *in vacuo*. The residue was purified by column chromatography (silica gel Merck 60, hexane/ethylacetate 1:0 - 9:1) yielding ethyl (Z)-[4-[3-(4-biphenylyl)-3-iodoprop-2-enylsulfanyl]-2-methylphenoxy]acetate.
Yield: 0.5 g (50%). R_{F} (SiO₂, hexane/ethyl acetate 4:1) 0.50.

### Step F:

To a solution of ethyl (Z)-[4-[3-(biphenyl-4-yl)-3-iodoallylsulfanyl]-2-methylphenoxy]-acetate (424 mg, 0.779 mmol) and tributyl-(furan-2-yl)tin (283 mg, 0.792 mmol, prepared in 78% yield according to Morimoto et al.: J.Med.Chem. 44, 3355 (2001)) in dry N,N-dimethylformamide (8 mL) Pd₂(dba)₃.CHCl₃ (23.8 mg, 0.023 mmol) was added. Traces of moisture and oxygen were removed and 0.20M solution of tri(tert.butyl)phosphine in cyclohexane (0.46 mL, 0.092 mmol) was added under atmosphere of nitrogen and the whole mixture was stirred at room temperature for 15 min and for further 3 h at 60°C. The dark solution was poured into 10% aqueous solution of potassium fluoride (15 mL) and subsequently ethyl acetate (50 mL) was added. The layers were separated, the aqueous layer was washed with ethyl acetate (2x15 mL) and the collected organic layers were washed with brine (2x20 mL), 10% solution of potassium fluoride (2x10 mL), water (2x10 mL) and brine (2x10 mL). The organic solution was dried with anhydrous sodium sulfate and its evaporation gave an oil that was purified by column chromatography (silica gel Fluka 60, hexane/ethyl acetate 10:1 + 0.1 % of triethylamine) yielding 330 mg of crude ethyl (Z)-[4-[3-(Biphenyl-4-yl)-3-(furan -2-yl)allylsulfanyl]-2-methylphenoxy]acetate.
Crude yield: 330 mg (88%). R_{F}= 0.25 (SiO₂, hexane/ethyl acetate 10:1).
¹H NMR spectrum (250 MHz, CDCl₃): 7.61 - 7.15 (m, 12 H); 6.59 (d, J = 8.3 Hz, 1 H); 6.40 (dd, J = 3.4 and 1.9 Hz, 1 H); 6.19 (d, J = 3.4 Hz, 1 H); 5.88 (t, J = 7.9 Hz, 1 H); 4.58 (s, 2 H); 4.23 (q, J = 7.2 Hz, 2 H); 3.96 (d, J = 8.0 Hz, 2 H); 2.21 (s, 3 H); 1.26 (t, J = 7.2 Hz, 3 H).

### General procedure B:

### Step A:

To a solution of the above ester (330 mg, 0.681 mmol) in a mixture of tetrahydrofuran/methanol (1:3, 8 mL) a solution of lithium hydroxide monohydrate (42 mg, 1.00 mmol) in distilled water (0.5 mL) was added. The resulting solution was stirred for 30 min and subsequently evaporated in vacuo. The residue was diluted with water (30 mL), neutralized with acetic acid (60 mg, 1.00 mmol) and extracted with ether (3x25 mL). The collected organic layers were washed with water (15 mL) and brine (2x15 mL) and dried with anhydrous sodium sulfate. The oil obtained by its evaporation was purified by column chromatography (silica gel Fluka 60, chloroform + 3-15% of methanol) yielding 161 mg of approximately equimolar mixture of both isomers of (E/Z)-[4-[3-(Biphenyl-4-yl)-3-(furan -2-yl)allylsulfanyl]-2-methylphenoxy]acetic acid.
Yield: 161 mg (52%). M.p.: --- (oil). R_{F}= 0.30 (SiO₂, chloroform/methanol 85:15).
¹H NMR spectrum (250 MHz, DMSO-d₆): 7.74 - 7.07 (m, 12 H); 6.75 (d, 1 H); 6.57 and 6.46 (dd, 1 H); 6.35 and 5.92 (d, 1 H); 6.28 and 5.93 (t, 1 H); 4.62 and 4.59 (s, 2 H); 3.93 and 3.53 (d, 2 H); 2.14 and 2.11 (s, 3 H).

The above acid (155 mg, 0.339 mmol) was dissolved in minimal amount of dry methylene chloride (about 1 mL), the formed solution was diluted with absolute methanol (8 mL) and L-lysine (47 mg, 0.321 mmol) was added. The reaction mixture was stirred at room temperature for 3 h, evaporated in vacuo and the residue was triturated with anhydrous ether (2x8 mL) yielding 140 mg of the L-lysinate of the title acid.
Yield: 140 mg (68 %). M.p.: 135 - 150°C (amorphous).
¹H NMR spectrum (250 MHz, DMSO-d₆): 7.73 - 6.63 (m, 13 H); 6.55 and 6.43 (dd, 1 H); 6.33 and 5.90 (d, 1 H); 6.25 and 5.91 (t, 1H); 4.26 and 4.24 (s, 2 H); 3.88 and 3.49 (d, 2 H); 3.25 (m, 1H); 2.71 (m, 2 H); 2.10 and 2.08 (s, 3 H); 1.79 - 1.27 (m, 6 H).

### Example 6 (General procedure (F))

### (E/Z)-[4-[3-(Benzo[b]thiophen-3-yl)-3-(biphenyl-4-yl)allylsulfanyl]-2-methylphenoxy]acetic acid

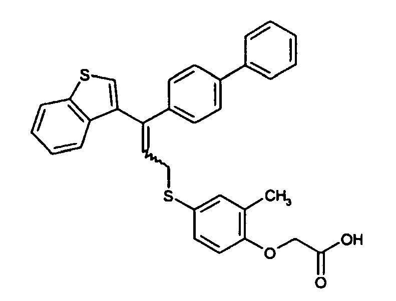

### Step F:

To a solution of ethyl (Z)-[4-[3-(biphenyl-4-yl)-3-iodoallylsulfanyl]-2-methylphenoxy]-acetate (307.4 mg, 0.565 mmol; example 5, step D-E) and (benzo[b]thiophen-3-yl)-tributyltin (242.4 mg, 0.573 mmol; prepared in 71% yield according to Morimoto et al.: J.Med.Chem. 44, 3355 (2001)) in dry N,N-dimethylformamide (3 mL) Pd₂(dba)₃-CHCl₃ (17.9 mg, 0.0173 mmol) was added. Traces of moisture and oxygen were removed and 0.20 M solution of tri(tert.butyl)phosphine in cyclohexane (0.367 mL, 0.073 mmol) was added under atmosphere of nitrogen and the whole mixture was stirred at 86°C for 6 h. The dark solution was poured into 10% aqueous solution of potassium fluoride (20 mL) and subsequently ethyl acetate (30 mL) was added. The layers were separated, the aqueous layer was washed with ethyl acetate (2x15 mL) and the collected organic layers were washed with brine (10 mL), 10% solution of potassium fluoride (20 mL), water (20 mL) and brine (20 mL). The organic solution was dried with anhydrous sodium sulfate and its evaporation gave an oil that was purified by column chromatography (silica gel Fluka 60, hexane/ethyl acetate 9:1) yielding 224 mg of crude ethyl (Z)-[4-[3-(Benzo[b]thiophen-3-yl)-3-(biphenyl-4-yl)allylsulfanyl]-2-methylphenoxy]acetate.
Yield: 224 mg (71%). R_{F}= 0.30 (SiO₂, hexane/ethyl acetate 9:1).
¹H NMR spectrum (250 MHz, CDCl₃): 6.61 - 7.60 (m, 16H); 6.59 (d, 1 H); 6.28 (t, 1 H); 4.61 (s, 2H); 4.24 (q, 2H); 3.75 (d, 2H); 2.19 (s, 3H); 1.26 (t, 3H).

### General procedure B:

### Step A:

To a solution of the above ester (222 mg, 0.403 mmol) in a mixture of tetrahydrofuran/ ethanol (1:1, 16.8 mL) 0.968M solution of lithium hydroxide monohydrate (0.52 mL, 0.503 mmol) was added. The resulting solution was stirred for 2 h and subsequently evaporated in vacuo. The residue was diluted with water (10 mL), acidified with 1 M hydrochloric acid to pH 2-3 and extracted with ether (40+15 mL). The collected organic layers were washed with water (25 mL) and brine (30 mL) and dried with anhydrous magnesium sulfate. The oil obtained by its evaporation was purified by column chromatography (silica gel Fluka 60, chloroform + 3-15% of methanol) yielding 87.5 mg of a mixture of both isomers of (E/Z)-[4-[3-(benzo[b]thiophen-3-yl)-3-(biphenyl-4-yl)allylsulfanyl]-2-methylphenoxy]acetic acid. Yield: 87.5 mg (42%). R_{F}= 0.10 (SiO₂, chloroform + 15% methanol).

The above acid (87.5 mg, 0.167 mmol) was dissolved in minimal amount of dry methylene chloride (about 2 mL), the formed solution was diluted with absolute methanol (5 mL) and L-lysine (23.5 mg, 0.161 mmol) was added. The reaction mixture was stirred at room temperature for 4.5 h, evaporated in vacuo and the residue was triturated with anhydrous ether (2x5 mL) yielding 50.6 mg of the L-lysinate of the title acid.
Yield: 50.6 mg (45%). M.p.: 125-145°C (amorphous).
¹H NMR spectrum (250 MHz, DMSO-d₆): 8.00 - 6.63 (m, 17H); 6.31 (t, 1 H); 4.22 + 4.21 (s, 2H); 3.74 + 3.51 (d, 2H); 3.18 (bt, 1 H); 2.71 (bt, 2H); 2.10 + 2.08 (s, 3H); 1.80 -1.30 (m, 6H).

### Example 7 (General procedure (F))

### [4-[(3-Benzo[b]thiophen-2-yl)-3-(biphenyl-4-yl)-allylsulfanyl]-2-methyl-phenoxy]-acetic acid

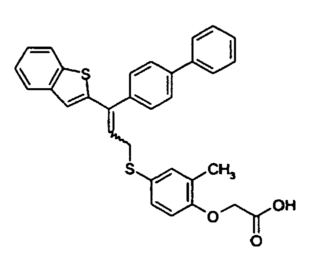

### Step F:

To a solution of ethyl (Z)-[4-[3-(biphenyl-4-yl)-3-iodoallylsulfanyl]-2-methylphenoxy]-acetate (419 mg, 0.770 mmol; example 5, step D-E) and (benzo[b]thiophen-2-yl)-tributyltin (335 mg, 0.792 mmol, prepared according to Morimoto et al.: J.Med.Chem. 44, 3355 (2001)) in dry N,N-dimethylformamide (9 mL) Pd₂(dba)₃.CHCl₃ (23.1 mg, 0.023 mmol) was added. Traces of moisture and oxygen were removed and 0.20M solution of tri(tert.butyl)phosphine in cyclohexane (0.39 mL, 0.098 mmol) was added under atmosphere of nitrogen and the whole mixture was stirred at 50°C for 10h and then left stand overnight. The dark solution was poured into 10% aqueous solution of potassium fluoride (30 mL) and subsequently ethyl acetate (40 mL) was added. The layers were separated, the aqueous layer was washed with ethyl acetate (2x15 mL) and the collected organic layers were washed with brine (10 mL), 10% solution of potassium fluoride (20 mL), water (20 mL) and brine (20 mL). The organic solution was dried with anhydrous sodium sulfate and its evaporation gave an oil that was purified by column chromatography (silica gel Fluka 60, hexane/ethyl acetate 93 : 7).
Yield 190 mg (45 %) of crude ethyl (Z)-[4-[(3-Benzo[b]thiophen-2-yl)-3-(biphenyl-4-yl)-allylsulfanyl]-2-methylphenoxy]acetate. R_{F}= 0.35 (SiO₂, hexane/ethyl acetate 9: 1 ).
¹H NMR spectrum (250 MHz, CDCl₃): 6.61 -7.60 (m, 16H); 6.59 (d, 1H); 6.28 (t, 1H); 4.61 (s, 2H); 4.24 (q, 2H); 3.75 (d, 2H); 2.19 (s, 3H); 1.26 (t, 3H).

### General procedure B:

### Step A:

To a solution of the above ester (190 mg, 0.345 mmol) in a mixture of tetrahydrofuran/ethanol (1:1, 16 mL) a solution of lithium hydroxide monohydrate (17.7 mg, 0.422 mmol) in water (0.15 mL) was added. The resulting solution was stirred for 2 h and subsequently evaporated in vacuo. The residue was diluted with water (10 mL), acidified with 1M hydrochloric acid to pH 2-3 and extracted with ether (30 +15 mL). The collected organic layers were washed with water (2 x 15 mL) and brine (30 mL) and dried with anhydrous magnesium sulfate. The oil obtained by its evaporation was purified by column chromatography (silica gel Fluka 60, toluene/methanol/acetic acid 100 : 5 : 1) yielding 66 mg of mixture of both isomers of (E/Z)-4-[(3-benzo[b]thiophen-2-yl)-3-(biphenyl-4-yl)-allylsulfanyl]-2-methyl-phenoxy]-acetic acid. Yield: 87.5 mg (49%). R_{F}= 0.10 (SiO₂, chloroform + 15% methanol).

The above acid (66 mg, 0.126 mmol) was dissolved in methanol (5 mL) and L-lysine (18.4 mg, 0.126 mmol) was added. The reaction mixture was stirred at room temperature for 1 h, evaporated in vacuo and the residue was triturated with anhydrous ether (3 x 5 mL) yielding 67 mg (78 %) of the L-lysinate of the title acid. M.p.: 140-155 °C (amorphous).
¹H NMR spectrum (250 MHz, DMSO-d₆): 7.98-6.82 (m, ~ 17); 6.12 (t, 1 H); 4.32 (s, ~ 2 H); 3.90 (m, ~ 2 H); 3.20 (t, 1 H); 2.73 (m, ~ 2 H); 2.16 (s, ~ 3 H); 1.78-1.30 (m, ~ 6 H).

### Example 8 (General procedure (F))

### (E/Z)-[4-[3-(4-Biphenylyl)-3-(5-methylthiophen-2-yl)allylsulfanyl]-2-methylphenoxy]acetic acid

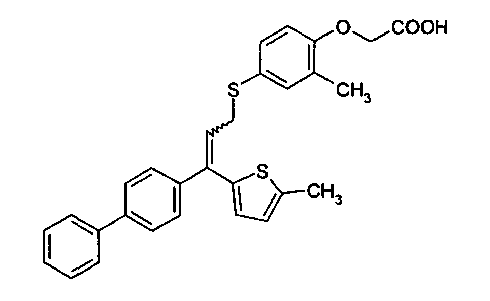

### Step F:

To a solution of ethyl (Z)-[4-[3-(4-biphenylyl)-3-iodoallylsulfanyl]-2-methylphenoxy]-acetate (240 mg, 0.441 mmol; Example 5, Step D-E) and tributyl-(5-methylthiophen-2-yl)tin (180 mg, 0.461 mmol, prepared in 86% yield according to Morimoto et al.: J.Med.Chem. 44, 3355 (2001)) in dry N,N-dimethylformamide (5 mL) tris(dibenzylideneacetone)dipalladium chloroform complex (13.5 mg, 0.013 mmol) was added. Traces of moisture and oxygen were removed and 0.25M solution of tri(tert-butyl)phosphine in cyclohexane (0.2 mL, 0.050 mmol) was added under atmosphere of nitrogen and the whole mixture was stirred at 50°C for 3 h. The dark solution was diluted with ethyl acetate (30 mL) and washed with brine (2x10 mL), water (2x10 mL), 10% solution of potassium fluoride (2x10 mL), 10% solution of sodium pyrosulfite (10 mL), 0.1 M hydrochloric acid (2x10 mL), water (10 mL), 10% solution of sodium hydrogencarbonate (2x10 mL) and brine (10 mL). The organic solution was dried with anhydrous sodium sulfate and its evaporation gave an oil. The crude product was purified by column chromatography (silica gel Fluka 60, hexane/ethyl acetate 8:1) yielding ethyl (Z)-[4-[3-(4-biphenylyl)-3-(5-methylthiophen-2-yl)allylsulfanyl]-2-methylphenoxy]acetate.
Yield: 190 mg (84%). R_{F} (SiO₂, hexane/ethyl acetate 8:1) 0.30.
¹H NMR spectrum (250 MHz, CDCl₃): 7.60-7.10 (m, 11 H); 6.63 (m, 3 H); 6.06 (t, J =7.8Hz, 1 H); 4.60 (s, 2 H); 4.23 (q, J=7.1 Hz, 2 H); 3.77 (d, J=7.8 Hz, 2 H); 2.48 (s, 3 H); 2.23 (s, 3 H); 1.26 (t, J=7.1 Hz, 3 H).

### General procedure B:

### Step A:

To a solution of the above ester (260 mg, 0.505 mmol) in a mixture tetrahydrofuran/ethanol (1:1, 6 mL) a solution of lithium hydroxide monohydrate (26 mg, 0.620 mmol) in distilled water (0.4 mL) was added. The resulting solution was stirred for 2 h and subsequently evaporated *in vacuo.* The residue was diluted with water (30 mL), acidified with 1 M hydrochloric acid to pH 3 and extracted with ether (3x20 mL). The collected organic layers were washed with water (20 mL) and 10% solution of potassium carbonate (3x20 mL). The alkaline solutions were collected, acidified with 1 M hydrochloric acid to pH 3 and extracted with ether (3x20 mL) again. The collected organic solutions were washed with water (20 mL) and brine (2x20 mL) and dried with anhydrous sodium sulfate. The oil obtained by its evaporation was purified by column chromatography (silica gel Fluka 60, chloroform/methanol 10:1) yielding the title compound as approximately equimolar mixture of both isomers.
Yield: 140 mg (60%). M.p. --- (foam). R_{F} (SiO₂, methylene chloride/methanol 9:1) 0.25.
¹H NMR spectrum (250 MHz, DMSO-d₆): 7.74-6.44 (m, 14 H); 6.09 and 6.06 (t, 1 H); 4.71 and 4.69 (s, 2 H); 4.13 and 3.76 (d, 2 H); 2.46 and 2.41 (s, 3 H); 2.16 and 2.12 (s, 3 H).

L-Lysine (42 mg, 0.287 mmol) was added to a solution of the above acid (140 mg, 0.288 mmol) in a mixture of dry methanol and ether (2:1, 6 mL). The formed suspension was evaporated and the residue was re-dissolved in a mixture of dry methanol and acetone (3:1, 4 mL). The formed solution was stirred for 2 h, evaporated *in vacuo* and the residue was repeatedly triturated with anhydrous ether yielding the L-lysinate of the title acid.
Yield: 170 mg (93 %). M.p.: 129 - 138°C (amorphous).
¹H NMR spectrum (250 MHz, DMSO-d₆): 7.75-6.45 (m, 14 H); 6.08 and 6.04 (t, 1 H); 4.28 (bs, 2 H); 3.72 and 3.42 (d, 2 H); 3.23 (bs, 1 H); 2.74 (bs, 2 H); 2.46 and 2.41 (s, 3 H); 2.14 and 2.10 (s, 3 H); 1.70-1.35 (m, 6 H).

### PHARMACOLOGICAL METHODS

### In vitro PPARalpha, PPARgamma and PPARdelta activation activity

The PPAR transient transactivation assays are based on transient transfection into human HEK293 cells of two plasmids encoding a chimeric test protein and a reporter protein respectively. The chimeric test protein is a fusion of the DNA binding domain (DBD) from the yeast GAL4 transcription factor to the ligand binding domain (LBD) of the human PPAR proteins. The PPAR-LBD moiety harbored in addition to the ligand binding pocket also the native activation domain (activating function 2 = AF2) allowing the fusion protein to function as a PPAR ligand dependent transcription factor. The GAL4 DBD will direct the chimeric protein to bind only to Gal4 enhancers (of which none existed in HEK293 cells). The reporter plasmid contained a Gal4 enhancer driving the expression of the firefly luciferase protein. After transfection, HEK293 cells expressed the GAL4-DBD-PPAR-LBD fusion protein. The fusion protein will in turn bind to the Gal4 enhancer controlling the luciferase expression, and do nothing in the absence of ligand. Upon addition to the cells of a PPAR ligand luciferase protein will be produced in amounts corresponding to the activation of the PPAR protein. The amount of luciferase protein is measured by light emission after addition of the appropriate substrate.

### CELL CULTURE AND TRANSFECTION

HEK293 cells were grown in DMEM + 10% FCS. Cells were seeded in 96-well plates the day before transfection to give a confluency of 50-80 % at transfection. A total of 0,8 µg DNA containing 0,64 µg pM1α/γLBD, 0,1 µg pCMVβGal, 0,08 µg pGL2(Gal4)₅ and 0,02 µg pADVANTAGE was transfected per well using FuGene transfection reagent according to the manufacturers instructions (Roche). Cells were allowed to express protein for 48 h followed by addition of compound.
Plasmids: Human PPAR α, γ and δ was obtained by PCR amplification using cDNA synthesized by reverse transcription of mRNA from human liver, adipose tissue and plancenta respectively. Amplified cDNAs were cloned into pCR2.1 and sequenced. The ligand binding domain (LBD) of each PPAR isoform was generated by PCR (PPARα: aa 167 - C-terminus; PPARγ: aa 165 - C-terminus; PPARδ: aa 128 - C-terminus) and fused to the DNA binding domain (DBD) of the yeast transcription factor GAL4 by subcloning fragments in frame into the vector pM1 (Sadowski et al. (1992), Gene 118, 137) generating the plasmids pM1αLBD, pM1γLBD and pM1δ. Ensuing fusions were verified by sequencing. The reporter was constructed by inserting an oligonucleotide encoding five repeats of the GAL4 recognition sequence (5 x CGGAGTACTGTCCTCCG(AG)) (Webster et al. (1988), Nucleic Acids Res. 16, 8192) into the vector pGL2 promotor (Promega) generating the plasmid pGL2(GAL4)₅. pCMVβGal was purchased from Clontech and pADVANTAGE was purchased from Promega.

### IN VITRO TRANSACTIVATION ASSAY

Compounds: All compounds were dissolved in DMSO and diluted 1:1000 upon addition to the cells. Compounds were tested in quadruple in concentrations ranging from 0.001 to 300 µM. Cells were treated with compound for 24 h followed by luciferase assay. Each compound was tested in at least two separate experiments.

Luciferase assay: Medium including test compound was aspirated and 100 µl PBS incl. 1mM Mg++ and Ca++ was added to each well. The luciferase assay was performed using the LucLite kit according to the manufacturers instructions (Packard Instruments). Light emission was quantified by counting on a Packard LumiCounter. To measure β-galactosidase activity 25 µl supernatant from each transfection lysate was transferred to a new microplate. β-galactosidase assays were performed in the microwell plates using a kit from Promega and read in a Labsystems Ascent Multiscan reader. The β-galactosidase data were used to normalize (transfection efficiency, cell growth etc.) the luciferase data.

### STATISTICAL METHODS

The activity of a compound is calculated as fold induction compared to an untreated sample. For each compound the efficacy (maximal activity) is given as a relative activity compared to Wy14,643 for PPARα, Rosiglitazone for PPARγ and Carbacyclin for PPARδ. The EC50 is the concentration giving 50% of maximal observed activity. EC50 values were calculated via non-linear regression using GraphPad PRISM 3.02 (GraphPad Software, San Diego, Ca). The results were expressed as means ± SD.

## Claims

1. A compound of the general formula (I): 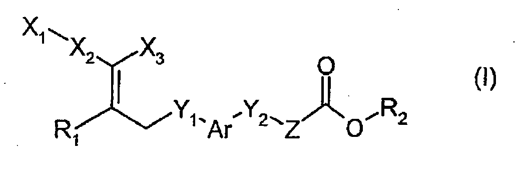
wherein X₁ is aryl or heteroaryl each of which is optionally substituted with one or more substituents selected from
• halogen, hydroxy, cyano, amino or carboxy; or
• C₁₋₆alkyl, C₃₋₆₇cycloalkyl, C₂₋₆-alkenyl, C₂₋₆-alkynyl, aryl, aralkyl, heteroaryl, heteroaralkyl, C₁₋₆alkoxy, C₃₋₆-cycloalkoxy, aryloxy, aralkoxy, heteroaralkoxy, C₁₋₈-alkylthio, arylthio, C₃₋₆-cycloalkylthio, C₁₋₆alkylcarbonyl, arylcarbonyl, C₁₋₆-alkylsulfonyl, arylsulfonyl, C₁₋₆alkylsulfonyloxy, arylsulfonyloxy, C₁₋₆-alkylamido, arylamido, C₁₋₆-alkylaminocarbonyl, C₁₋₆-alkylamino, C₁₋₆-dialkylamino or C₃₋₆cycloalkylamino each of which is optionally substituted with one or more halogens; and
X₂ is arylene or heteroarylene each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, C₁₋₆-alkoxy, C₃₋₆-cycloalkoxy, C₁₋₆-alkylthio, C₃₋₆-cycloalkylthio, C₁₋₆-alkylamino, C₁₋₆-dialkylamino or C₃₋₆-cycloalkylamino each of which is optionally substituted with one or more halogens; and
X₃ is aryl or heteroaryl each of which is optionally substituted with one or more substituents selected from
• halogen, hydroxy, cyano, amino or carboxy; or
• C₁₋₆-alkyl C₃₋₆-cycloalkyl, C₂₋₆-alkenyl, C₂₋₆-alkynyl, aralkyl, heteroaralkyl, C₁₋₆-alkoxy, C₃₋₆-cycloalkoxy, aryloxy, aralkoxy, heteroaralkoxy, C₁₋₆-alkylthio, arylthio, C₃₋₆-cycloalkylthio, C₁₋₆-alkylcarbonyl, arylcarbonyl, C₁₋₆-alkylsulfonyl, arylsulfonyl, C₁₋₆-alkylsulfonyloxy, arylsulfonyloxy, C₁₋₆-alkylamido, arylamido, C₁₋₆-alkylaminocarbonyl, C₁₋₆-alkylamino, C₁₋₆-dialkylamino or C₃₋₆-cycloalkylamino each of which is optionally substituted with one or more halogens; and
Ar is arylene which is optionally substituted with one or more substituents selected from
• halogen, hydroxy or cyano; or
• C₁₋₆-alkyl, C₃₋₆-cycloalkyl, C₂₋₆-alkenyl, C₂₋₆-alkynyl, aryl, heteroaryl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆cycloalkylthio each of which is optionally substituted with one or more halogens; and
Y₁ is O or S; and
Y₂ is O or S; and
Z is -(CH₂)ₙ- wherein n is 1, 2 or 3; and
R₁ is hydrogen, halogen or a substituent selected from
• C₁₋₆-alkyl, C₃₋₆-cycloalkyl, C₂₋₆-alkenyl, C₂₋₆-alkynyl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio each of which is optionally substituted with one or more halogens; and
R₂ is hydrogen, C₁₋₆-alkyl, C₃₋₆-cycloalkyl, C₂₋₆-alkenyl, C₂₋₆-alkynyl, C₄₋₆-alkenynyl or aryl; or
a pharmaceutically acceptable salt thereof, or a pharmaceutically acceptable solvate thereof, or any tautomeric forms, stereoisomers, mixture of stereoisomers including a racemic mixture, or polymorphs.

2. A compound according to claim 1, wherein X₁ is aryl or heteroaryl optionally substituted with one or more substituents selected from
• halogen; or
• C₁₋₆-alkyl, aryl, C₁₋₆alkoxy, C₁₋₆-alkylsulfonyl or C₁₋₆-alkylsulfonyloxy each of which is optionally substituted with one or more halogens.

3. A compound according to claim 2, wherein X₁ is phenyl, furyl, thienyl, benzothienyl or benzofuranyl optionally substituted with one or more substituents selected from
• halogen; or
• C₁₋₆-alkyl, aryl, C₁₋₆-alkoxy, C₁₋₆-alkylsulfonyl or C₁₋₆alkylsulfonyloxy each of which is optionally substituted with one or more halogens.

4. A compound according to claim 3, wherein X₁ is phenyl optionally substituted with one or more substituents selected from halogen, C₁₋₈-alkyl, aryl or perhalomethyl.

5. A compound according to claim 4, wherein X₁ is phenyl optionally substituted with one or more substituents seleced from phenyl or trifluoromethyl.

6. A compound according to claim 5, wherein X₁ is phenyl.

7. A compound according to claim 3, wherein X₁ is furyl, thienyl, benzothienyl or benzofuranyl with one or more substituents seleced from halogen, C₁₋₆-alkyl, aryl or perhalomethyl.

8. A compound according to claim 7, wherein X₁ is furyl optionally substituted with one or more substituents seleced from halogen, C₁₋₆-alkyl, phenyl or trifluoromethyl.

9. A compound according to claim 7, wherein X₁ is thienyl optionally substituted with one or more substituents seleced from halogen, C₁₋₆-alkyl, phenyl or trifluoromethyl.

10. A compound according to claim 7, wherein X₁ is benzothienyl optionally substituted with one or more substituents seleced from halogen, C₁₋₆-alkyl, phenyl or trifluoromethyl.

11. A compound according to any one of the preceding claims, wherein X₂ is arylene or heteroarylene optionally substituted with one or more substituents selected from
• halogen; or
• C₁₋₆-alkyl or C₁₋₆-alkoxy each of which is optionally substituted with one or more halogens.

12. A compound according to claim 11, wherein X₂ is phenylene optionally substituted with one or more substituents selected from
• halogen; or
• C₁₋₆-alkyl or C₁₋₆-alkoxy each of which is optionally substituted with one or more halogens.

13. A compound according to claim 12, wherein X₂ is phenylene optionally substituted with one or more halogens.

14. A compound according to claim 13, wherein X₂ is phenylene.

15. A compound according to claim 11, wherein X₂ is benzofuranylene optionally substituted with one or more substituents selected from
• halogen; or
• C₁₋₆-alkyl or C₁₋₆-alkoxy each of which is optionally substituted with one or more halogens.

16. A compound according to claim 15, wherein X₂ is benzofuranylene optionally substituted with C₁₋₆-alkyl.

17. A compound according to any one of the preceding claims, wherein X₃ is aryl or heteroaryl optionally substituted with one or more substituents selected from
• halogen; or
• C₁₋₆₇alkyl, C₁₋₆alkoxy, C₁₋₆-alkylsulfonyl or C₁₋₆-alkylsulfonyloxy each of which is optionally substituted with one or more halogens.

18. A compound according to claim 17, wherein X₃ is phenyl, furyl, thienyl, benzothienyl or benzofuranyl optionally substituted with one or more substituents selected from
• halogen; or
• C₁₋₆-alkyl, C₁₋₆-alkoxy, C₁₋₆alkylsulfonyl or C₁₋₆-alkylsulfonyloxy each of which is optionally substituted with one or more halogens.

19. A compound according to claim 18, wherein X₃ is phenyl optionally substituted with one or more halogens.

20. A compound according to claim 18, wherein X₃ is phenyl optionally substituted with one or more substituents selected from C₁₋₆-alkyl or perhalomethyl.

21. A compound according to claim 18, wherein X₃ is phenyl.

22. A compound according to claim 18, wherein X₃ is furyl, thienyl, benzothienyl or benzofuranyl optionally substituted with one or more substituents selected from halogen, C₁₋₆-alkyl or perhalomethyl.

23. A compound according to claim 22, wherein X₃ is furyl optionally substituted with one or more substituents selected from halogen, C₁₋₆-alkyl or trifluoromethyl.

24. A compound according to claim 22, wherein X₃ is thienyl optionally substituted with one or more substituents selected from halogen, C₁₋₆-alkyl or trifluoromethyl.

25. A compound according to claim 22, wherein X₃ is benzothienyl optionally substituted with one or more substituents selected from halogen, C₁₋₆-alkyl or trifluoromethyl.

26. A compound according to claim 22, wherein X₃ is benzofuranyl optionally substituted with one or more substituents selected from halogen, C₁₋₆-alkyl or trifluoromethyl.

27. A compound according to any one of the preceding claims, wherein Ar is phenylene which is optionally substituted with one or more substituents selected from
• halogen, hydroxy or cyano; or
• C₁₋₆-alkyl, C₃₋₆-cycloalkyl, C₂₋₆-alkenyl, C₂₋₆-alkynyl, aryl, heteroaryl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio each of which is optionally substituted with one or more halogens.

28. A compound according to claim 27, wherein Ar is phenylene which is optionally substituted with one or more substituents selected from
• halogen; or
• C₁₋₆-alkyl, C₁₋₆-alkoxy, aryloxy or aralkoxy each of which is optionally substituted with one or more halogens.

29. A compound according to claim 28, wherein Ar is phenylene which is optionally substituted with methyl.

30. A compound according to claim 29, wherein Ar is phenylene.

31. A compound according to any one of the preceding claims, wherein Y₁ is S.

32. A compound according to any one of the preceding claims, wherein Y₂ is O.

33. A compound according to any one of the preceding claims, wherein n is 1.

34. A compound according to any one of the preceding claims, wherein R₁ is hydrogen or a substituent selected from C₁₋₆-alkyl, aralkyl, C₁₋₆-alkoxy, aryloxy, aralkoxy each of which is optionally substituted with one or more halogens.

35. A compound according to claim 34, wherein R₁ is hydrogen or a substituent selected from C₁₋₆-alkyl or C₁₋₆alkoxy each of which is optionally substituted with one or more halogens.

36. A compound according to claim 35, wherein R₁ is hydrogen.

37. A compound according to any one of the preceding claims, wherein R₂ is hydrogen.

38. A compound according to any one of the preceding claims, wherein R₂ is methyl or ethyl.

39. A compound according to any one of the preceding claims, which is:
(E/Z) {4-[3-Biphenyl-4-yl-3-(4-bromo-phenyl)-allylsulfanyl]-phenoxy}-acetic acid;
(E/Z) {4-[3-(4-Bromo-phenyl)-3-(3'-trifluoromethyl-biphenyl-4-yl)-allylsulfanyl]-2-methylphenoxy}-acetic acid; or
a pharmaceutically acceptable salt thereof, or a pharmaceutically acceptable solvate thereof, or any tautomeric forms, stereoisomers, mixture of stereoisomers including a racemic mixture, or polymorphs.

40. A compound according to any one of the claims 1 to 38, which is:
{4-[3-(4-Bromo-phenyl)-3-[1,1';4',1"] terphenyl-4"-yl-allylsulfanyl]-2-methyl-phenoxy}-acetic acid;
(E/Z)-[2-Methyl-4-[3-[5-(5-methylthiophen-2-yl)benzo[b]furan-2-yl]-3-(thiophen-2-yl)allylsulfanyl]phenoxy]acetic acid;
(E/Z)-[4-[3-(Biphenyl-4-yl)-3-(furan-2-yl)allylsulfanyl]-2-methylphenoxy]acetic acid;
(E/Z)-[4-[3-(Benzo[b]thiophen-3-yl)-3-(biphenyl-4-yl)allylsulfanyl]-2-methylphenoxy]acetic acid;
[4-[(3-Benzo[b]thiophen-2-yl)-3-(biphenyl-4-yl)-allylsulfanyl]-2-methyl-phenoxy]-acetic acid;
(E/Z)-[4-[3-(4-Biphenylyl)-3-(5-methylthiophen-2-yl)allylsulfanyl]-2-methylphenoxy]acetic acid;
or a pharmaceutically acceptable salt thereof, or a pharmaceutically acceptable solvate thereof, or any tautomeric forms, stereoisomers, mixture of stereoisomers including a racemic mixture, or polymorphs.

41. A compound according to any one of the claims 1 to 38, which is:
(E) {4-[3-Biphenyl-4-yl-3-(2-fluoro-phenyl)-allylsulfanyl]-phenoxy}acetic acid;
(Z) {4-[3-Biphenyl-4-yl-3-(2-fluoro-phenyl)-allylsulfanyl]-phenoxy}-acetic acid;
(E) {4-[3-Biphenyl-4-yl-3-(2-chloro-phenyl)-allylsulfanyl]-phenoxy}-acetic acid;
(Z) {4-[3-Biphenyl-4-yl-3-(2-chloro-phenyl)-allylsulfanyl]-phenoxy}-acetic acid;
(E) {4-[3-Biphenyl-4-yl-3-(2-bromo-phenyl)-allylsulfanyl]-phenoxy}acetic acid;
(Z) {4-[3-Biphenyl-4-yl-3-(2-bromo-phenyl)-allylsuffanyl]-phenoxy}-acetic acid;
(E) {4-[3-Biphenyl-4-yl-3-(2-iodo-phenyl)-allylsulfanyl]-phenoxy}-acetic acid;
(Z) {4-[3-Biphenyl-4-yl-3-(2-iodo-phenyl)-allylsulfanyl]-phenoxy}-acetic acid;
(E) {4-[3-Biphenyl-4-yl-3-(2-methoxy-phenyl)-allylsulfanyl]-phenoxy}-acetic acid;
(Z) {4-[3-Biphenyl-4-yl-3-(2-methoxy-phenyl)-allylsulfanyl]-phenoxy}-acetic acid;
(E) {4-[3-Biphenyl-4-yl-3-(2-trifluoromethoxy-phenyl)-allylsulfanyl]-phenoxy}-acetic acid;
(Z) {4-[3-Biphenyl-4-yl-3-(2-trifluoromethoxy-phenyl)-allylsulfanyl]-phenoxy}-acetic acid;
(E) {4-[3-Biphenyl-4-yl-3-(2-methyl-phenyl)-allylsulfanyl]-phenoxy}-acetic acid;
(Z) {4-[3-Biphenyl-4-yl-3-(2-methyl-phenyl)-allylsulfanyl]-phenoxy}-acetic acid;
(E) {4-[3-Biphenyl-4-yl-3-(2-trifluoromethyl-phenyl)-allylsulfanyl]-phenoxy}-acetic acid;
(Z) {4-[3-Biphenyl-4-yl-3-(2-trifluoromethyl-phenyl)-allylsulfanyl]-phenoxy}-acetic acid;
(E) {4-[3-Biphenyl-4-yl-3-(3-fluoro-phenyl)-allylsulfanyl]-phenoxy}-acetic acid;
(Z) {4-[3-Biphenyl-4-yl-3-(3-fluoro-phenyl)-allylsulfanyl]-phenoxy}-acetic acid;
(E) {4-[3-Biphenyl-4-yl-3-(3-chloro-phenyl)-allylsulfanyl]-phenoxy}-acetic acid;
(Z) {4-[3-Biphenyl-4-yl-3-(3-chloro-phenyl)-allylsulfanyl]-phenoxy}-acetic acid;
(E) {4-[3-Biphenyl-4-yl-3-(3-bromo-phenyl)-allylsulfanyl]-phenoxy}-acetic acid;
(Z) {4-[3-Biphenyl-4-yl-3-(3-bromo-phenyl)-allylsulfanyl]-phenoxy}-acetic acid;
(E) {4-[3-Biphenyl-4-yl-3-(3-iodo-phenyl)-allylsulfanyl]-phenoxy}-acetic acid;
(Z) {4-[3-Biphenyl-4-yl-3-(3-iodo-phenyl)-allylsulfanyl]phenoxy}-acetic acid;
(E) {4-[3-Biphenyl-4-yl-3-(3-methoxy-phenyl)-allylsulfanyl]-phenoxy}-acetic acid;
(Z) {4-[3-Biphenyl-4-yl-3-(3-methoxy-phenyl)-allylsulfanyl]-phenoxy}-acetic acid;
(E) {4-[3-Biphenyl-4-yl-3-(3-trifluoromethoxy-phenyl)-allylsulfanyl]-phenoxy}-acetic acid;
(Z) {4-[3-Biphenyl-4-yl-3-(3-trifluoromethoxy-phenyl)-allylsulfanyl]-phenoxy}-acetic acid;
(E) {4-[3-Biphenyl-4-yl-3-(3-methyl-phenyl)-allylsulfanyl]-phenoxy}acetic acid;
(Z) {4-[3-Biphenyl-4-yl-3-(3-methyl-phenyl)-allylsulfanyl]-phenoxy}-acetic acid;
(E) {4-[3-Biphenyl-4-yl-3-(3-trifluoromethyl-phenyl)-allylsulfanyl]-phenoxy}-acetic acid;
(Z) {4-[3-Biphenyl-4-yl-3-(3-trifluoromethyl-phenyl)-allylsulfanyl]-phenoxy}-acetic acid;
(E) {4-[3-Biphenyl-4-yl-3-(4-fluoro-phenyl)-allylsulfanyl]-phenoxy}-acetic acid;
(Z) {4-[3-Biphenyl-4-yl-3-(4-fluoro-phenyl)-allylsulfanyl]-phenoxy}-acetic acid;
(E) {4-[3-Biphenyl-4-yl-3-(4-chloro-phenyl)-allylsulfanyl]-phenoxy}-acetic acid;
(Z) {4-[3-Biphenyl-4-yl-3-(4-chloro-phenyl)-allylsulfanyl]-phenoxy}-acetic acid;
(E/Z) {4-[3-Biphenyl4-yl-3-(4-bromo-phenyl)-allylsulfanyl]-phenoxy}-acetic acid;
(E) {4-[3-Biphenyl-4-yl-3-(4-bromo-phenyl)-allylsulfanyl]-phenoxy}-acetic acid;
(Z) {4-[3-Biphenyl-4-yl-3-(4-bromo-phenyl)-allylsulfanyl]-phenoxy}-acetic acid;
(E) {4-[3-Biphenyl-4-yl-3-(4-iodo-phenyl)-allylsulfanyl]-phenoxy}-acetic acid;
(Z) {4-[3-Biphenyl-4-yl-3-(4-iodo-phenyl)-allylsulfanyl]-phenoxy}-acetic acid;
(E) {4-[3-Biphenyl-4-yl-3-(4-methoxy-phenyl)-allylsulfanyl]-phenoxy}-acetic acid;
(Z) {4-[3-Biphenyl-4-yl-3-(4-methoxy-phenyl)-allylsulfanyl]-phenoxy}-acetic acid;
(E) {4-[3-Biphenyl-4-yl-3-(4-trifluoromethoxy-phenyl)-allylsulfanyl]-phenoxy}-acetic acid;
(Z) {4-[3-Biphenyl-4-yl-3-(4-trifluoromethoxy-phenyl)-allylsulfanyl]-phenoxy}-acetic acid;
(E) {4-[3-Biphenyl-4-yl-3-(4-methyl-phenyl)-allylsulfanyl]-phenoxy}-acetic acid;
(Z) {4-[3-Biphenyl-4-yl-3-(4-methyl-phenyl)-allylsul*fanyl]-phenoxy}-acetic acid;
(E) {4-[3-Biphenyl-4-yl-3-(4-trifluoromethyl-phenyl)-allylsulfanyl]-phenoxy}-acetic acid;
(Z) {4-[3-Biphenyl-4-yl-3-(4-trifluoromethyl-phenyl)-allylsulfanyl]-phenoxy}-acetic acid;
(E) {4-[3-(4-Fluoro-phenyl)-3-(2'-trifluoromethyl-biphenyl-4-yl)-allylsulfanyl]-2-methylphenoxy}-acetic acid;
(Z) {4-[3-(4-Fluoro-phenyl)-3-(2'-trifluoromethyl-biphenyl-4-yl)-allylsulfanyl]-2-methylphenoxy}-acetic acid;
(E/Z) {4-[3-(4-Fluoro-phenyl)-3-(3'-trifluoromethyl-biphenyl-4-yl)-allylsulfanyl]-2-methylphenoxy}-acetic acid;
(E) {4-[3-(4-Fluoro-phenyl)-3-(3'-trifluoromethyl-biphenyl-4-yl)-allylsulfanyl]-2-methylphenoxy}-acetic acid;
(Z) {4-[3-(4-Fluoro-phenyl)-3-(3'-trifluoromethyl-biphenyl-4-yl)-allylsulfanyl]-2-methylphenoxy}-acetic acid;
(E) {4-[3-(4-Fluoro-phenyl)-3-(4'-trifluoromethyl-biphenyl-4-yl)-allylsulfanyl]-2-methylphenoxy}-acetic acid;
(Z) {4-[3-(4-Fluoro-phenyl)-3-(4'-trifluoromethyl-biphenyl-4-yl)-allylsulfanyl]-2-methylphenoxy}-acetic acid;
(E) {4-[3-(4-Fluoro-phenyl)-3-(3'-chloro-biphenyl-4-yl)-allylsulfanyl]-2-methyl-phenoxy}-acetic acid;
(Z) {4-[3-(4-Fluoro-phenyl)-3-(3'-chloro-biphenyl-4-yl)-allylsulfanyl]-2-methyl-phenoxy}acetic acid;
(E) {4-[3-(4-Fluoro-phenyl)-3-(4'-chloro-biphenyl-4-yl)-allylsulfanyl]-2-methyl-phenoxy}-acetic acid;
(Z) {4-[3-(4-Fluoro-phenyl)-3-(4'-chloro-biphenyl-4-yl)-allylsulfanyl]-2-methyl-phenoxy}-acetic acid;
(E) {4-[3-(4-Fluoro-phenyl)-3-(3'-methoxy-biph enyl-4-yl)-allylsulfanyl]-2-methyl-phenoxy}-acetic acid;
(Z) {4-[3-(4-Fluoro-phenyl)-3-(3'-methoxy-biphenyl-4-yl)-allylsulfanyl]-2-methyl-phenoxy}-acetic acid;
(E) {4-[3-(4-Fluoro-phenyl)-3-(4'-methoxy-biphenyl-4-yl)-allylsulfanyl]-2-methyl-phenoxy}-acetic acid;
(Z) {4-[3-(4-Fluoro-phenyl)-3-(4'-methoxy-biphenyl-4-yl)-allylsulfanyl]-2-methyl-phenoxy}-acetic acid;
(E) {4-[3-(4-Chloro-phenyl)-3-(2'-trifluoromethyl-biphenyl-4-yl)-allylsulfanyl]-2-methylphenoxy}-acetic acid;
(Z) {4-[3-(4-Chloro-phenyl)-3-(2'-trifluoromethyl-biphenyl-4-yl)-allylsulfanyl]-2-methylphenoxy}-acetic acid;
(E/Z) {4-[3-(4-Chloro-phenyl)-3-(3'-trifluoromethyl-biphenyl-4-yl)-allylsulfanyl]-2-methylphenoxy}-acetic acid;
(E) {4-[3-(4-Chloro-phenyl)-3-(3'-trifluoromethyl-biphenyl-4-yl)-allylsulfanyl]-2-methylphenoxy}-acefic acid;
(Z) {4-[3-(4-Chloro-phenyl)-3-(3'-trifluoromethyl-biphenyl-4-yl)-allylsulfanyl]-2-methylphenoxy}-acetic acid;
(E) {4-[3-(4-Chloro-phenyl)-3-(4'-trifluoromethyl-biphenyl-4-yl)-allylsulfanyl]-2-methylphenoxy}-acetic acid;
(Z) {4-[3-(4-Chloro-phenyl)-3-(4'-trifluoromethyl-biphenyl-4-yl)-allylsulfanyl]-2-methylphenoxy}-acetic acid;
(E) {4-[3-(4-Chloro-phenyl)-3-(3'-chloro-biphenyl-4-yl)-allylsulfanyl]-2-methyl-phenoxy}-acetic acid;
(Z) {4-[3-(4-Chloro-phenyl)-3-(3'-chloro-biphenyl-4-yl)-allylsulfanyl]-2-methyl-phenoxy}-acetic acid;
(E) {4-[3-(4-Chloro-phenyl)-3-(4'-chloro-biphenyl-4-yl)-allylsulfanyl]-2-methyl-phenoxy}-acetic acid;
(Z) {4-[3-(4-Chloro-phenyl)-3-(4'-chloro-biphenyl-4-yl)-allylsulfanyl]-2-methyl-phenoxy}-acetic acid;
(E) {4-[3-(4-Chloro-phenyl)-3-(3'-methoxy-biphenyl-4-yl)-allylsulfanyl]-2-methyl-phenoxy}-acetic acid;
(Z) {4-[3-(4-Chloro-phenyl)-3-(3'-methoxy-biphenyl-4-yl)-allylsulfanyl]-2-methyl-phenoxy}-acetic acid;
(E) {4-[3-(4-Chloro-phenyl)-3-(4'-methoxy-biphenyl-4-yl)-allylsulfanyl]-2-methyl-phenoxy} acetic acid;
(Z) {4-[3-(4-Chloro-phenyl)-3-(4'-methoxy-biphenyl-4-yl)-allylsulfanyl]-2-methyl-phenoxy} acetic acid;
(E) {4-[3-(4-Bromo-phenyl)-3-(2'-trifluoromethyl-biphenyl-4-yl)-allylsulfanyl]-2-methylphenoxy}-acetic acid;
(Z) {4-[3-(4-Bromo-phenyl)-3-(2'-trifluoromethyl-biphenyl-4-yl)-allylsulfanyl]-2-methylphenoxy}-acetic acid;
(E/Z) {4-[3-(4-Bromo-phenyl)-3-(3'-trifluoromethyl-biphenyl-4-yl)-allylsulfanyl]-2-methylphenoxy}-acetic acid;
(E) {4-[3-(4-Bromo-phenyl)-3-(3'-trifluoromethyl-biphenyl-4-yl)-allylsulfanyl]-2-methylphenoxy}-acetic acid;
(Z) {4-[3-(4-Bromo-phenyl)-3-(3'-trifluoromethyl-biphenyl-4-yl)-allylsulfanyl]-2-methylphenoxy}-acetic acid;
(E) {4-[3-(4-Bromo-phenyl)-3-(4'-trifluoromethyl-biphenyl-4-yl)-allylsulfanyl]-2-methylphenoxy}-acetic acid;
(Z) {4-[3-(4-Bromo-phenyl)-3-(4'-trifluoromethyl-biphenyl-4-yl)-allylsulfanyl]-2-methylphenoxy}-acetic acid;
(E) {4-[3-(4-Bromo-phenyl)-3-(3'-chloro-biphenyl-4-yl)-allylsulfanyl]-2-methyl-phenoxy}-acetic acid;
(Z) {4-[3-(4-Bromo-phenyl)-3-(3'-chloro-biphenyl-4-yl)-allylsulfanyl]-2-methyl-phenoxy}-acetic acid;
(E) {4-[3-(4-Bromo-phenyl)-3-(4'-chloro-biphenyl-4-yl)-allylsulfanyl]-2-methyl-phenoxy}-acetic acid;
(Z) {4-[3-(4-Bromo-phenyl)-3-(4'-chloro-biphenyl-4-yl)-allylsulfanyl]-2-methyl-phenoxy}-acetic acid;
(E) {4-[3-(4-Bromo-phenyl)-3-(3'-methoxy-biphenyl-4-yl)-allylsulfanyl]-2-methyl-phenoxy} acetic acid;
(Z) {4-[3-(4-Bromo-phenyl)-3-(3'-methoxy-biphenyl-4-yl)-allylsulfanyl]-2-methyl-phenoxy}-acetic acid;
(E) {4-[3-(4-Bromo-phenyl)-3-(4'-methoxy-biphenyl-4-yl)-allylsulfanyl]-2-methyl-phenoxy}-acetic acid;
(Z) {4-[3-(4-Bromo-phenyl)-3-(4'-methoxy-biphenyl-4-yl)-allylsulfanyl]-2-methyl-phenoxy}-acetic acid; or
a pharmaceutically acceptable salt thereof, or a pharmaceutically acceptable solvate thereof, or any tautomeric forms, stereoisomers, mixture of stereoisomers including a racemic mixture, or polymorphs.

42. A combination of a compound as defined in any one of claims 1 to 41 and one or more additional pharmacologically active substances.

43. The combination according to claim 42 wherein the one or more additional pharmacologically active substances are selected from antiobesity agents, antidiabetics, antihypertensive agents, agents for the treatment and/or prevention of complications resulting from or associated with diabetes and agents for the treatment and/or prevention of complications and disorders resulting from or associated with obesity.

44. A pharmaceutical composition comprising, as an active ingredient, at least one compound as defined in any one of claims 1 to 41, or a combination according to claim 42 or 43, together with one or more pharmaceutically acceptable carriers or excipients.

45. A pharmaceutical composition according to claim 44 in unit dosage form, comprising from about 0.05 mg to about 1000 mg, preferably from about 0.1 to about 500 mg of and especially preferred from about 0.5 mg to about 200 mg per day of compound according to any one of claims 1 to 41.

46. A pharmaceutical composition according to claim 44 or claim 45 for oral, nasal, transdermal, pulmonal or parenteral administration.

47. A compound as defined in any one of claims 1 to 41, a combination as defined in claim 42 or 43, or a composition as defined in any one of claims 44 to 46, for use as a medicament.

48. A compound, combination or composition according to claim 47 for the treatment and/or prevention of conditions mediated by the Peroxisome Proliferator-Activated Receptors (PPAR).

49. A compound, combination or composition according to claim 47 for the treatment and/or prevention of Type 1 diabetes; Type 2 diabetes; dyslipidemia; syndrome X including metabolic syndrome, impaired glucose tolerance, insulin resistance, hypertriglyceridaemia and/or obesity; cardiovascular diseases, including atherosclerosis; and hypercholesteremia.

50. A compound, combination or composition according to claim 47 for the treatment and/or prevention of hyperglycaemia and hyperlipidemia.

51. A process for preparing a compound as defined in any one of claims 1 to 41, wherein said process is selected from (A) to (F), below:
(A) Reacting a compound of formula (II) 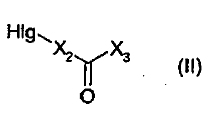
wherein X₂ and X₃ are defined in claim 1 and wherein Hlg is bromine or iodine, through a Wittig-like process with (EtO)₂PO(CHR₁)COOR₆ wherein R₆ is an alkyl group and R₁ is defined as in claim 1, in the presence of a base to give a compound of formula (III) 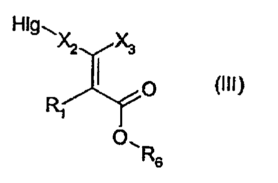
wherein X₂, X₃, R₁ and R₆ are defined as above and wherein Hlg is bromine or iodine, and
reducing the compound of formula (III) to give a compound of formula (IV) 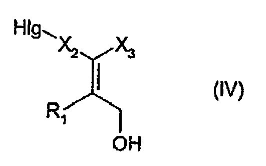
wherein X₂, X₃ and R₁ are defined as above and wherein HIg is bromine or iodine, and
reacting the compound of formula (IV), wherein X₂, X₃ and R₁ are defined as above and wherein Hlg is bromine or iodine, except that when X₂ or X₃ are substituted with hydroxy, this functionality has to be protected with a compound of formula (V) 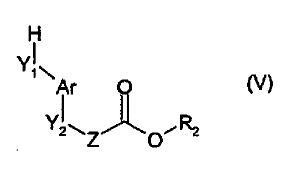
wherein Y₁, Ar, Y₂, Z and R₂ are defined as in claim 1, except that R₂ is not hydrogen under Mitsunobu conditions, using a reagent such as triphenylphosphine/diethylazodicarboxylate to obtain a compound of formula (VI) 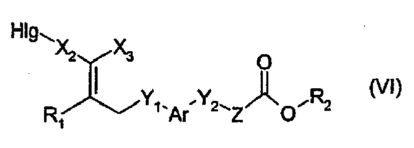
wherein X₂, X₃, Y₁, Y₂, Ar, Z, R₁ and R₂ are defined as above, except that R₂ is not hydrogen and wherein HIg is bromine or iodine, and
reacting a compound of formula (VI) with a boronic acid or with a tributyltin derivative of X₁ under appropriate coupling conditions to give a compound of formula (I), wherein X₁, X₂, X₃, Y₁, Y₂, Ar, Z, R₁ and R₂ are defined as above, except that R₂ is not hydrogen; or
(B) Chemically or enzymatically saponifying a compound of formula (I) wherein X₁, X₂, X₃, Y₁, Y₂, Ar, Z, R₁ and R₂ are defined as in claim 1, except that R₂ is not hydrogen, to give a compound of formula (I) wherein X₁, X₂, X₃, Y₁, Y₂, Ar, Z, R₁, and R₂ are defined as above, except that R₂ is hydrogen; or
(C) Chemically or enzymatically saponifying a compound of formula (VI) wherein X₂, X₃, Y₁, Y₂, Ar, Z, R₁, and R₂ are defined as in claim 1, except that R₂ is not hydrogen, and wherein Hlg is bromine or iodine, to give a compound of formula (VI) wherein X₁, X₂, X₃, Y₁, Y₂, Ar, Z, R₁, and R₂ are defined as above, except that R₂ is hydrogen and wherein Hlg is bromine or iodine, and
reacting a compound of formula (VI) with a boronic acid derivative of X₁, under appropriate coupling conditions to give a compound of formula (I), wherein X₁, X₂, X₃, Y₁, Y₂, Ar, Z, R₁ and R₂ are defined as above, except that R₂ is hydrogen; or
(D) Reacting a compound of formula (VII) 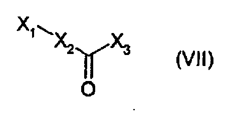
wherein X₁, X₂ and X₃ are defined as in claim 1; through a Wittig-like process with (EtO)₂PO(CHR₁)COOR₆, wherein R₆ in an alkyl group and R₁ is defined as in claim 1, in the presence of a base to give a compound of for-mula (VII) 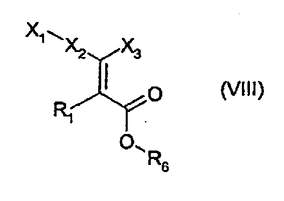
wherein X₁, X₂, X₃, R₁ and R₆ are defined as above, and
reducing the compound of formula (VIII) wherein X₁, X₂, X₃, R₁ and R₆ are defined as above to give a compound of formula (IX) 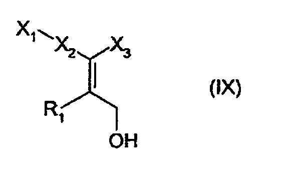
wherein X₁, X₂, X₃, and R₁ are defined as above, and
reacting the compound of formula (IX), wherein X₁, X₂ and X₃, and R₁ are defined as above, except that when X₁, X₂ or X₃ are substituted with hydroxy, this functionality has to be protected with a compound of formula (V) as defined in process (A) above, under Mitsunobu conditions, using a reagent such as triphenylphosphine/diethylazodicarboxylate to obtain a compound of formula (I), wherein X₁, X₂, X₃, Y₁, Y₂, Ar, Z, R₁ and R₂ are defined as above, except that R₂ is not hydrogen; or
(E) Converting the -OH functionality in the compound of formula (IX) as defined in process (D) above, to an appropriate leaving group (L) such as p-toluenesulfonate, methanesulfonate, halogen or triflate to give a compound of formula (X) 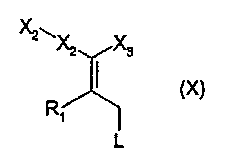
wherein X₁, X₂, X₃, R₁, and L are defined as above,
reacting the compound of formula (X) with a compound of formula (V) as defined in process (A) above, to give a compound of formula (I) wherein X₁, X₂, X₃, Y₁, Y₂, Ar, Z, R₁ and R₂ are defined as above, except that R₂ is not hydrogen; or
(F) Reacting a compound of formula (XI) 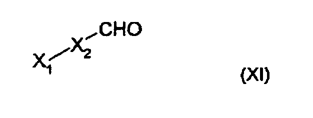
wherein X₁ and X₂ are as defined in claim 1, with carbon tetrabromide and triphenylphosphine to give a compound of formula (XII) 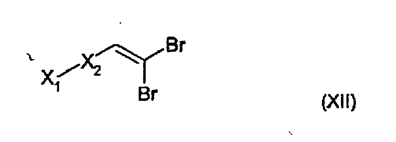
wherein X₁, and X₂ are as defined above,
reacting the compound of formula (XII), with paraformaldehyde in the presence of a strong base, to give a compound of formula (XIII) 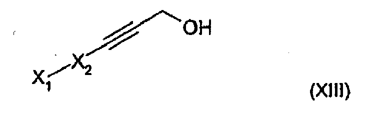
wherein X₁ and X₂ are as defined above,
reducing the compound of formula (XIII), with LiAlH in the presence of a base, followed by treatment with dimethylcarbonate and iodine to give a compound of formula (XIV) 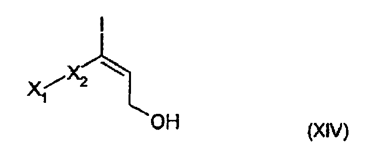
wherein X₁, and X₂ are as defined above,
converting the hydroxyl function in the compound of formula (XIV) to a leaving group (L), as described in (E), to give a compound of formula (XV) 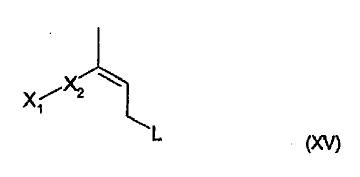
wherein X₁ and X₂ are as defined above and L is a leaving group such as p-toluenesulfonate, methanesulfonate, halogen or triflate,
reacting the compound of formula (XV), with the compound of formula (V), as defined in process (A), to give a compound of formula (XVI) 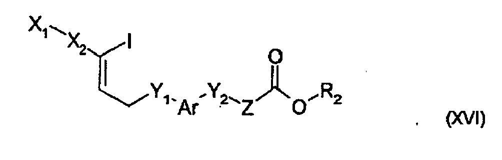
wherein X₁, X₂, Y₁, Y₂, Ar, Z and R₂ are as defined above, except that R₂ is not hydrogen,
reacting the compound of formula (XVI), with X₂-tributyltin in the presence of a palladium catalyst, and tri(t-butyl)phosphine to give the compound of formula (I), wherein X₁, X₂, X₃, Y₁, Y₂, Ar, Z, R₁ and R₂ are defined as above, except that R₁ is hydrogen and R₂ is not hydrogen;
and optionally converting the product of any of (A) to (F) into a pharmaceutically acceptable salt.

## Patentansprüche

1. Verbindung mit der allgemeinen Formel (I): 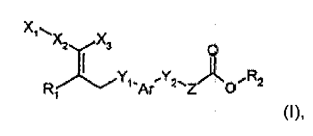
wobei X₁ Aryl oder Heteroaryl ist, wovon jedes optional mit einem oder mehreren Substituenten substituiert ist, die ausgewählt sind aus
• Halogen, Hydroxy, Cyano, Amino oder Carboxy, oder
• C₁₋₆-Alkyl, C₃₋₆-Cycloalkyl, C₂₋₆-Alkenyl, C₂₋₆-Alkinyl, Aryl, Aralkyl, Heteroaryl Heteroaralkyl, C₁₋₆-Alkoxy, C₃₋₆-Cycloalkoxy, Aryloxy, Aralkoxy, Heteroaralkoxy, C₁₋₆-Alkylthio, Arylthio, C₃₋₆-Cycloalkylthio, C₁₋₆-Alkylcarbonyl, Arylcarbonyl; C₁₋₆-Alkylsulfonyl, Arylsulfonyl, C₁₋₆-Alkylsulfonyloxy, Arylsulfonyloxy, C₁₋₆-Alkylamid, Arylamid, C₁₋₆-Alkylaminocarbonyl, C₁₋₆-Alkylarriino, C₁₋₆-Dialkylamino oder C₃₋₆-Cycloalkylamino, wovon jedes optional mit einem oder mehreren Halogenen substituiert ist, und
X₂ Arylen oder Heteroarylen ist, wovon jedes optional mit einem oder mehreren Substituenten substituiert ist, die ausgewählt sind aus
• Halogen, Hydroxy, Cyano, Amino oder Carboxy, oder
• C₁₋₆-Alkyl, C₃₋₆-Cycloalkyl, C₂₋₆-Alkenyl, C₂₋₆-Alkinyl, C₁₋₆-Alkoxy, C₃₋₆-Cycloalkoxy, C₁₋₆-Alkylthio, C₃₋₆-Cycloalkylthio, C₁₋₆-Alkylamiro, C₁₋₆-Dialkylamino oder C₃₋₆-Cycloalkylamino, wovon jedes optional mit einem oder mehreren Halogenen substituiert ist, und
X₃ Aryl oder Heteroaryl ist, wovon jedes optional mit einem oder mehreren Substituenten substituiert ist, die ausgewählt sind aus
• Halogen, Hydroxy, Cyano, Amino oder Carboxy, oder
• C₁₋₆-Alkyl, C₃₋₆-Cycloalkyl, C₂₋₆-Alkenyl, C₂₋₆-Alkinyl, Aralkyl, Heteroaralkyl, C₁₋₆-Alkoxy, C₃₋₆-Cycloalkoxy, Aryloxy, Aralkoxy, Heteroaralkoxy, C₁₋₆-Alkylthio, Arylthio, C₃₋₆-Cycloalkylthio, C₁₋₆-Alkylcarbonyl, Arylcarbonyl, C₁₋₆-Alkylsulfonyl, Arylsulfonyl, C₁₋₆-Alkylsulfonyloxy, Arylsulfonyloxy, C₁₋₆-Alkylamid, Arylamid, C₁₋₆-Alkylaminocarbonyl, C₁₋₆-Alkylamino, C₁₋₆-Dialkylamino oder C₃₋₆-Cycloalkylamino, wovon jedes optional mit einem oder mehreren Halogenen substituiert ist, und
Ar Arylen ist, das optional mit einem oder mehreren Substituenten substituiert ist, die ausgewählt sind aus
• Halogen, Hydroxy oder Cyano, oder
• C₁₋₆-Alkyl, C₃₋₆-Cycloalkyl, C₂₋₆-Alkenyl, C₂₋₆-Alkinyl, Aryl, Heteroaryl, Aralkyl, Heteroaralkyl, C₁₋₆-Alkaxy, C₃₋₆-Cycloalkoxy, Aryloxy, Aralkoxy, Heteroaralkoxy, C₁₋₆-Alkylthio, Arylthio oder C₃₋₆-Cycloalkylthio, wovon jedes optional mit einem oder mehreren Halogenen substituiert ist, und
Y₁ O oder S ist und
Y₂ O oder S ist und
Z -(CH₂)ₙ- ist, wobei n gleich 1, 2 oder 3 ist, und
R₁ Wasserstoff, Halogen oder ein Substituent ausgewählt aus
• C₁₋₆-Alkyl, C₃₋₆-Cycloalkyl, C₂₋₆-Alkenyl, C₂₋₆-Alkinyl, Aralkyl, Heteroaralkyl, C₁₋₆-Alkoxy, C₃₋₆-Cycloalkoxy, Aryloxy, Aralkoxy, Heteroaralkoxy, C₁₋₆-Alkylthio, Arylthio oder C₃₋₆-Cycloalkylthio ist, wovon jedes optional mit einem oder mehreren Halogenen substituiert ist, und
R₂ Wasserstoff, C₁₋₆-Alkyl, C₃₋₆-Cycloalkyl, C₂₋₆-Alkenyl, C₂₋₆-Alkiny, C₄₋₆-Alkeninyl oder Aryl ist, oder
ein pharmazeutisch akzeptables Salz davon oder ein pharmazeutisch akzeptables Solvat davon oder beliebige tautomere Formen, Stereoisomere, Mischungen von Stereoisomeren darunter eine racemische Mischung oder Polymorphe.

2. Verbindung nach Anspruch 1, wobei X₁ Aryl oder Heteroaryl ist, das optional mit einem oder mehreren Substituenten substituiert ist, die ausgewählt sind aus
• Halogen oder
• C₁₋₆-Alkyl, Aryl, C₁₋₆-Alkoxy, C₁₋₆-Alkylsulfonyl oder C₁₋₆-Alkylsulfonyloxy, wovon jedes optional mit einem oder mehreren Halogenen substituiert ist.

3. Verbindung nach Anspruch 2, wobei X₁ Phenyl, Furyl, Thienyl, Benzothienyl oder Benzofuranyl ist, das optional mit einem oder mehreren Substituenten substituiert ist, die ausgewählt sind aus
• Halogen oder
• C₁₋₆-Alkyl, Aryl C₁₋₆-Alkoxy, C₁₋₆-Alkylsulfonyl oder C₁₋₆-Alkylsulfonyloxy, wovon jedes optional mit einem oder mehreren Halogenen substituiert ist.

4. Verbindung nach Anspruch 3, wobei X₁ Phenyl ist, das optional mit einem oder mehreren Substituenten substituiert ist, die ausgewählt sind aus Halogen, C₁₋₆-Alkyl, Aryl oder Perhalomethyl.

5. Verbindung nach Anspruch 4, wobei X₁ Phenyl ist, das optional mit einem oder mehreren Substituenten substituiert ist, die ausgewählt sind aus Phenyl oder Trifluormethyl.

6. Verbindung nach Anspruch 5, wobei X₁ Phenyl ist.

7. Verbindung nach Anspruch 3, wobei X₁ Furyl, Thienyl, Benzothienyl oder Benzofuranyl ist, mit einem oder mehreren Substituenten ausgewählt aus Halogen, C₁₋₆-Alkyl, Aryl oder Perhalomethyl.

8. Verbindung nach Anspruch 7, wobei X₁ Furyl ist, das optional mit einem oder mehreren Substituenten substituiert ist, die ausgewählt sind aus Halogen, C₁₋₆-Alkyl, Phenyl oder Trifluormethyl.

9. Verbindung nach Anspruch 7, wobei X₁ Thienyl ist, das optional mit einem oder mehreren Substituenten substituiert ist, die ausgewählt sind aus Halogen, C₁₋₆-Alkyl, Phenyl oder Trifluormeth.

10. Verbindung nach Anspruch 7, wobei X₁ Benzothienyl ist, das optional mit einem oder mehreren Substituenten substituiert ist, die ausgewählt sind aus Halogen, C₁₋₆-Alkyl, Phenyl oder Trifluormethyl.

11. Verbindung nach einem der vorhergehenden Ansprüche, wobei X₂ Arylen oder Heteroarylen ist, das optional mit einem oder mehreren Substituenten substituiert ist, die ausgewählt sind aus
• Halogen oder
• C₁₋₆-Alkyl oder C₁₋₆-Alkoxy, wovon jedes optional mit einem oder mehreren Halogenen substituiert ist.

12. Verbindung nach Anspruch 11, wobei X₂ Phenylen ist, das optional mit einem oder mehreren Substituenten substituiert ist, die ausgewählt sind aus
• Halogen oder
• C₁₋₆-Alkyl oder C₁₋₆-Alkoxy, wovon jedes optional mit einem oder mehreren Halogenen substituiert ist.

13. Verbindung nach Anspruch 12, wobei X₂ Phenylen ist, das optional mit einem oder mehreren Halogenen substituiert ist.

14. Verbindung nach Anspruch 13, wobei X₂ Phenylen ist.

15. Verbindung nach Anspruch 11, wobei X₂ Benzofuranylen ist, das optional mit einem oder mehreren Substituenten substituiert ist, die ausgewählt sind aus
• Halogen oder
• C₁₋₆-Alkyl oder C₁₋₆-Alkoxy, wovon jedes optional mit einem oder mehreren Halogenen substituiert ist.

16. Verbindung nach Anspruch 15, wobei X₂ Benzofuranylen ist, das optional mit C₁₋₆-Alkyl substituiert ist.

17. Verbindung nach einem der vorhergehenden Ansprüche, wobei X₃ Aryl oder Heteroaryl ist, das optional mit einem oder mehreren Substituenten substituiert ist, die ausgewählt sind aus
• Halogen oder
• C₁₋₆-Alkyl, C₁₋₆-Alkoxy, C₁₋₆-Alkylsulfonyl oder C₁₋₆-Alkylsulfonyloxy, wovon jedes optional mit einem oder mehreren Halogenen substituiert ist.

18. Verbindung nach Anspruch 17, wobei X₃ Phenyl, Furyl, Thienyl, Benzothienyl oder Benzofuranyl ist, das optional mit einem oder mehreren Substituenten substituiert ist, die ausgewählt sind aus
• Halogen oder
• C₁₋₆-Alkyl, C₁₋₆-Alkoxy, C₁₋₆-Alkylsulfonyl oder C₁₋₈-Alkylsulfonyloxy, wovon jedes optional mit einem oder mehreren Halogenen substituiert ist.

19. Verbindung nach Anspruch 18, wobei X₃ Phenyl ist, das optional mit einem oder mehreren Halogenen substituiert ist.

20. Verbindung nach Anspruch 18, wobei X₃ Phenyl ist, das optional mit einem oder mehreren Substituenten substituiert ist, die ausgewählt sind aus C₁₋₆-Alkyl oder Perhalomethyl.

21. Verbindung nach Anspruch 18, wobei X₃ Phenyl ist.

22. Verbindung nach Anspruch 18, wobei X₃ Furyl, Thienyl, Benzothienyl oder Benzofuranyl ist, das optional mit einem oder mehreren Substituenten substituiert ist, die ausgewählt sind aus Halogen, C₁₋₆-Alkyl oder Perhalomethyl.

23. Verbindung nach Anspruch 22, wobei X₃ Furyl ist, das optional mit einem oder mehreren Substituenten substituiert ist, die ausgewählt sind aus Halogen, C₁₋₆-Alkyl oder Trifluormethyl.

24. Verbindung nach Anspruch 22, wobei X₃ Thienyl ist, das optional mit einem oder mehreren Substituenten substituiert ist, die ausgewählt sind aus Halogen, C₁₋₆-Alkyl oder Trifluormethyl.

25. Verbindung nach Anspruch 22, wobei X₃ Benzothienyl ist, das optional mit einem oder mehreren Substituenten substituiert ist, die ausgewählt sind aus Halogen, C₁₋₆-Alkyl oder Trifluormethyl.

26. Verbindung nach Anspruch 22, wobei X₃ Benzofuranyl ist, das optional mit einem oder mehreren Substituenten substituiert ist, die ausgewählt sind aus Halogen, C₁₋₆-Alkyl oder Trifluormethyl,

27. Verbindung nach einem der vorhergehenden Ansprüche, wobei Ar Phenylen ist, das optional mit einem oder mehreren Substituenten substituiert ist, die ausgewählt sind aus
• Halogen, Hydroxy oder Cyano, oder
• C₁₋₆-Alkyl, C₃₋₆-Cycloalkyl, C₂₋₆-Alkenyl, C₂₋₆-Alkinyl, Aryl, Heteroaryl, Aralkyl, Heteroaralkyl, C₁₋₆-Alkoxy, C₃₋₆-Cycloalkoxy, Aryloxy, Aralkoxy, Heteroaralkoxy, C₁₋₆-Alkylthio, Arylthio oder C₃₋₆-cycloalkylthio, wovon jedes optional mit einem oder mehreren Halogenen substituiert ist.

28. Verbindung nach Anspruch 27, wobei Ar Phenylen ist, das optional mit einem oder mehreren Substituenten substituiert ist, die ausgewählt sind aus
• Halogen oder
• C₁₋₆-Alkyl, C₁₋₆-Alkoxy, Aryloxy oder Aralkoxy, das optional mit einem oder mehreren Halogenen substituiert ist.

29. Verbindung nach Anspruch 28, wobei Ar Phenylen ist, das optional mit Methyl substituiert ist.

30. Verbindung nach Anspruch 29, wobei Ar Phenylen ist.

31. Verbindung nach einem der vorhergehenden Ansprüche, wobei Y₁ S ist.

32. Verbindung nach einem der vorhergehenden Ansprüche, wobei Y₁ O ist.

33. Verbindung nach einem der vorhergehenden Ansprüche, wobei n gleich 1 ist.

34. Verbindung nach einem der vorhergehenden Ansprüche, wobei R₁ Wasserstoff oder ein Substituent ausgewählt aus C₁₋₆-Alkyl, Aralkyl, C₁₋₆-Alkoxy, Aryloxy, Aralkoxy ist, wovon jedes optional mit einem oder mehreren Halogenen substituiert ist.

35. Verbindung nach Anspruch 34, wobei R₁ Wasserstoff oder ein Substituent ausgewählt aus C₁₋₆-Alkyl oder C₁₋₆-Alkoxy ist, das optional mit einem oder mehreren Halogenen substituiert ist.

36. Verbindung nach Anspruch 35, wobei R₁ Wasserstoff ist.

37. Verbindung nach einem der vorhergehenden Ansprüche, wobei R₂ Wasserstoff ist.

38. Verbindung nach einem der vorhergehenden Ansprüche, wobei R₂ Methyl oder Ethyl ist.

39. Verbindung nach einem der vorhergehenden Ansprüche, die
(E/Z) {4-[3-Biphenyl-4-yl-3-(4-brom-phenyl)-allylsulfanyl]-phenoxy}-essigsäure,
(E/Z) {4-[3-(4-Brom-phenyl)-3-(3'-trifluormethyl-biphenyl-4-yl)-allylsulfanyl]-2-methyl-phenoxy}-essigsäure oder
ein pharmazeutisch akzeptables Salz davon oder ein pharmazeutisch akzeptables Solvat davon oder beliebige tautomere Formen, Stereoisomere, Mischungen von Stereoisomeren darunter eine racemische Mischung oder Polymorphe darstellt.

40. Verbindung nach einem der Ansprüche 1 bis 38, die
{4-[3-(4-Brom-phenyl)-3-[1,1',4',1"]-terphenyl-4"-yl-allylsulfanyl]-2-methylphenoxy}-essigsäure,
(E/Z)-[2-Methyl-4-[3-[5-(5-methylthiophen-2-yl)-benzo[b]furan-2-yl]-3-(thiophen-2-yl)-allylsulfanyl]-phenoxy]-essigsäure,
(E/Z)-[4-[3-(Biphenyl-4-yl)-3-(furan-2-yl)-allylsulfanyl]-2-methyl-phenoxy]-essigsäure,
(E/Z)-[4-[3-(Benzo[b]thiophen-3-yl)-3-(biphenyl-4-yl)-allylsulfanyl]-2-methyl-phenoxy]-essigsäure,
[4-[(3-Benzo[b]thiophen-2-yl)-3-(biphenyl-4-yl)-allylsulfanyl]-2-methylphenoxyl-essigsäure,
(E/Z)-[4-[3-(4-Biphenylyl)-3-(5-methylthiophen-2-yl)-allylsulfanyl]-2-methyl-phenoxy]-essigsäure oder
ein pharmazeutisch akzeptables Salz davon oder ein pharmazeutisch akzeptables Solvat davon oder beliebige tautomere Formen, Stereoisomere, Mischungen von Stereoisomeren darunter eine racemische Mischung oder Polymorphe darstellt.

41. Verbindung nach einem der Ansprüche 1 bis 38, die
(E) {4-[3-Biphenyl-4-yl-3-(2-fluor-phenyl)-allylsulfanyl]-phenoxy}-essigSäure,
(Z) {4-[3-Biphenyl-4-yl-3-(2-fluor-phenyl)-allylsulfanyl]-phenoxy}-essigsäure,
(E) {4-[3-Biphenyl-4-yl-3-(2-chlor-phenyl)-allylsulfanyl]-phenoxy}-essigsäure,
(Z) {4-[3-Biphenyl-4-yl-3-(2-chlor-phenyl)-allylsulfanyl]-phenoxy}-essigsäure,
(E) {4-[3-Biphenyl-4-yl-3-(2-brom-phenyl)-allylsulfanyl]-phenoxy}-essigsäure,
(Z) {4-[3-Biphenyl-4-yl-3-(2-brom-phenyl)-allylsulfanyl]-phenoxy}-essigsäure,
(E) {4-[3-Biphenyl-4-yl-3-(2-iod-phenyl)-allylsulfanyl]-phenoxy}-essigsäure,
(Z) {4-[3-Biphenyl-4-yl-3-(2-iod-phenyl)-allylsulfanyl]-phenoxy}-essigsäure,
(E) {4-[3-Biphenyl-4-yl-3-(2-methoxy-phenyl)-allylsulfanyl]-phenoxy}-essigsäure,
(Z) {4-[3-Biphenyl-4-y1-3-(2-methoxy-phenyl)-allylsulfanyl]-phenoxy}-essigsäure,
(E) {4-[3-Biphenyl-4-yl-3-(2-trifluormethoxy-phenyl)-allylsulfanyl]-phenoxy}-essigsäure,
(Z) {4-[3-Biphenyl-4-yl-3-(2-trifluormethoxy-phenyl)-allylsulfanyl]-phenoxy}-essigsäure,
(E) {4-[3-Biphenyl-4-yl-3-(2-methyl-phenyl)-allylsulfanyl]-phenoxy}-essigsäure,
(Z) {4-[3-Biphenyl-4-yl-3-(2-methyl-phenyyl)-allylsulfanyl]-phenoxy}-essigsäure,
(E) {4-[3-Biphenyl-4-yl-3-(2-trifluormethyl-phenyl)-allylsulfanyl]-phenoxy}-essigsäure,
(Z) {4-[3-Biphenyl-4-yl-3-(2-trifluormethyl-phenyl)-allylsulfanyl]-phenoxy}-essigsäure,
(E) {4-[3-Biphenyl-4-yl-3-(3-fluor-phenyl)-allylsulfanyl]-phenoxy}-essigsäure,
(Z) {4-[3-Biphenyl-4-yl-3-(3-fluor-phenyl)-allylsulfanyl]-phenoxy}-essigsäure,
(E) {4-[3-Biphenyl-4-yl-3-(3-chlor-phenyl)-allylsulfanyl]-phenoxy}-essigsäure,
(Z) {4-[3-Biphenyl-4-yl-3-(3-chlor-phenyl)-allylsulfanyl]-phenoxy}-essigsäure,
(E) {4-[3-Biphenyl-4-yl-3-(3-brom-phenyl)-allylsulfanyl]-phenoxy}-essigsäure,
(Z) {4-[3-Biphenyl-4-yl-3-(3-brom-phenyl)-allylsulfanyl]-phenoxy}-essigsäure,
(E) {4-[3-Biphenyl-4-yl-3-(3-iod-phenyl)-allylsulfanyl]-phenoxy}-essigsäure,
(Z) {4-[3-Biphenyl-4-yl-3-(3-iod-phenyl)-allylsulfanyl]-phenoxy}-essigsäure,
(E) {4-[3-Biphenyl-4-yl-3-(3-methoxy-phenyl)-allylsulfanyl]-phenoxy}-essigsäure,
(Z) {4-[3-Biphenyl-4-yl-3-(3-methoxy-phenyl)-allylsulfanyl]-phenoxy}-essigsäure,
(E) {4-[3-Biphenyl-4-yl-3-(3-trifluormethoxy-phenyl)-allylsulfanyl]-phenoxy}-essigsäure,
(Z) {4-[3-Biphenyl-4-yl-3-(3-trifluomethoxy-phenyl)-allylsulfanyl]-phenoxy}-essigsäure,
(E) {4-[3-Biphenyl-4-yl-3-(3-methyl-phenyl)-allylsulfanyl]-phenoxy}-essigsäure,
(Z) {4-[3-Biphenyl-4-yl-3-(3-methyl-phenyl)-allylsulfanyl]-phenoxy}-essigsäure,
(E) {4-[3-Biphenyl-4-yl-3-(3-trifluormethyl-phenyl)-allylsulfanyl]-phenoxy}-essigsäure,
(Z) {4-[3-Biphenyl-4-yl-3-(3-trifluormethyl-phenyl)-allylsulfanyl]-phenoxy}-essigsäure,
(E) {4-[3-Biphenyl-4-yl-3-(4-fluor-phenyl)-allylsulfanyl]-phenoxy}-essigsäure,
(Z) {4-[3-Biphenyl-4-yl-3-(4-fluor-phenyl)-allylsulfanyl]-phenoxy}-essigsäure,
(E) {4-[3-Biphenyl-4-yl-3-(4-chlor-phenyl)-allylsulfanyl]-phenoxy}-essigsäure,
(Z) {4-[3-Biphenyl-4-yl-3-(4-chlor-phenyl)-allylsulfanyl]-phenoxy}-essigsäure,
(E/Z) {4-[3-Biphenyl-4-yl-3-(4-brom-phenyl)-allylsulfanyl]-phenoxy}-essigsäure,
(E) {4-[3-Bjphenyl-4-yl-3-(4-brom-phenyl)-allylsulfanyl]-phenoxy}-essigsäure,
(Z) {4-[3-Biphenyl-4-yl-3-(4-brom-phenyl)-allylsulfanyl]-phenoxy}-essigsäure,
(E) {4-[3-Biphenyl-4-yl-3-(4-iod-phenyl)-allylsulfanyl]-phenoxy}-essigsäure,
(Z) {4-[3-Biphenyl-4-yl-3-(4-iod-phenyl)-allylsulfanyl]-phenoxy}-essigsäure,
(E) {4-[3-Biphenyl-4-yl-3-(4-methoxy-phenyl)-allylsulfanyl]-phenoxy}-essigsäure,
(Z) {4-[3-Biphenyl-4-yl-3-(4-methoxy-phenyl)-allylsulfanyl]-phenoxy}-essigsäure,
(E) {4-[3-Biphenyl-4-yl-3-(4-trifluormethoxy-phenyl)-allylsulfanyl]-phenoxy}-essigsäure,
(Z) {4-[3-Biphenyl-4-yl-3-(4-trifluormethoxy-phenyl)-allylsulfanyl]-phenoxy}essigsäure,
(E) {4-[3-Biphenyl-4-yl-3-(4-methyl-phenyl)-allylsulfanyl]-phenoxy}-essigsäure,
(Z) {4-[3-Biphenyl-4-yl-3-(4-methyl-phenyl)-allylsulfanyl]-phenoxy}-essigsäure,
(E) {4-[3-Biphenyl-4-yl-3-(4-trifluormethyl-phenyl)-allylsulfanyl]-phenoxy}-essigsäure,
(Z) {4-[3-Biphenyl-4-yl-3-(4-trifluormethyl-phenyl)-allylsulfanyl]-phenoxy}-essigsäure,
(E) {4-[3-(4-Fluor-phenyl)-3-(2'-trifluormethyl-biphenyl-4-yl)-allylsulfanyl]-2-methyl-phenoxy}-essigsäure,
(Z) {4-[3-(4-Fluor-phenyl)-3-(2'-trifluormethyl-biphenyl-4-yl)-allylsulfanyl]-2-methyl-phenoxy}-essigsäure,
(E/Z) {4-[3-(4-Fluor-phenyl)-3-(3'-trifluormethyl-biphenyl-4-yl)-allylsulfanyl]-2-methyl-phenoxy}-essigsäure,
(E) {4-[3-(4-Fluor-phenyl)-3-(3'-trifluormethyl-biphenyl-4-yl)-allylsulfanyl]-2-methyl-phenoxy}-essigsäure,
(Z) {4-[3-(4-Fluor-phenyl)-3-(3'-trifluormethyl-biphenyl-4-yl)-allylsulfanyl]-2-methyl-phenouy}-essigsäure,
(E) {4-[3-(4-Fluor-phenyl)-3-(4'-trifluormethyl-biphenyl-4-yl)-allylsulfanyl]-2-methyl-phenoxy}-essigsäure,
(Z) {4-[3-(4-Fluor-phenyl)-3-(4'-trifluormethyl-biphenyl-4-yl)-allylsulfanyl]. 2-methyl-phenoxy}-essigsäure,
(E) {4-[3-(4-Fluor-phenyl)-3-(3'-chlor-biphenyl-4-yl)-allylsulfanyl]-2-methyl-phenoxy}-essigsäure,
(Z) {4-[3-(4-Fluor-phenyl)-3-(3'-chlor-biphenyl-4-yl)-allylsulfanyl]-2-methyl-phonoxyl-essigsäure,
(E) {4-[3-(4-Fluor-phenyl)-3-(4'-chlor-biphenyl-4-yl)-allylsulfanyl]-2-methyl-phenoxy}-essigsäure,
(Z) {4-[3-(4-Fluor-phenyl)-3-(4'-chlor-biphenyl-4-yl)-allylsulfanyl]-2-methyl-phenoxy}-essigsäure,
(E) {4-[3-(4-Fluor-phenyl)-3-(3'-methoxy-biphenyl-4-yl)-allylsulfanyl]-2-methyl-phenoxy}-essigsäure,
(Z) {4-[3-(4-Fluor-phenyl)-3-(3'-methoxy-biphenyl-4-yl)-allylsulfanyl]-2-methyl-phenoxy}-essigsäure,
(E) {4-[3-(4-Fluorphenyl)-3-(4'-methoxy-biphenyl-4-yl)-allylsulfanyl]-2. methyl-phenoxy}-essigsäure,
(Z) {4-[3-(4-Fluor-phenyl)-3-(4'-methoxy-biphenyl-4-yl)-allylsulfanyl]-2-methyl-phenoxy}-essigsäure,
(E) {4-[3-(4-Chlor-phenyl)-3-(2'-trifluormethyl-biphenyl-4-yl)-allylsullfanyl]-2-methyl-phenoxy}-essigsäure,
(Z) {4-[3-(4-Chlor-phenyl)-3-(2'-trifluormethyl-biphenyl-4-yl)-allylsulfanyl]-2-methyl-phenoxy}-essigsäure,
(E/Z) {4-[3-(4-Chlor-phenyl)-3-(3'-trifluormethyl-biphenyl-4-yl)-allylsulfanyl]-2-methyl-phenoxy}-essigsäure,
(E) {4-[3-(4-Chlor-phenyl)-3-(3'-trifluormethyl-biphenyl-4-yl)-allylsulfanyl]-2-methyl-phenoxy}-essigsäure,
(Z) {4-[3-(4-Chlor-phenyl)-3-(3'-trifluormethyl-biphenyl-4-yl)-allylsufanyl]-2-methyl-phenoxy}-essigsäure,
(E) {4-[3-(4-Chlor-phenyl)-3-(4'-trifluormethyl-biphenyl-4-yl)-allylsulfanyl]-2-methyl-phenoxy}-essigsäure,
(Z) {4-[3-(4-Chlor-phenyl)-3-(4'-trifluormethyl-biphenyl-4-yl)-allylsulfanyl]. 2-methyl-phenoxy}-essigsäure,
(E) {4-[3-(4-Chlor-phenyl)-3-(3'-chlor-biphenyl-4-yl)-allylsulfanyl]-2-methyl-phenoxy}-essigsäure,
(Z) {4-[3-(4-Chlor-phenyl)-3-(3'-chlor-biphenyl-4-yl)-allylsulfanyl]-2-methyl-phenoxy}-essigsäure,
(E) {4-[3-(4-Chlor-phenyl)-3-(4'-chlor-biphenyl-4-yl)-allylsulfanyl]-2-methyl-phenoxy}-essigsäure,
(Z) {4-[3-(4-Chlor-phenyl)-3-(4'-chlor-biphenyl-4-yl)-allylsulfanyl]-2-methyl-phenoxy}-essigsäure,
(E) {4-[3-(4-Chlor-phenyl)-3-(3'-methoxy-biphenyl-4-yl)-allylsulfanyl]-2-methyl-phenoxy}-essigsäure,
(Z) {4-[3-(4-Chlor-phenyl)-3-(3'-methoxy-biphenyl-4-yl)-allylsulfanyl]-2-methyl-phenoxy}-essigsäure,
(E) {4-[3-(4-Chlor-phenyl)-3-(4'-methoxy-biphenyl-4-yl)-allylsulfanyl]-2-methyl-phenoxy}-essigsäure,
(Z) {4-[3-(4-Chlor-phenyl)-3-(4'-methoxy-biphenyl-4-yl)-allylsulfanyl]-2-methyl-phenoxy}-essigsäure,
(E) {4-[3-(4-Brom-phenyl)-3-(2'-trifluormethyl-biphenyl-4-yl)-allylsulfanyl]-2-methyl-phenoxy}-essigsäure,
(Z) {4-[3-(4-Brom-phenyl)-3-(2'-trifluormethyl-biphenyl-4-yl)-allylsullfanyl]-2-methyl-phenoxy}-essigsäure,
(E/Z) {4-[3-(4-Brom-phenyl)-3-(3'-trifluormethyl-biphenyl-4-yl)-allylsulfanyl]-2-methyl-phenoxy}-essigsäure,
(E) {4-[3-(4-Brom-phenyl)-3-(3'-trifluormethyl-biphenyl-4-yl)-allylsulfanyl]-2-methyl-phenoxy}-essigsäure,
(Z) {4-[3-(4-Brom-phenyl)-3-(3'-trifluormethyl-biphenyl-4-yl)-allylsulfanyl]-2-methyl-phenoxy}-essigsäure,
(E) {4-[3-(4-Brom-phenyl)-3-(4'-trifluormethyl-biphenyl-4-yl)-allylsulfanyl]-2-methyl-phenoxy}-essigsäure,
(Z) {4-[3-(4-Brom-phenyl)-3-(4'-trifluormethyl-biphenyl-4-yl)-allylsulfanyl]-2-methyl-phenoxy}-essigsäure,
(E) {4-[3-(4-Brom-phenyl)-3-(3'-chlor-biphenyl-4-yl)-allylsulfanyl]-2-methyl-phenoxy}-essigsäure,
(Z) {4-[3-(4-Brom-phenyl)-3-(3'-chlor-biphenyl-4-yl)-allylsulfanyl]-2-methyl-phenoxy}-essigsäure,
(E) {4-[3-(4-Brom-phenyl)-3-(4'-chlor-biphenyl-4-yl)-allylsulfanyl]-2-methyl-phenoxy}-essigsäure,
(Z) {4-[3-(4-Brom-phenyl)-3-(4'-chlor-biphenyl-4-yl)-allylsulfahyl]-2-methyl-phenoxy}-essigsäure,
(E) {4-[3-(4-Brom-phenyl)-3-(3'-methoxy-biphenyl-4-yl)-allylsulfanyl]-2-methyl-phenoxy}-essigsäure,
(Z) {4-[3-(4-Brom-phenyl)-3-(3'-methoxy-biphenyl-4-yl)-allylsulfanyl]-2-methyl-phenoxy}-essigsäure,
(E) {4-[3-(4-Brom-phenyl)-3-(4'-methoxy-biphenyl-4-yl)-allylsulfanyl]-2-methyl-phenoxy}-essigsäure,
(Z) {4-[3-(4-Brom-phenyl)-3-(4'-methoxy-biphenyl-4-yl)-allylsulfanyl]-2-methyl-phenoxy}-essigsäure oder
ein pharmazeutisch akzeptables Salz davon oder ein pharmazeutisch akzeptables Solvat davon oder beliebige tautomere Formen, Stereoisomere, Mischungen von Stereoisomeren darunter eine racemische Mischung oder Polymorphe darstellt.

42. Kombination aus einer Verbindung wie in einem der Ansprüche 1 bis 41 definiert und einer oder mehreren zusätzlichen pharmakologischen Wirksubstanzen.

43. Kombination nach Anspruch 42, wobei die eine oder mehreren zusätzlichen pharmakologischen Wirksubstanzen ausgewählt sind aus Mitteln gegen Fettleibigkeit, Antidiabetika, Mitteln gegen Hochdruck, Mitteln für die Behandlung und/oder Vorbeugung von Komplikationen, die durch Diabetes bedingt oder damit verbunden sind, und Mitteln für die Behandlung und/oder Vorbeugung von Komplikationen und Störungen, die durch Fettleibigkeit bedingt oder damit verbunden sind.

44. Pharmazeutische Zusammensetzung umfassend als Wirkstoff mindestens eine Verbindung wie in einem der Ansprüche 1 bis 41 definiert oder eine Kombination nach Anspruch 42 oder 43 zusammen mit einem oder mehreren pharmazeutisch akzeptablen Trägern oder Exzipienten.

45. Pharmazeutische Zusammensetzung nach Anspruch 44 in Form von Dosiseinheiten, umfassend von ungefähr 0,05 mg bis ungefähr 1000 mg, bevorzugt von ungefähr 0,1 mg bis ungefähr 500 mg und besonders bevorzugt von ungefähr 0,5 mg bis ungefähr 200 mg pro Tag einer Verbindung nach einem der Ansprüche 1 bis 41.

46. Pharmazeutische Zusammensetzung nach Anspruch 44 oder Anspruch 45 zur oralen, nasalen, transdermalen, pulmonalen oder parenteralen Verabreichung.

47. Verbindung wie in einem der Ansprüche 1 bis 41 definiert, eine Kombination wie in Anspruch 42 oder 43 definiert oder eine Zusammensetzung wie in einem der Ansprüche 44 bis 46 definiert zur Verwendung als Medikament.

48. Verbindung, Kombination oder Zusammensetzung nach Anspruch 47 für die Behandlung und/oder Vorbeugung von Zuständen, die durch Peroxisom-Proliferator-aktivierte Rezeptoren (PPAR) mediiert sind.

49. Verbindung, Kombination oder Zusammensetzung nach Anspruch 47 für die Behandlung und/oder Vorbeugung von Diabetes Typ 1, Diabetes Typ 2, Dyslipidämie, Syndrom X, darunter metabolisches Syndrom, gestörte Glucosetoleranz, Insulinresistenz, Hypertriglyceridämie und/oder Fettleibigkeit, kardiovaskuläre Erkrankungen, darunter Artheriosklerose, und Hypercholersterämie.

50. Verbindung, Kombination oder Zusammensetzung nach Anspruch 47 für die Behandlung und/oder Vorbeugung von Hyperglycämie und Hyperlipidämie.

51. Verfahren zur Herstellung einer Verbindung wie in einem der Ansprüche 1 bis 41 definiert, wobei das Verfahren ausgewählt ist aus den unten angeführten Schritten (A) bis (F):
(A) Umsetzen einer Verbindung mit der Formel (II) 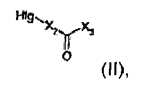
wobei X₂ und X₃ wie in Anspruch 1 definiert sind und wobei Hlg Brom oder Iod ist, durch eine Wittig-ähnliche Reaktion mit (EtO)₂PO(CHR₁)COOR₆, wobei R₆ eine Alkylgruppe ist und R₁ wie in Anspruch 1 definiert ist, in Gegenwart einer Base, so dass eine Verbindung mit der Formel (III) 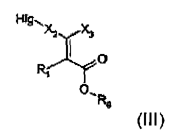
gebildet wird, wobei X₂, X₃, R₁ und R₆ wie oben definiert sind und wobei Hlg Brom oder Iod ist, und
Reduzieren der Verbindung mit der Formel (III), so dass eine Verbindung mit der Formel (IV) 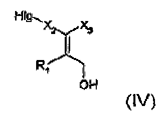
gebildet wird, wobei X₂, X₃ und R₁ wie oben definiert sind und wobei Hlg Brom oder Iod ist, und
Umsetzen der Verbindung mit der Formel (IV), wobei X₂, X₃ und R₁ wie oben definiert sind und wobei HIg Brom oder Iod ist, mit der Ausnahme, dass wenn X₂ oder X₃ mit Hydroxy substituiert sind, diese Funktionalität geschützt sein muss, mit einer Verbindung der Formel (V) 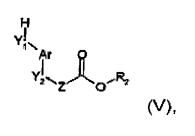
wobei Y₁, Ar, Y₂, Z und R₂ wie in Anspruch 1 definiert sind, mit der Ausnahme, dass R₂ kein Wasserstoff ist, unter Mitsunobu-Bedingungen unter Verwendung eines Reagens wie Triphenylphosphin/Diethylazodicarboxylat, so dass eine Verbindung mit der Formel (VI) 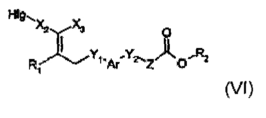
erhalten wird, wobei X₂, X₃, Y₁, Y₂, Ar, Z, R₁ und R₂ wie oben definiert sind, mit der Ausnahme, dass R₂ kein Wasserstoff ist und wobei Hlg Brom oder Iod ist, und
Umsetzen einer Verbindung mit der Formel (VI) mit einer Borsäure oder mit einem Tributylzinnderivat von X₁ unter geeigneten Kupplungsbedingungen, so dass eine Verbindung mit der Formel (I) gebildet wird, wobei X₁, X₂, X₃, Y₁, Y₂, Ar, Z, R₁ und R₂ wie oben definiert sind, mit der Ausnahme, dass R₂ kein Wasserstoff ist, oder
(B) chemisches oder enzymatisches Verseifen einer Verbindung mit der Formel (I), wobei X₁, X₂, X₃, Y₁, Y₂, Ar, Z, R₁ und R₂ wie in Anspruch 1 definiert sind, mit der Ausnahme, dass R₂ kein Wasserstoff ist, so dass eine Verbindung mit der Formel (I) gebildet wird, wobei X₁, X₂, X₃, Y₁, Y₂, Ar, Z, R₁ und R₂ wie oben definiert sind, mit der Ausnahme, dass R₂ Wasserstoff ist, oder
(C) chemisches oder enzymatisches Verseifen einer Verbindung mit der Formel (VI), wobei X₂, X₃, Y₁, Y₂, Ar, Z, R₁ und R₂ wie in Anspruch 1 definiert sind, mit der Ausnahme, dass R₂ kein Wasserstoff ist und wobei Hlg Brom oder Iod ist, so dass eine Verbindung mit der Formel (VI) gebildet wird, wobei X₁, X₂, X₃, Y₁, Y₂, Ar, Z, R₁ und R₂ wie oben definiert sind, mit der Ausnahme, dass R₂ Wasserstoff ist und wobei Hlg Brom oder Iod ist, und
Umsetzen einer Verbindung mit der Formel (VI) mit einem Borsäurederivat von X₁ unter geeigneten Kupplungsbedingungen, so dass eine Verbindung mit der Formel (I) gebildet wird, wobei X₁, X₂, X₃, Y₁, Y₂, Ar, Z, R₁ und R₂ wie oben definiert sind, mit der Ausnahme, dass R₂ Wasserstoff ist, oder
(D) Umsetzen einer Verbindung mit der Formel (VII) 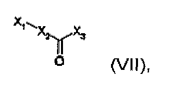
wobei X₁, X₂ und X₃ wie in Anspruch 1 definiert sind, durch eine Wittig-ähnliche Reaktion mit (EtO)₂PO(CHR₁)COOR₆, wobei R₆ eine Alkylgruppe ist und R₁ wie in Anspruch 1 definiert ist, in Gegenwart einer Base, so dass eine Verbindung mit der Formel (VIII) 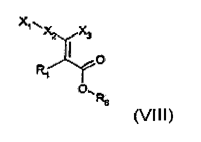
gebildet wird, wobei X₁, X₂, X₃, R₁ und R₆ wie oben definiert sind, und
Reduzieren der Verbindung mit der Formel (VIII), wobei X₁, X₂, X₃, R₁ und R₆ wie oben definiert sind, so dass eine Verbindung mit der Formel (IX) 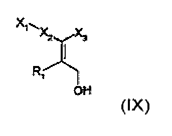
gebildet wird, wobei X₁, X₂, X₃ und R₁ wie oben definiert sind, und
Umsetzen der Verbindung mit der Formel (IX), wobei X₁, X₂ und X₃ und R₁ wie oben definiert sind, mit der Ausnahme, dass, wenn X₁, X₂ oder X₃ mit Hydroxy substituiert ist, diese Funktionalität geschützt sein muss, mit einer Verbindung der Formel (V) wie im Schritt (A) oben definiert, unter Mitsunobu-Bedingungen unter Verwendung eines Reagens wie Triphenylphosphin/Diethylazodicarboxylat, so dass eine Verbindung mit der Formel (I) erhalten wird, wobei X₁, X₂, X₃, Y₁, Y₂, Ar, Z, R₁ und R₂ wie oben definiert sind, mit der Ausnahme, dass R₂ kein Wasserstoff ist, oder
(E) Umwandeln der -OH-Funktionalität in der Verbindung mit der Formel (IX) wie im Schritt (D) oben definiert, in eine geeignete Austrittsgruppe (L) wie p-Toluolsulfonat, Methansulfonat, Halogen oder Triflat, so dass eine Verbindung mit der Formel (X) 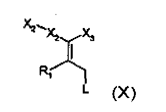
gebildet wird, wobei X₁, X₂, X₃, R₁ und L wie oben definiert sind,
Umsetzen der Verbindung mit der Formel (X) mit einer Verbindung mit der Formel (V), wie im Schritt (A) oben definiert, so dass eine Verbindung mit der Formel (I) erhalten wird, wobei X₁, X₂, X₃, Y₁, Y₂, Ar, Z, R₁ und R₂ wie oben definiert sind, mit der Ausnahme, dass R₂ kein Wasserstoff ist, oder
(F) Umsetzen einer Verbindung mit der Formel (XI) 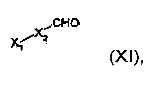
wobei X₁ und X₂ wie in Anspruch 1 definiert sind, mit Kohlenstofftetrabromid und Triphenylphosphin, so dass eine Verbindung mit der Formel (XII) 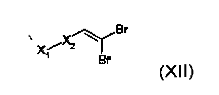
gebildet wird, wobei X₁ und X₂ wie oben definiert sind,
Umsetzen der Verbindung mit der Formel (XII) mit Paraformaldelhyd in Gegenwart einer starken Base, so dass eine Verbindung mit der Formel (XIII) 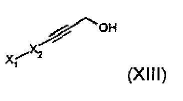
gebildet wird, wobei X₁ und X₂ wie oben definiert sind,
Reduzieren der Verbindung mit der Formel (XIII) mit LiAlH in Gegenwart einer Base, gefolgt von einer Behandlung mit Dimethylcarbonat und Iod, so dass eine Verbindung mit der Formel (XIV) 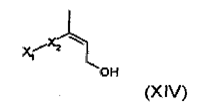
gebildet wird, wobei X₁ und X₂ wie oben definiert sind,
Umwandeln der Hydroxylfunktion in der Verbindung mit der Formel (XIV) in eine Austrittsgruppe (L), wie in (E) beschrieben, so dass eine Verbindung mit der Formel (XV) 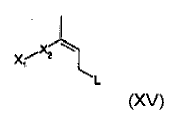
gebildet wird, wobei X₁ und X₂ wie oben definiert sind und L eine Austrittsgruppe wie p-Toluolsulfonat, Methansulfonat, Halogen oder Triflat ist,
Umsetzen der Verbindung mit der Formel (XV) mit der Verbindung mit der Formel (V), wie in Schritt (A) definiert, so dass eine Verbindung mit der Formel (XVI) 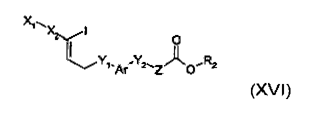
gebildet wird, wobei X₁, X₂, Y₁, Y₂, Ar, Z und R₂ wie oben definiert sind, mit der Ausnahme, dass R₂ kein Wasserstoff ist,
Umsetzen der Verbindung mit der Formel (XVI) mit X₂-Tributylzinn in Gegenwart eines Palladiumkatalysators und Tri(t-butyl)phosphün, so dass eine Verbindung mit der Formel (I) gebildet wird, wobei X₁, X₂, X₃, Y₁, Y₂, Ar, Z, R₁ und R₂ wie oben definiert sind, mit der Ausnahme, dass R₁ Wasserstoff ist und R₂ kein Wasserstoff ist,
und optional Umwandeln des Produkts aus einem der Schritte (A) bis (F) in ein pharmazeutisch akzeptables Salz.

## Revendications

1. Composé de formule (I) générale : 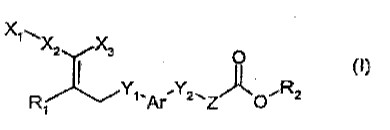
dans laquelle X₁ est un aryle ou un hétéroaryle, chacun étant facultativement substitué avec un ou plusieurs substituants choisis parmi :
- un halogène, un hydroxy, un cyano, un amino, ou un carboxy ; ou
- un alkyle en C₁ à C₆, un cycloalkyle en C₃ à C₆, un alcényle en C₂ à C₆, un alcynyle en C₂ à C₆, un aryle, un aralkyle, un hétéroaryle, un hétéroaralkyle, un alcoxy en C₁ à C₆, un cycloalcoxy en C₃ à C₆, un aryloxy, un aralcoxy, un hétéroaralcoxy, un alkylthio en C₁ à C₆, un arylthio, un cycloalkylthio en C₃ à C₆, un alkylcarbonyle en C₁ à C₆, un aryl-carbonyle, un alkylsulfonyle en C₁ à C₆, un arylsulfonyle, un alkylsulfonyloxy en C₁ à C₆, un arylsulfonyloxy, un alkylamido en C₁ à C₆, un arylamido, un (alkyl en C₁ à C₆) amino-carbonyle, un alkylamino en C₁ à C₆, un dialkylamino en C₁ à C₆ ou un cycloalkylamino en C₃ à C₆, chacun étant facultativement substitué avec un ou plusieurs halogènes ; et
X₂ est un arylène ou un hétéroarylène, chacun étant facultativement substitué avec un ou plusieurs substituants choisis parmi :
- un halogène, un hydroxy, un cyano, un amino ou un carboxy ; ou
- un alkyle en C₁ à C₆, un cycloalkyle en C₃ à C₆, un alcényle en C₂ à C₆, un alcynyle en C₂ à C₆, un alcoxy en C₁ à C₆, un cycloalcoxy en C₃ à C₆, un alkylthio en C₁ à C₆, un cycloalkylthio en C₃, à C₆, un alkylamino en C₁ à C₆, un dialkylamino en C₁ à C₆ ou un cycloalkylamino en C₃ à C₆, chacun étant facultativement substitué avec un ou plusieurs halogènes ; et
X₃ est un aryle ou un hétéroaryle, chacun étant facultativement substitué avec un ou plusieurs substituants choisis parmi :
- un halogène, un hydroxy, un cyano, un amino ou un carboxy ; ou
- un alkyle en C₁ à C₆, un cycloalkyle en C₃ à C₆, un alcényle en C₂ à C₆, un alcynyle en C₂ à C₆, un aralkyle, un hétéroaralkyle, un alcoxy en C₁ à C₆, un cycloalcoxy en C₃ à C₆, un aryloxy, un aralcoxy, un hétéroaralcoxy, un alkylthio en C₁ à C₆, un arylthio, un cycloalkylthio en C₃ à C₆, un alkylcarbonyle en C₁ à C₆, un aryl-carbonyle, un alkylsulfonyle en C₁ à C₆, un arylsulfonyle, un alkylsulfonyloxy en C₁ à C₆, un arylsulfonyloxy, un alkylamido en C₁ à C₆, un arylamido, un (alkyl en C₁ à C₆)amino-carbonyle, un alkylamino en C₁ à C₆, un dialkylamino en C₁ à C₆ ou un cycloalkylamino en C₃ à C₆, chacun étant facultativement substitué avec un ou plusieurs halogènes ; et
Ar est un arylène qui est facultativement substitué avec un ou plusieurs substituants choisis parmi :
- un halogène, un hydroxy ou un cyano ; ou
- un alkyle en C₁ à C₆, un cycloalkyle en C₃ à C₆, un alcényle en C₂ à C₆, un alcynyle en C₂ à C₆, un aryle, un hétéroaryle, un aralkyle un hétéroaralkyle, un alcoxy en C₁ à C₆, un cycloalcoxy en C₃ à C₆, un aryloxy, un aralcoxy, un hétéroaralcoxy, un alkylthio en C₁ à C₆, un arylthio ou un cycloalkylthio en C₃ à C₆, chacun étant facultativement substitué avec un ou plusieurs halogénes ; et
Y₁ est O ou S ; et
Y₂ est O ou S ; et
Z est -(CH₂)ₙ-, où n est 1, 2 ou 3 ; et
R₁ est un hydrogène, un halogène ou un substituant choisi parmi :
- un alkyle en C₁ à C₆, un cycloalkyle en C₃ à C₆, un alcényle en C₂ à C₆, un alcynyle en C₂ à C₆, un aralkyle, un hétéroaralkyle, un alcoxy en C₁ à C₆, un cycloalcoxy en C₃ à C₆, un aryloxy, un aralcoxy, un hétéroaralcoxy, un alkylthio en C₁ à C₆, un arylthio ou un cycloalkylthio en C₃ à C₆, chacun étant facultativement substitué avec un ou plusieurs halogénes ; et
R₂ est un hydrogène, une alkyle en C₁ à C₆, un cycloalkyle en C₃ à C₆, un alcényle en C₂ à C₆, un alcynyle en C₂ à C₆, un alcénynyle en C₄ à C₆ ou un aryle ; ou
l'un de ses sels pharmaceutiquement acceptables, ou l'un de ses solvates pharmaceutiquement acceptables, ou toutes formes tautomères, tous stéréoisomères, tout mélange de stéréoisomères y compris un mélange racémique, ou tous polymorphes.

2. Composé selon la revendication 1, dans lequel X₁ est un aryle ou un hétéroaryle facultativement substitué avec un ou plusieurs substituants choisis parmi :
- un halogène ; ou
- un alkyle en C₁ à C₆, un aryle, un alcoxy en C₁ à C₆, un alkylsulfonyle en C₁ à C₆ ou un alkylsulfonyloxy en C₁ à C₆, chacun étant facultativement substitué avec un ou plusieurs halogènes.

3. Composé selon la revendication 2, dans lequel X₁ est un phényle, un furyle, un thiényle, un benzothiényle ou un benzofuranyle facultativement substitué avec un ou plusieurs substituants choisis parmi :
- un halogène ; ou
- un alkyle en C₁ à C₆, un aryle, un alcoxy en C₁ à C₆, un alkylsulfonyle en C₁ à C₆ ou un alkylsulfonyloxy en C₁ à C₆, chacun étant facultativement substitué avec un ou plusieurs halogènes.

4. Composé selon la revendication 3, dans lequel X₁ est un phényle facultativement substitué avec un ou plusieurs substituants choisis parmi un halogène, un alkyle en C₁ à C₆, un aryle ou un perhalogénométhyle.

5. Composé selon la revendication 4, dans lequel X₁ est un phényle facultativement substitué avec un ou plusieurs substituants choisis parmi un phényle ou un trifluorométhyle.

6. Composé selon la revendication 5, dans lequel X₁ est un phényle.

7. Composé selon la revendication 3, dans lequel X₁ est un furyle, un thiényle, un benzothiényle ou un benzofuranyle avec un ou plusieurs substituents choisis parmi un halogène, un alkyl en C₁ à C₆, un aryle ou un perhalogénométhyle.

8. Composé selon la revendication 7, dans lequel X₁ est un furyle facultativement substitué avec un ou plusieurs substituants choisis parmi un halogène, un alkyle en C₁ à C₆, un phényle ou un trifluorométhyle.

9. Composé selon la revendication 7, dans lequel X₁ est un thiényle facultativement substitué avec un ou plusieurs substituants choisis parmi un halogène, un alkyle en C₁ à C₆, un phényle ou un trifluorométhyle.

10. Composé selon la revendication 7, dans lequel X₁ est un benzothiényle facultativement substitué avec un ou plusieurs substituants choisis parmi un halogène, un alkyle en C₁ à C₆, un phényle ou un trifluorométhyle.

11. Composé selon l'une quelconque des revendications précédentes, dans lequel X₂ est un arylene ou un hétéroarylène facultativement substitué avec un ou plusieurs substituants choisis parmi :
- un halogène ; ou
- un alkyle en C₁ à C₆ ou un alcoxy en C₁ à C₆, chacun étant facultativement substitué avec un ou plusieurs halogènes.

12. Composé selon la revendication 11, dans lequel X₂ est un phénylène facultativement substitué avec un ou plusieurs substituants choisis parmi :
- un halogène ; ou
- un alkyle en C₁ à C₆ ou un alcoxy en C₁ à C₆, chacun étant facultativement substitué avec un ou plusieurs halogènes.

13. Composé selon la revendication 12, dans lequel X₂ est un phénylène facultativement substitué avec un ou plusieurs halogènes.

14. Composé selon la revendication 13, dans lequel X₂ est un phénylène.

15. Composé selon la revendication 11, dans lequel X₂ est un benzofuranylène facultativement substitué avec un ou plusieurs substituants choisis parmi :
- un halogéne ; ou
- un alkyle en C₁ à C₆ ou un alcoxy en C₁ à C₆, chacun étant facultativement substitué avec un ou plusieurs halogènes.

16. Composé selon la revendication 15, dans lequel X₂ est un benzofuranylène facultativement substitué avec un alkyle en C₁ à C₆.

17. Composé selon l'une quelconque des revendications précédentes, dans lequel X₃ est un aryle ou un hétéroaryle facultativement substitué avec un ou plusieurs substituants choisis parmi :
- un halogène ; ou
- un alkyle en C₁ à C₆, une alcoxy en C₁ à C₆, un alkylsulfonyle en C₁ à C₆ ou un alkyl-sulfonyloxy en C₁ à C₆, chacun étant facultativement substitué avec un ou plusieurs halogènes.

18. Composé selon la revendication 17, dans lequel X₃ est un phényle, un furyle, un thiényle, un benzothiényle ou un benzofuranyl facultativement substitué avec un ou plusieurs substituants choisis parmi :
- un halogène ; ou
- un alkyle en C₁ à C₆, un alcoxy en C₁ à C₆, un alkylsulfonyle en C₁ à C₆ ou un alkyl-sulfonyloxy en C₁ à C₆, chacun étant facultativement substitué avec un ou plusieurs halogènes.

19. Composé selon la revendication 18, dans lequel X₃ est un phényle facultativement substitué avec un ou plusieurs halogènes.

20. Composé selon la revendication 18, dans lequel X₃ est un phényle facultativement substitué avec un ou plusieurs substituants choisis parmi un alkyle en C₁ à C₆ ou un perhalogénométhyle.

21. Composé selon la revendication 18, dans lequel X₃ est un phényle.

22. Composé selon la revendication 18, dans lequel X₃ est un furyle, un thiényle, un benzothiényle ou un benzofuranyle facultativement substitué avec un ou plusieurs substituants choisis parmi un halogène, un alkyle en C₁ à C₆ ou une perhalogénométhyle.

23. Composé selon la revendication 22, dans lequel X₃ est un furyle facultativement substitué avec un ou plusieurs substituants choisis parmi un halogène, un alkyl en C₁ à C₆ ou un trifluorométhyle.

24. Composé selon la revendication 22, dans lequel X₃ est un thiényle facultativement substitué avec un ou plusieurs substituants choisis parmi un halogène, un alkyle en C₁ à C₆ ou un trifluorométhyle.

25. Composé selon la revendication 22, dans lequel X₃ est un benzothiényle facultativement substitué avec un ou plusieurs substituants choisis parmi un halogène, un alkyle en C₁ à C₆ ou un trifluorométhyle.

26. Composé selon la revendication 22, dans lequel X₃ est un benzofuranyle facultativement substitué avec un ou plusieurs substituants choisis parmi un halogène, un alkyle en C₁ à C₆ ou un trifluorométhyle.

27. Composé selon l'une quelconque des revendications précédentes, dans lequel Ar est un phénylène qui est facultativement substitué avec un ou plusieurs substituants choisis parmi :
- un halogène, un hydroxy ou un cyano ; ou
- un alkyle en C₁ à C₆, un cycloalkyle en C₃ à C₆, un alcényle en C₂ à C₆, un alcynyle en C₂ à C₆, un aryle, un hétéroaryle, un aralkyle, un hétéroaralkyle, un alcoxy en C₁ à C₆, un cycloalcoxy en C₃ à C₆, un aryloxy, un aralcoxy, un hétéroaralcoxy, un alkylthio en C₁ à C₆, un arylthio ou un cycloalkylthio en C₃ à C₆, chacun étant facultativement substitué avec un ou plusieurs halogènes.

28. Composé selon la revendication 27, dans lequel Ar est un phénylène qui est facultativement substitué avec un ou plusieurs substituants choisis parmi :
- un halogène ; ou
- un alkyle en C₁ à C₆, un alcoxy en C₁ à C₆, un aryloxy ou un aralcoxy, chacun étant facultativement substitué avec un ou plusieurs halogènes.

29. Composé selon la revendication 28, dans lequel Ar est un phénylène qui est facultativement substitué avec un méthyle.

30. Composé selon la revendication 29, dans lequel Ar est un phénylène.

31. Composé selon l'une quelconque des revendications précédentes, dans lequel Y₁ est S.

32. Composé selon l'une quelconque des revendications précédentes, dans lequel Y₂ est O.

33. Composé selon l'une quelconque des revendications précédentes, dans lequel n est 1.

34. Composé selon l'une quelconque des revendications précédentes, dans lequel R₁ est un hydrogène ou un substituant choisi parmi un alkyle en C₁ à C₆, un aralkyle, un alcoxy en C₁ à C₆, un aryloxy, un aralcoxy, chacun étant facultativement substitué avec un ou plusieurs halogènes.

35. Composé selon la revendication 34, dans lequel R₁ est un hydrogène ou un substituant choisi parmi un alkyle en C₁ à C₆ ou un alcoxy en C₁ à C₆, chacun étant facultativement substitué avec un ou plusieurs halogènes.

36. Composé selon la revendication 35, dans lequel R₁ est un hydrogène.

37. Composé selon l'une quelconque des revendications précédentes, dans lequel R₂ est un hydrogène.

38. Composé selon l'une quelconque des revendications précédentes, dans lequel R₂ est un méthyle ou un éthyle.

39. Composé selon l'une quelconque des revendications précédentes, qui est :
l'acide (E/Z)-{4-[3-biphényl-4-yl-3-(4-bromo-phényl)-allylsulfanyl]phénoxy}acétique ;
l'acide (E/Z)-{4-[3-(4-bromophényl)-3-(3'-trifluorométhylbiphényl-4-yl)-allylsulfonyl]-2-méthyl-phénoxy}-acétique, ou
l'un de leurs sels pharmaceutiquement acceptable, ou l'un de leurs solvates pharmaceutiquement acceptables, ou toutes formes tautomères, tous stéréoisomères, tout mélange de stéréoisomères y compris un mélange racémique, ou tous polymorphes.

40. Composé selon l'une quelconque des revendications 1 à 38, qui est :
l'acide {4-[3-(4-bromophényl)-3-[1,1';4',1'']-terphényl-4''-ylallylsulfonyl]-2-méthylphénoxy}acétique ;
l'acide (E/Z)-[2-méthyl-4-[3-[5-(5-méthylthiophén-2-yl)benzo[b]furan-2-yl]-3-(thiophén-2-yl)-allylsulfanyl]phénoxy]acétique ;
l'acide (E/Z)-[4-[3-(biphényl-4-yl)-3-(furan-2-yl)-allylsulfanyl]-2-méthylphénoxylacétique ;
l'acide (E/Z)- [4-[3-(benzo[b]thiophén-3-yl)-3-(biphényl-4-yl)allylsulfanyl]-2-méthylphènoxy]acétique ;
l'acide [4-[(3-benzo[b]thiophén-2-yl)-3-(biphényl-4-yl)-allylsulfanyl]-2-méthylphènoxy]-acétique ;
l'acide (E/Z)-[4-[-3-(4-biphénylyl)-3-(5-méthylthiophén-2-yl)-allylsulfanyl]-2-méthylphénoxy]acétique ;
ou l'un de leurs sels pharmaceutiquement acceptables, ou l'un de leurs solvates pharmaceutiquement acceptables, ou toutes formes tautomères, tous stéréoisomères, tout mélange de stéréoisoméxes y compris un mélange racémique, ou tous polymorphes.

41. Composé selon l'une quelconque des revendications 1 à 38, qui est :
l'acide (E)-{4-[3-biphényl-4-yl-3-(2-fluorophényl)allylsulfanyl]phénoxy}acétique ;
l'acide (Z)-{4-[3-biphényl-4-yl-3-(2-fluorophényl)allylsulfanyl]phénoxy}acétique ;
l'acide (E)-{4-[3-biphényl-4-yl-3-(2-chlorophényl)allylsulfanyl]phénoxy}acétique ;
l'acide (Z)-{4-[3-biphényl-4-yl-3-(2-chlorophényl)allylsulfanyl]phénoxy}acétique ;
l'acide (E)-{4-[3-biphényl-4-yl-3-(2-bromophényl)-allylsulfanyl]phénoxy}acétique ;
l'acide (Z)-{4-[3-biphényl-4-yl-3-(2-bromophényl)-allylsulfanyl]phénoxy}acétique ;
l'acide (E)-{4-[3-biphényl-4-yl-3-(2-iodophényl)-allylsulfanyl]phénoxy}acétique ;
l'acide (Z)-{4-[3-biphényl-4-yl-3-(2-iodophényl)-allylsulfanyl]phénoxy}acétique;
l'acide (E)-{4-[3-biphényl-4-yl-3-(2-méthoxy-phényl)-allylsulfanyl]phénoxy}acétique;
l'acide (Z)-{4-[3-biphényl-4-yl-3-(2-méthoxy-phenyl)-allylsulfanyl]phénoxy}acétique ;
l'acide (E)-{4-[3-biphényl-4-yl-3-(2-trifluorométhoxyphényl)allylsulfanyl]phénoxy}acétique ;
l'acide (Z)-{4-[3-biphényl-4-yl-3-(2-trifluorométhoxyphényl)allylsulfanyl]phénoxy}acétique ;
l'acide (E)-{4-[3-biphényl-4-yl-3-(2-méthyl-phényl)-allylsulfanyl]phénoxy}acétique ;
l'acide (Z)-{4-[3-biphényl-4-yl-3-(2-méthyl-phényl)-allylsulfanyl]phénoxy}acétique ;
l'acide (E)-{4-[3-biphényl-4-yl-3-(2-trifluoro-méthylphényl)allylsulfanyl]phénoxy}acétique;
l'acide (E)-{4-[3-biphényl-4-yl-3-(2-trifluoro-méthylphényl)allylsulfanyl]phénoxy}acétique ;
l'acide (E)-{4-[3-biphényl-4-yl-3-(3-fluorophényl)allylsulfanyl]phénoxy}acétique ;
l'acide (Z)-{4-[3-biphényl-4-yl-3-(3-fluorophényl)allylsulfanyl]phénoxy}acétique ;
l'acide (E)-{4-[3-biphényl-4-yl-3-(3-Chlorophényl)allylsulfanyl]phénoxy}acétique ;
l'acide (Z)-{4-[3-biphényl-4-yl-3-(3-chlorophényl)allylsulfanyl]phénoxy}acétique ;
l'acide (E)-{4-[3-biphényl-4-yl-3-(3-bromophényl)-allylsulfanyl]phénoxy}acétique ;
l'acide (Z)-{4-[3-biphényl-4-yl-3-(3-bromophényl)-allylsulfanyl]phénoxy}acétique ;
l'acide (E)-{4-[3-biphényl-4-yl-3-(3-iodophényl)-allylsulfanyl]phénoxy}acétique ;
l'acide (Z)-{4-[3-biphényl-4-yl-3-(3-xodophényl)-allylsulfanyl]phénoxy}acétique ;
l'acide (E)-{4-[3-biphényl-4-yl-3-(3-méthoxy-phényl)-allylsulfanyl]phénoxy}acétique ;
l'acide (Z)-{4-[3-biphényl-4-yl-3-(3-méthoxy-phényl)-allylsulfanyl]phénoxy}acétique ;
l'acide (E)-{4-[3-biphényl-4-yl-3-(3-trifluorométhoxyphényl)allylsulfanyl]phénoxy}acétique
l'acide (Z)-{4-[3-biphényl-4-yl-3-(3-trifluorométhoxyphényl)allylsulfanyl]phénoxy}acétique
l'acide (E)-{4-[3-biphényl-4-yl-3-(méthyl-phényl)-allylsulfanyl]phénoxy}acétique ;
l'acide (Z)-{4-[3-biphényl-4-yl-3-(3-méthyl-phényl)-allylsulfanyl]phénoxy}acétique ;
l'acide (E)-{4-[3-biphényl-4-yl-3-(3-trifluoro-méthylphényl)allylsulfanyl]phénoxy}acétique ;
l'acide (Z)-{4-[3-biphényl-4-yl-3-(3-trifluoro-méthylphényl)allylsulfanyl]phénoxy}acétique ;
l'acide (E)-{4-[3-biphényl-4-yl-3-(4-fluorophényl)allylsulfanyl]phénoxy}acétique ;
l'acide (Z)-{4-[3-biphényl-4-yl-3-(4-fluorophényl)allylsulfanyl]phénoxy}acétique ;
l'acide (E)-{4-[3-biphényl-4-yl-3-(4-chlorophényl)allylsulfanyl]phénoxy}acétique ;
l'acide (Z)-{4-[3-biphényl-4-yl-3-(4-chlorophényl)allylsulfanyl]phénoxy}acétique ;
l'acide (E/Z)-{4-[3-biphényl-4-yl-3-(4-bromo-phényl)-allylsulfanyl]phénoxy}acétique ;
l'acide (E)-{4-[3-biphényl-4-yl-3-(4-bromo-phényl)-allylsulfanyl]phénoxy}acétique ;
l'acide (Z)-{4-[3-biphényl-4-yl-3-(4-bromophényl)-allylsulfanyl]phénoxy}acétique ;
l'acide (E)-{4-[3-biphényl-4-yl-3-(4-iodophényl)-allylsulfanyl]phénoxy}acétique ;
l'acide (Z)-{4-[3-biphényl-4-yl-3-(4-iodophényl)-allylsulfanyl]phénoxy}acétique ;
l'acide (E)-{4-[3-biphényl-4-yl-3-(4-méthoxy-phényl)-allylsulfanyl]phénoxy}acétique ;
l'acide (Z)-{4-[3-biphényl-4-yl-3-(4-méthoxy-phényl)-allylsulfanyl]phénoxy}acétique ;
l'acide (E)-{4-[3-biphényl-4-yl-3-(4-trifluorométhoxyphényl)allylsulfanyl]phénoxy}acétique ;
l'acide (Z)-{4-[3-biphényl-4-yl-3-(4-trifluorométhoxyphényl)allylsulfanyl]phénoxy}acétique;
l'acide (E)-{4-[3-biphényl-4-yl-3-(4-méthyl-phényl)-allylsulfanyl]phénoxy}acétique ;
l'acide (Z)-{4-[3-biphényl-4-yl-3-(4-méthyl-phényl)-allylsulfanyl]phénoxy}acétique ;
l'acide (E)-{4-[3-biphényl-4-yl-3-(4-trifluoro-méthylphényl)allylsulfanyl]phénoxy}acétique ;
l'acide (Z)-{4-[3-biphényl-4-yl-3-(4-trifluoro-méthylphényl)allylsulfanyl]phénoxy}acétique ;
l'acide (E)-{4-[3-(4-fluorophényl)-3-(2'-trifluorométhyl-biphényl-4-yl)allylsulfanyl]-2-méthylphénoxy}acétique ;
l'acide (Z)-{4-[3-(4-fluorophényl)-3-(2'-trifluorométhyl-biphényl-4-yl)allylsulfanyl]-2-méthylphénoxy}acétique ;
l'acide (E/z)-{4-[3-(4-fluorophényl)-3-(3'-trifluorométhyl-biphényl-4-yl)allylsulfanyl]-2-méthylphénoxy}acétique ;
l'acide (E)-{4-[3-(4-fluorophényl)-3-(3'-trifluorométhyl-biphényl-4-yl)allylsulfanyl]-2-méthylphénoxy}acétique ;
l'acide (Z)-{4-[3-(4-fluorophényl)-3-(3'-trifluorométhyl-biphényl-4-yl)allylsulfanyl]-2-méthylphénoxy}acétique ;
l'acide (E)-{4-[3-(4-fluorophényl)-3-(4'-trifluorométhyl-biphényl-4-yl)allylsullanyl]-2-méthylphénoxy}acétique;
l'acide (Z)-{4-[3-(4-fluorophényl)-3-(4'-trifluorométhyl-biphényl-4-yl)allylsulfanyl]-2-méthylphénoxy}acétique ;
l'acide (E)-{4-[3-(4-fluorophènyl)-3-(3'-chlorobiphényl-4-yl)allylsulfanyl]-2-méthylphénoxy}-acétique ;
l'acide (Z)-{4-[3-(4-fluorophényl)-3-(3'-chlorobiphényl-4-yl)allylsulfanyl]-2-méthylphénoxy}-acétique ;
l'acide (E)-{4-[3-(4-fluorophényl)-3-(4'-chlorobiphényl-4-yl)allylsulfanyl]-2-méthylphénoxy}-acétique ;
l'acide (Z)-{4-[3-(4-fluorophényl)-3-(4'-chlorobiphényl-4-yl)allylsulfanyl]-2-méthylphénoxy}-acétique ;
l'acide (E)-{4-[3-(4-fluorophényl)-3-(3'-méthoxybiphényl-4-yl)allylsulfanyl]-2-méthylphénoxy}-acétique ;
l'acide (Z)-{4-[3-(4-fluorophényl)-3-(3'-méthoxybiphényl-4-yl)allylsulfanyl]-2-méthylphénoxy}-acétique ;
l'acide (E)-{4-[3-(4-fluorophényl)-3-(4'-méthoxybiphényl-4-yl)allylsulfanyl]-2-méthylphénoxy}-acétique ;
l'acide (Z)-{4-[3-(4-flluorophényl)-3-(4'-méthoxybiphényl-4-yl)allylsulfanyl]-2-méthylphénoxy} acétique ;
l'acide (E)-{4-[3-(4-chlorophényl)-3-(2'-trifluorométhyl-biphényl-4-yl)allylsulfanyl]-2-méthylphénoxy}acétique ;
l'acide (Z)-{4-[3-(4-chlorophényl)-3-(2'-trifluorométhyl-biphényl-4-yl)allylsulfanyl]-2-mêthylphénoxy}acétique ;
l'acide (E/Z)-{4-[3-(4-chlorophenyl)-3-(3'-trifluorométhyl-biphényl-4-yl)allylsulfanyl]-2-méthylphénoxy}acétique ;
l'acide (E)-{4-[3-(4-chlorophényl)-3-(3'-trifluorométhyl-biphényl-4-yl)allylsulfanyl]-2-méthylphénoxy}acétique ,
l'acide (Z)-{4-[3-(4-chlorophényl)-3-(3'-trifluorométhyl-biphényl-4-yl)allylsulfanyl]-2-méthylphénoxy}acétique ;
l'acide (E)-{4-[3-(4-chlorophényl)-3-(4'-trifluorométhyl-biphényl-4-yl)allylsulfanyl]-2-méthylphénoxy}acétique ;
l'acide (Z)-{4-[3-(4-chlorophényl)-3-(4'-trifluorométhyl-biphényl-4-yl)allylsulfanyl]-2-méthylphénoxy}acétique. ;
l'acide (E)-{4-[3-(4-chlorophényl)-3-(3'-chlorobiphényl-4-yl)allylsulfanyl]-2-méthylphénoxy}-acétique ;
l'acide (Z)-{4-[3-(4-chlorophényl)-3-(3'-chlorobiphényl-4-yl)allylsulfanyl]-2-méthylphénoxy}-acétique ;
l'acide (E)-{4-[3-(4-chlorophényl)-3-(4'-chlorobiphényl-4-yl)allylsulfanyl]-2-méthylphénoxy}-acétique ;
l'acide (Z)-{4-[3-(4-chlorophényl)-3-(4'-chlorobiphényl-4-yl)allylsulfanyl]-2-méthylphénoxy}-acétique ;
l'acide (E)-{4-[3-(4-chlorophényl)-3-(3'-méthoxybiphényl-4-yl)allylsulfanyl]-2-méthylphénoxy}-acétique ;
l'acide (Z)-{4-[3-(4-chlorophényl)-3-(3'-méthoxybiphényl-4-yl)allylsulfanyl]-2-méthylphénoxy}-acétique ;
l'acide (E)-{4-[3-(4-chlorophényl)-3-(4'-méthoxybiphényl-4-yl)allylsulfanyl]-2-méthylphénoxy}-acétique ;
l'acide (Z)-{4-[3-(chlorophényl)-3-(4'-méthoxybiphényl-4-yl)allylsulfanyl]-2-méthylphénoxy}-acétique ;
l'acide (E)-{4-[3-(4-bromophényl)-3-(2'-trifluorométhyl-biphényl-4-yl)allylsulfanyl]-2-méthylphénoxy}acétique ;
l'acide (Z)-{4-[3-(4-bromophényl)-3-(2'-trifluorométhyl-biphényl-4-yl)allylsulfanyl]-2-méthylphénoxy}acétique ;
l'acide (E/Z)-{4-[3-(4-bromophényl)-3-(3'-trifluorométhyl-biphényl-4-yl)allylsulfanyl]-2-méthylphénoxy}acétique ;
l'acide (E)-{4-[3-(4-bromophényl)-3-(3'-trifluorométhyl-biphényl-4-yl)allylsulfanyl]-2-méthylphénoxy}acétique ;
l'acide (Z)-{4-[3-(4-bromophényl)-3-(3'-trifluorométhyl-biphényl-4-yl)allylsulfanyl]-2-méthylphénoxy}acétique ;
l'acide (E)-{4-[3-(4-bromophényl)-3-(4'-trifluorométhyl-biphényl-4-yl)allylsulfanyl]-2-méthylphénoxy}acétique ;
l'acide (Z)-{4-[3-(4-bromophényl)-3-(4'-trifluorométhyl-biphényl-4-yl)allylsulfanyl]-2-méthylphènoxy}acétique ;
l'acide (E)-{4-[3-(4-bromophényl)-3-(3'-chlorobiphényl-4-yl)allylsulfanyl]-2-méthylphénoxy} acétique ;
l'acide (Z)-{4-[3-(4-bromophényl)-3-(3'-chlorobiphényl-4-yl)allylsulfanyl]-2-méthylphénoxy}-acétique ;
l'acide (E)-{4-[3-(4-bromophényl)-3-(4'-chlorobiphényl-4-yl)allylsulfanyl]-2-méthylphénoxy}-acétique ;
l'acide (Z)-{4-[3-(4-bromophényl)-3-(4'-chlorobiphényl-4-yl)allylsulfanyl]-2-méthylphénoxy}-acétique ;
l'acide (E)-{4-[3-(4-bromophényl)-3-(3'-méthoxybiphényl-4-yl)allylsulfanyl]-2-méthylphénoxy}-acétique ;
l'acide (Z)-{4-[3-(4-bromophényl)-3-(3'-méthoxybiphényl-4-yl)allylsulfanyl]-2-méthylphénoxy}-acétique ;
l'acide (E)-{4-[3-(4-bromophényl)-3-(4'-méthoxybiphényl-4-yl)allysulfanyl]-2-méthylphénoxy}-acétique ;
l'acide (Z)-{4-[3-(4-bromophényl)-3-(4'-méthoxybiphényl-4-yl)allylsulfanyl]-2-méthylphénoxy} acétique ; ou
l'un de leurs sels pharmaceutiquement acceptables, ou l'un de leurs solvates pharmaceutiquement acceptables, ou toutes formes tautomères, tous stéréoisomères, tout mélange de stéréoisomères y compris un mélange racémique, ou tous polymorphes.

42. Combinaison entre un composé selon l'une quelconque des revendications 1 à 41 et une ou plusieurs substances pharmacologiquement actives supplémentaires.

43. Combinaison selon la revendication 42, dans laquelle la ou les substances pharmacologiquement actives supplémentaires sont choisies parmi les agents anti-obésité, les antidiabétiques, les agents antihypertenseurs, les agents destinés au traitement et/ou à la prévention des complications résultant du diabète ou associées au diabète et les agents destinés au traitement et/ou à la prévention des complications et des troubles résultant de l'obésité ou associés à l'obésité.

44. Composition pharmaceutique comprenant, en tant qu'ingrédient actif, au moins un composé selon l'une quelconque des revendications 1 à 41, ou une combinaison selon la revendication 42 ou 43, avec un ou plusieurs supports ou excipients pharmaceutiquement acceptables.

45. Composition pharmaceutique selon la revendication 44 sous une forme galénique unitaire, comprenant environ 0,05 mg à 1 000 mg, de préférence environ 0,1 mg à environ 500 mg, et de manière particulièrement préférée environ 0,5 mg à environ 200 mg par jour d'un composé selon l'une quelconque des revendications 1 à 41.

46. Composition pharmaceutique selon la revendication 44 ou la revendication 45, pour une administration par voie orale, nasale, transdermique, pulmonaire ou parentérale.

47. Composé selon l'une quelconque des revendications 1 à 41, combinaison selon la revendication 42 ou 43 ou composition selon l'une quelconque des revendications 44 à 46, pour un usage en tant que médicament.

48. Composé, combinaison ou composition selon la revendication 47, pour le traitement et/ou la prévention d'affections dont les médiateurs sont les récepteurs activés par les proliférateurs des peroxysomes (PPAR).

49. Composé, combinaison ou composition selon la revendication 47, pour le traitement et/ou la prévention du diabète de type 1 ; du diabète de type 2 ; de la dyslipidémie ; du syndrome X comme le syndrome métabolique, de l'intolérance au glucose, de l'insulino-résistance, de l'hypertriglyceridémie et/ou de l'obésité ; des maladies cardiovasculaires, comme l'athérosclérose ; et l'hyper-cholestérolémie.

50. Composé, combinaison ou composition selon la revendication 47, pour le traitement et/ou la prévention de l'hyperglycémie et de l'hyperlipidémie.

51. Procédé de préparation d'un composé selon l'une quelconque des revendications 1 à 41, dans lequel ledit procédé est choisi parmi les procédés (A) à (F) ci-dessous :
(A) la réaction d'un composé de formule (II) 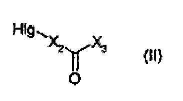
dans laquelle X₂ et X₃ sont définis comme dans la revendication 1 et dans laquelle Hlg est un brome ou un iode, par l'intermédiaire d'un procédé de type Wittig avec (EtO)₂PO(CHR₁)COOR₆ où R₆ est un groupe alkyle et R₁ est défini comme dans la revendication 1, en présence d'une base pour donner un composé de formule (III) 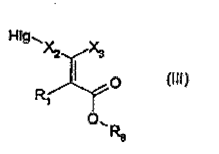
dans laquelle X₂, X₃, R₁ et R₆ sont tels que définis ci-dessus et dans laquelle Hlg est un brome ou un iode, et
la réduction du composé de formule (III) pour donner un composé de formule (IV) 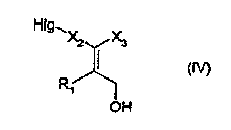
dans laquelle X₂, X₃ et R₁ sont tels que définis ci-dessus et dans laquelle Hlg est un brome ou un iode, et
la réaction du composé de formule (IV), dans laquelle X₂, X₃ et R₁ sont tels que définis ci-dessus et dans laquelle Hlg est un brome ou un iode, sauf que quand X₂ ou X₃ est substitué avec un hydroxy, cette fonctionnalité doit être protégée, avec un composé de formule (V) 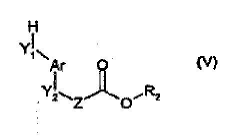
dans laquelle Y₁, Ar, Y₂, Z et R₂ sont définis comme dans la revendication 1, sauf que R₂ n'est pas une hydrogène dans les conditions de Mitsunobu, au moyen d'un réactif tel que le couple triphénylphosphine/azodicarboxylate de diéthyle pour obtenir un composé de formule (VI) 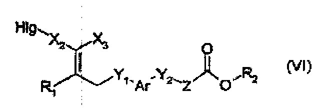
dans laquelle X₂, X₃, Y₁, Y₂, Ar, Z, R₁ et R₂ sont tels que définis ci-dessus, sauf que R₂ n'est pas un hydrogène, et dans laquelle Hlg est un brome ou un iode, et
la réaction d'un composé de formule (VI) avec un acide boronique ou avec un dérivé de X₁ contenant du tributylétain dans des conditions de couplage appropriées pour donner un composé de formule (I), dans laquelle X₁, X₂, X₃, Y₁, Y₂, Ar, Z, R₁ et R₂ sont tels que définis ci-dessus, sauf que R₂ n'est pas un hydrogène ; ou
(B) la saponification par voie chimique ou enzymatique d'un composé de formule (I), dans laquelle X₁, X₂, X₃, Y₁, Y₂, Ar, Z, R₁ et R₂ sont définis comme dans la revendication 1, sauf que R₂ n'est pas un hydrogène, pour donner un composé de formule (I) dans laquelle X₁, X₂, X₃, Y₁, Y₂, Ar, Z, R₁ et R₂ sont tels que définis ci-dessus, sauf que R₂ est un hydrogène ; ou
(C) la saponification par voie chimique ou enzymatique d'un composé de formule (VI), dans laquelle X₂, X₃, Y₁, Y₂, Ar, Z, R₁ et R₂ sont définis comme dans la revendication 1, sauf que R₂ n'est pas un hydrogène, et dans laquelle Hlg est un brome ou un iode, pour donner un composé de formule (VI) dans laquelle X₁, X₂, X₃, Y₁, Y₂, Ar, Z, R₁ et R₂ sont tels que définis ci-dessus, sauf que R₂ est un hydrogène et dans laquelle Hlg est un brome ou un iode ; et
la réaction d'un composé de formule (VI) avec un dérivé d'acide boronique de X₁, dans des conditions de couplage appropriées pour donner un composé de formule (I), dans laquelle X₁, X₂, X₃, Y₁, Y₂, Ar, Z, R₁ et R₂ sont tels que définis ci-dessus, sauf que R₂ est un hydrogène ; ou
(D) la réaction d'un composé de formule (VII) 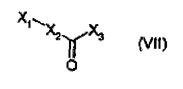
dans laquelle X₁, X₂ et X₃ sont définis comme dans la revendication 1, par l'intermédiaire d'un procédé de type Wittig avec (EtO)₂PO(CHR₁)COOR₆ où R₆ est un groupe alkyle et R₁ est défini comme dans la revendication 1, en présence d'une base pour donner un composé de formule (VIII) 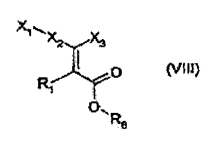
dans laquelle X₁, X₂, X₃, R₁ et R₆ sont tels que définis ci-dessus, et
la réduction du composé de formule (VIII) dans laquelle X₁, X₂, X₃, R₁ et R₆ sont tels que définis ci-dessus pour donner un composé de formule (IX) 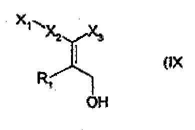
dans laquelle X₁, X₂, X₃ et R₁ sont tels que définis ci-dessus, et
la réaction du composé de formule (IX), dans laquelle X₁, X₂, X₃ et R₁ sont tels que définis ci-dessus, sauf que quand X₁, X₂ ou X₃ est substitué avec un hydroxy, cette fonctionnalité droit être protégée, avec un composé de formule (V) tel que défini dans le procédé (A) ci-dessus dans les conditions de Mitsunobu, au moyen d'un réactif tel que le couple triphénylphosphine/azodicarboxylate de diéthyle pour obtenir un composé de formule (I), dans laquelle X₁, X₂, X₃, Y₁, Y₂, Ar, Z, R₁ et R₂ sont tels que définis ci-dessus, sauf que R₂ n'est pas un hydrogène ; ou
(E) la conversion de la fonctionnalité -OH dans le composé de formule (IX) tel que défini dans le procédé (D) ci-dessus, en groupe partant (L) approprié, tel qu'un p-toluènesulfonate, un méthanesulfonate, un halogène ou un triflate, pour donner un composé de formule (X) 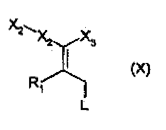
dans laquelle X₁, X₂, X₃, R₁ et L sont tels que définis ci-dessus,
la réaction du composé de formule (X) avec un composé de formule (V) tel que défini dans le procédé (A) ci-dessus, pour donner un composé de formule (I) laquelle X₁, X₂, X₃, Y₁, Y₂, Ar, Z, R₁ et R₂ sont tels que définis ci-dessus, sauf que R₂ n'est pas un hydrogène ; ou
(F) la réaction d'un composé de formule (XI) 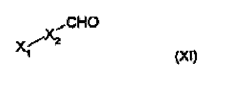
dans laquelle X₁ et X₂ sont définis comme dans la revendication 1, avec du tétrabromure de carbone et de la triphénylphosphine pour donner un composé de formule (XII) 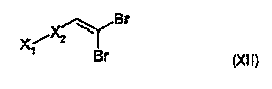
dans laquelle X₁ et X₂ sont tels que définis ci-dessus,
la réaction du composé de formule (XII), avec du paraformaldéhyde en présence d'une base forte, pour donner un composé de formule (XIII) 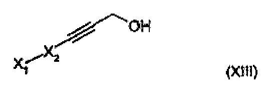
dans laquelle X₁ et X₂ sont tels que définis ci-dessus,
la réduction du composé de formule (XIII) avec du LiAlH en présence d'une base, suivie d'un traitement avec le carbonate de diméthyle et de l'iode pour donner un composé de formule (XIV) 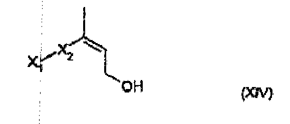
dans laquelle X₁ et X₂ sont tels que définis ci-dessus,
la conversion de la fonction hydroxyl du composé de formule (XIV) en groupe partant (L), comme décrit dans (E), pour donner un composé de formule (XV) 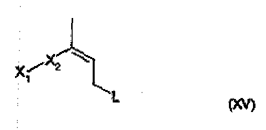
dans laquelle X₁ et X₂ sont tels que définis ci-dessus et L est un groupe partant tel qu'un p-toluènesulfonate, un méthalnesulfonate, un halogène ou un triflate,
la réaction du composé de formule (XV) avec le composé de formule (v), tel que défini dans le procédé (A), pour donner un composé de formule (XVI) 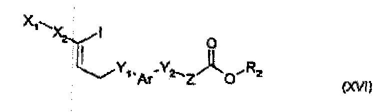
dans laquelle X₁, X₂, Y₁, Y₂, Ar, Z et R₂ sont tels que définis ci-dessus, sauf que R₂ n'est pas un hydrogène,
la réaction du composé de formule (XVI) avec un X₂-tributylétain en présence d'un catalyseur à base de palladium et de tri(t-butyl)phosphine pour donner le composé de formule (I), dans laquelle X₁, X₂, X₃, Y₁, Y₂, Ar, Z, R₁ et R₂ sont tels que définis ci-dessus, sauf que R₁ est un hydrogène et R₂ n'est pas un hydrogène ;
et facultativement la conversion du produit issu de l'un quelconque des procédés (A) à (F) en sel pharmaceutiquement acceptable.
